# EUROPEAN PATENT SPECIFICATION

(11) **EP 1 518 558 B1**
(45) Date of publication and mention of the grant of the patent: **20.11.2013**
(21) Application number: 04077590.0
(22) Date of filing: 08.04.1998
(51) Int. Cl.: A61K 39/102, A61K 39/295, G01N 33/569, G01N 33/68, C12N 1/20, C12N 1/21

(54) **Vaccine against infection with Actinobacillus pleuropneumoniae comprising purified ApxIV toxin**
Impfstoffe gegen Actinobacillus pleuropneumoniae Infektion, die hochreines Toxin enthalten
Vaccin contre l'infection par Actinobacillus pleuropneumoniae comprenant la toxine ApxIV purifiée

(30) Priority: 10.04.1997 EP 97201032
(43) Date of publication of application: 30.03.2005
(62) Divisional of application: 98201115.7
(73) Proprietor: Intervet International B.V., 5831 AN Boxmeer (NL)
(72) Inventor: Frey, Joachim, 3012 Bern (CH); Segers, Ruud Philip Antoon Maria, 5831 MZ Boxmeer (NL)
(74) Representative: Keus, Jacobus Albertus Ronald

(56) References cited:
- ROSENDAL ET AL: "Characterization of an attenuated strain of Actinobacillus pleuropneumoniae strain of Actinobacillus pleuropneumoniae, serotype 1" AMERICAN JOURNAL OF VETERINARY RESEARCH, vol. 51, no. 5, May 1990 (1990-05), page 711-717, XP002039387
- FREY J.: "Virulence in Actinobacillus pleuropneumoniae and RTX toxins" TRENDS IN MICROBIOLOGY, vol. 3, no. 7, July 1995 (1995-07), page 257-261, XP002039389
- FREY ET AL: "Typisierung der Apx-Toxin-Gene von Actinobacillus pleuronpneumoniae mittels PCR" SCHWEIZER ARCHIV FÜR TIERHEILKUNDE, vol. 138, no. 3, March 1996 (1996-03), pages 121-124, XP002039390
- KOLODRUBETZ D ET AL: "cis Elements and trans factors are both important in strain-specific regulation of the leukotoxin gene in Actinobacillus actinomycetemcomitans." INFECTION AND IMMUNITY SEP 1996 LNKD- PUBMED:8751884, vol. 64, no. 9, September 1996 (1996-09), pages 3451-3460, XP002592033 ISSN: 0019-9567
- HRITZ M ET AL: "Differential regulation of the leukotoxin operon in highly leukotoxic and minimally leukotoxic strains of Actinobacillus actinomycetemcomitans." INFECTION AND IMMUNITY JUL 1996 LNKD- PUBMED:8698501, vol. 64, no. 7, July 1996 (1996-07), pages 2724-2729, XP002592034 ISSN: 0019-9567
- SCHALLER ET AL: "Characterization of apxIVA, a new RTX determinant of Actinobacillus pleuropneumoniae" MICROBIOLOGY, SOCIETY FOR GENERAL MICROBIOLOGY, READING, GB, vol. 145, 1999, pages 2105-2116, XP002128690 ISSN: 1350-0872
- KUHN ET AL: "Characterization of a novel RTX determinant, apxIV, a Actinobacillus pleuropneumoniae" ZENTRALBLATT FUER BAKTERIOLOGIE. SUPPLEMENT, FISCHER, STUTTGART, DE, no. SUPPL. 29, 1 January 1998 (1998-01-01), page 51/52, XP002128689 ISSN: 0941-018X

## Description

The present invention relates to live attenuated bacteria of the genus *Actinobacillus pleuropneumoniae,* having a mutation in a gene encoding a toxin, methods for the production of such bacteria, to vaccines comprising such bacteria, methods for the production of such vaccines, to vaccines comprising a toxin, methods for the production of such vaccines and methods for the protection of animals against infection with bacteria of the genus *Actinobacillus pleuropneumoniae.*

Bacteria belonging to the genus *Actinobacillus* all produce so-called RTX-toxins. (RTX stands for repeat in toxin).

It is the presence of these RTX-toxins that highly contributes to the pathogenic character of these bacteria.

The RTX-toxins have been extensively reviewed by Braun et al. (Critical Rev. in Microbiol. 18(2): 115-158 (1991)). RTX-toxins in Gram-negative strains have also been reviewed in Welch, R.A. (Molecular Microbiology 5/3: 521-528 (1991)) and in Welch et al. (Inf. Agents and Disease 4: 254-272 (1995)).

All known RTX-toxins display some kind of cytotoxic or cytolytic activity. The target-cell- and host-specificity differ however, depending on the toxin and on differences in acylation (McWhinney et al.; J. Bact. 174: 291-297 (1992) and Hackett et al.; J. Biol. Chem. 270: 20250-20253 (1995)) . As a result of the difference in target cells, the various toxins of the RTX-toxin family are known e.g. as haemolysin, cytolysin or cytotoxin.

The genus *Actinobacillus* comprises a number of different species, inter alia, *Actinobacillus pleuropneumoniae, A. actinomycetemcomitans, A. suis, A. rossii, A. equuli and A. lignieresii.*

*Actinobacillus pleuropneumoniae* produces serotype-dependent RTX-toxins that are cytotoxic/cytolytic to pig, horse, bovine and human erythrocytes, to rabbit and porcine neutrophils and to porcine alveolar macrophages. (Rosendal et al; Am. J. Vet. Res. 49: 1053-1058 (1988), Maudsley J.R. and Kadis S; Can. J. Microbiol. 32: 801-805 (1986), Frey. J and Nicolet. J; Inf. & Imm, 56:2570-2575 (1988), Bendixon et al; Inf. & Imm, 33: 673-676 (1981), Kamp, E.M. and van Leengoed, L.A.M.G.; J. Clin. Microbiol. 27: 1187-1191 (1989)).

Infections with *Actinobacillus* in pigs are the cause of severe economic losses to pig industry, due to acute mortality in young pigs and reduced weight gain in older animals.

The genetic organisation of the operons involved in the synthesis, activation and transportation of the RTX toxins in Gram-negative bacteria has been reviewed recently by Coote, J.G. (FEMS Microbiology reviews 88: 137-162 (1992)). Frey has specifically reviewed the known three RTX-toxins in *Actinobacillus pleuropneumoniae* in Bacterial Protein Toxins, Zbl Bakt. Suppl. 24, p. 322-, Freer et al. (Eds.), Gustaf Fischer, Stutttgart, Jena, New York, 1994.

In *Actinobacillus pleuropneumoniae,* this kind of RTX-operon contains four genes: the actual Toxin-gene (A), an Activator-gene (C), and two genes (B and D) encoding proteins involved in secretion of the toxin into the surrounding medium. The primary translation-product of the Toxin-gene (A) is a non-toxic protein, of which the toxic activity is activated by the Activator-gene (C) product.

Until recently, it was assumed that only three RTX-toxins, all having the above-described genetic organisation or at least having the Toxin-gene (A) and Activator-gene (C), existed in *Actinobacillus* species.

These three RTX-toxins, Apxl, Apx-II and Apx-III have respectively a pronounced haemolytic activity (Apxl), a mild haemolytic activity (Apx-II) or a macrophage-cytotoxic activity (Apx-III).

The various toxic activities are fairly randomly divided over the serotypes. There are four subgroups:
a subgroup A, represented by serotypes 1, 5, 9 and 11, producing Apxl and Apx-II,
a subgroup B, represented by serotypes 2, 3, 4, 6 and 8, producing Apx-II and
Apx-III,
a subgroup C, represented by serotype 10, producing Apxl only,
a subgroup D, represented by serotype 7 and 12, producing Apx-II only,

It is known, that Apxl, -II, and -III all are essential elements in universal vaccines against *Actinobacillus pleuropneumoniae* infection: a vaccine not comprising at least Apxl, -II, and -III will not provide protection against all *Actinobacillus pleuropneumoniae* serotypes. Also, a vaccine not comprising at least the Apx-toxins of one specific serotype will not even induce protection against that single serotype.

Subunit vaccines based on in vitro synthesised RTX-toxins from *A. pleuropneumoniae* that lost their toxicity have been described earlier, e.g. in European Patent EP No. 0.354.628, in which subunit vaccines based upon a haemolysin and a cytotoxin of *A. pleuropneumoniae* are disclosed, and in European Patent EP No 0.453.024, in which *A. pleuropneumoniae* subunit vaccines based upon haemolysins, cytotoxins and outer membrane proteins are disclosed.

There are however four important disadvantages to subunit vaccines in general:
- high amounts of antigenic material are needed in order to adequately trigger the immune system.
- usually, only B-cell immunity is triggered.
- several protective antigens are only triggered *in vivo,* and therefore can not be present in subunit vaccines.
- a live pathogenic bacterium has many important immunogenic molecules, such as Outer Membrane Proteins and capsular polysaccharides, all potentially important for protection and thus to be included in an efficient subunit vaccine.

Next to the obvious problems mentioned under points one and two, especially the fourth point makes it difficult to make an efficient subunit vaccine.

This is e.g. illustrated by the *A. pleuropneumoniae* subunit vaccine disclosed in European Patent EP No 0.453.024 mentioned above, in which four different subunits (three RTX-toxins and an outer membrane protein) are combined in one vaccine.

It is clear, that in order to overcome the disadvantages of subunit vaccines against *Pas*teurellaceae-infection, a live attenuated vaccine would be highly desirable.

A live attenuated vaccine has the following advantages:
it can be administered in low doses (it is self-replicating)
it closely mimics the natural/wild-type infection
it provides all the possible immunologically important antigens at the same time. Nevertheless, in spite of the clear advantages, no live vaccines based on *Actinobacillus pleuropneumoniae* were commercially available prior to the present invention.

The reason for this lies in the following paradox: as mentioned before, Apxl, -II, and -III all are essential elements of universal vaccines against *Actinobacillus pleuropneumoniae* infection. Live vaccines therefore have to produce these three RTX-toxins. These three RTX-toxins are however strong virulence factors in all *Actinobacillus* species (see e.g. Coote, J.G.; FEMS Microbiology reviews 88: 137-162 (1992), Tascon et al.; Mol. Microbiol. 14: 207-216 (1994)), Jansen et al.; Inf. & Imm. 63: 27-37 (1995)).

Deletion of the RTX-toxins in order to attenuate the virulence of live App strains is technically feasible, but this does not provide a solution for the dilemma: such RTX-negative strains would be useless as live attenuated vaccine strains since they do no longer induce immunity in the host against the haemolytic/cytotoxic activity of *Actinobacillus pleuropneumoniae* field strains.

Virulence factors that, although important in the induction of immunity, do play a less important role in building up immunity than Apxl, -II and -III, and thus can in principle be deleted are however currently not known.

It would thus be highly desirable to have a site on the genome of App that attributes to virulence and therefore leads to an attenuated App strain when modified, whereas at the same time it is, although useful in triggering immunity, dispensable from a vaccine point of view. No such sites are however currently known. Moreover, it would be highly desirable if such a site would be universally present in all App strains, instead of being restricted to certain serotypes. Such a site would then allow all different serotypes to be attenuated by deletion of that same site.

It is one of the objectives of the present invention to provide such an attenuation site, universally present in all *Actinobacillus pleuropneumoniae* strains regardless their serotype.

Recently, a new gene was found in a serotype 1 strain of *Actinobacillus pleuropneumoniae* (Thesis T.J. Anderson Nov. 1995).

Although this gene does not resemble the known *Actinobacillus* Apxl, -II and -III genes, it bears resemblance to RTX-toxin genes known from bacteria belonging to *Neisseria meningitidis,* for which reason it was named RTX-gene *apxIV.* The gene however differs in almost all aspects from the three known RTX-toxin genes *apxI,* -*II* and -*III* present in the various species of the *Actinobacillus* family as described above. First of all, the genomic organisation is completely different. Secondly, there is no activator-mechanism as is found for the known Apx-toxins. In the third place, no specific *in vivo* haemolytic or cytotoxic activity could at that time be attributed to the gene, or it's possible gene product.

It was now surprisingly found that this gene, fully in contrast with the three known RTX-genes, is present in all bacteria of the species *Actinobacillus pleuropneumoniae,* regardless their serotype. This was determined by hybridisation of a probe comprising apxIV coding sequences with restriction fragments of the DNA from *Actinobacillus pleuropneumoniae* of all serotypes as described in Example 6 and 7.

Unexpectedly it was found now, that *apxIV* deletion mutants are viable, but they behave less virulent compared to their *apxIV*-possessing parent strains.

Therefore, it was determined that the gene product, the ApxIV toxin is a virulence factor in all *Actinobacillus pleuropneumoniae* strains. This is an unexpected conclusion, since up until now, no effects at al, let alone effects possibly influencing virulence had been attributed to the gene product *in vivo.* In fact, up until now there was not even proof that the gene was expressed in *Actinobacillus pleuropneumoniae in vivo* or *in vitro* anyway.

It therefore is one of the merits of the invention that it was found that:
- the *apxIV* gene is present in all *A. pleuropneumoniae* strains regardless the serotype,
- the *apxIV* gene product is a virulence factor in all *A. pleuropneumoniae* serotypes,
- *A. pleuropneumoniae* strains with a deletion in the *apxIV* gene are still viable but have a decreased virulence without significantly impairing the immunogenic properties of the strains,

Therefore, for the first time a live attenuated bacteria of the species *Actinobacillus pleuropneumoniae* is provided, that do not produce a functional ApxIV toxin.

A functional ApxIV toxin is considered to be a protein that has all the characteristics of the ApxIV toxin as expressed in a wild-type bacterium, and is expressed at the wild-type level. Therefore, a non-functional ApxIV toxin is considered to be a toxin that lacks some or all of the characteristics of the ApxIV toxin as expressed in a wild-type bacterium, and/or is expressed at a level, insufficient to obtain wild-type effects of ApxIV toxin.

The inability to produce the ApxIV toxin can e.g. be due to modifications in the coding sequence encoding the ApxIV toxin. It may also be e.g. the result of modifications in regions known to be involved in transcription of the *apxIV* gene, such as the promotor region, or of modifications in regions involved in translation, such as the ribosome binding site.

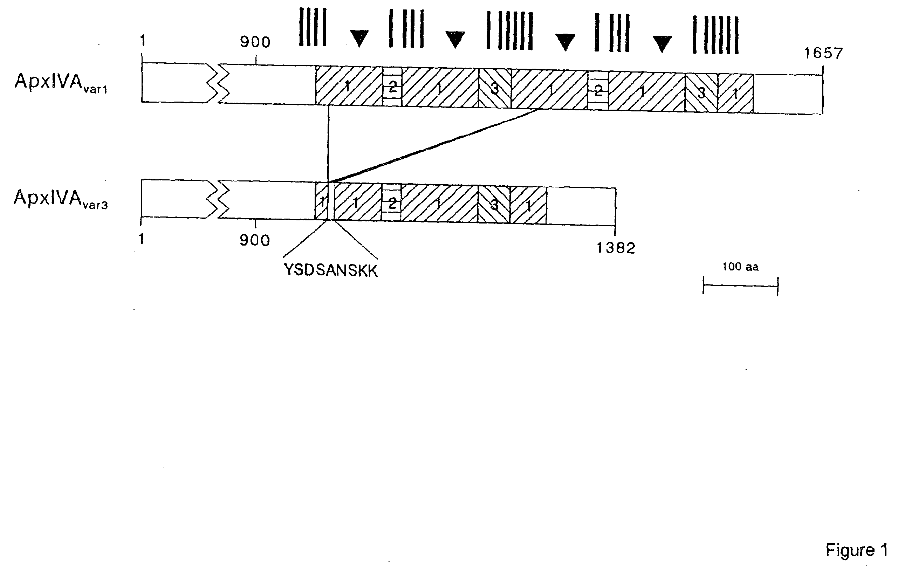

The overall structure of the *apxIV gene* is given in figure 1.

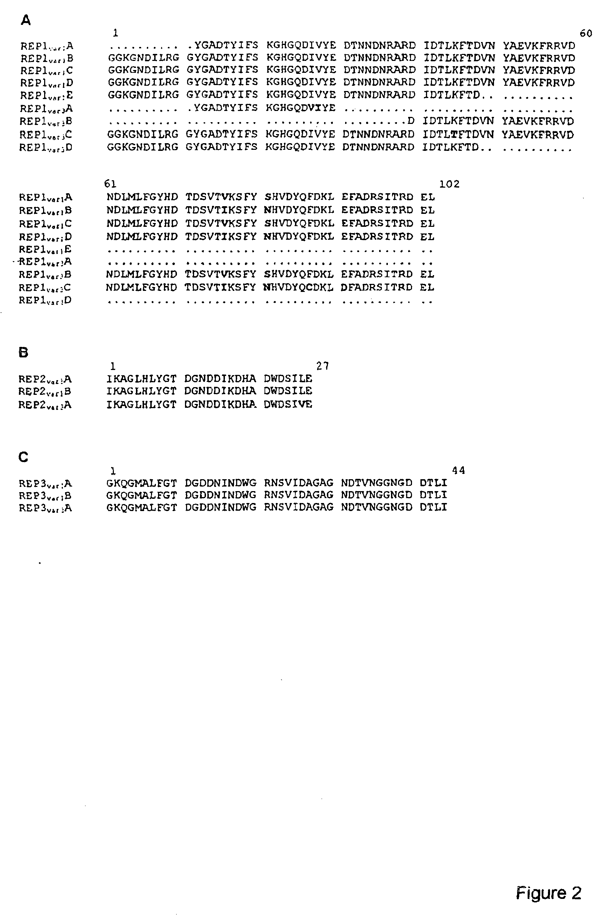

In this figure, the direct repeat regions, characteristic for ApxIV toxin are indicated by dashed boxes, whereas the also ApxIV-specific glycine-rich nonapeptide regions are indicated by black arrows. The repeats are found at the C-terminal part of ApxIV. These characteristic features are present in all *Actinobacillus pleuropneumoniae* serotypes. The nucleic acid sequence and amino acid sequence of two serotypes are represented in SEQ. ID. No. 1-4. SEQ. ID. NO. 1 shows the nucleic acid sequence of the *apxIV* gene of App serotype 1, and SEQ. ID. NO. 2 shows the matching amino acid sequence of the serotype 1 ApxIV toxin. SEQ. ID. NO. 3 shows the nucleotide sequence of the *apxIV* gene of App serotype 3, whereas SEQ. ID. NO. 4 shows the matching amino acid sequence of the serotype 3 ApxIV toxin. Figure 2 shows the strikingly high level of conservation at amino acid level, especially in the N-terminal 650 amino acids, between the Apx-toxins of the various *Actinobacillus pleuropneumoniae* serotypes. This is also a remarkable characteristic of the *apxIV* genes. It is clear from figure 1, that a variation in the number of repeats at the C-terminal part of the toxin may occur, depending on the serotype. This variation accounts for the difference in size of the genes and encoded toxins obtained from the various serotypes.

There may be some variation in nucleic acid sequence even between *apxIV* genes isolated from different isolates of *Actinobacillus pleuropneumoniae,* belonging to the same serotype. This is due to natural variation well known in the art to exist in all organisms. It is possible that some amino acids in the ApxIV toxin encoded by the *apxIV* gene are replaced by others in the ApxIV toxin of another serotype, while the polypeptide is not altered in its function. For instance, a polypeptide containing Asp at a certain site, and its variant containing Asn at the comparable site still have the same properties. This process in which an amino acid is replaced by an functionally analogous amino-acid is called functional displacement. In this case the variant proteins are called functional variants. Another cause of variation is the phenomenon of degeneracy of the genetic code. Shortly, it means, that e.g. the amino acid glutamic acid is coded for by both GAT and GAA. This phenomenon holds for all amino acids, except Met and Trp. Thus, it is obvious, that e.g. the ApxIV toxin of serotype 1, as given in the present invention can not only be coded for by the nucleotide sequence given in SEQ ID NO: 1 but also by a very large variety of other sequences, still all giving the same or functionally the same polypeptides.

Therefore, a variant apxIV sequence encoding a polypeptide that is functionally comparable to the ApxIV toxin falls within the scope of the present invention.

Live attenuated bacteria can be obtained in several ways. One possible way of obtaining such bacteria is by means of classical methods such as the treatment of wild-type *Actinobacillus pleuropneumoniae* bacteria with mutagenic agents such as base analogues, treatment with ultraviolet light or temperature treatment. Strains that do not produce a functional ApxIV toxin do not or to a lesser extend induce anti-ApxIV toxin antibodies, and therefore can easily be selected in animal tests.

The necessary antiserum can be obtained as described below in Example 3.

Another possibility is to deliberately introduce, using recombinant DNA-technology, a well-defined mutation in the gene encoding the ApxIV toxin. Such a mutation may be an insertion, a deletion, a replacement of one nucleotide by another one or a combination thereof, with the only proviso that the mutated gene no longer encodes a functional ApxIV toxin.

It can easily be seen, that especially mutations introducing a stop-codon in the open reading frame, or mutations causing a frame-shift in the open reading frame are very suitable to obtain a strain which no longer encodes a functional ApxIV toxin. Such mutations can e.g. be made by means of in vitro site directed mutagenesis using the Transformer® kit sold by Clontech. Many other standard recombinant DNA techniques such as digestion of the gene with a restriction enzyme, followed by endonuclease treatment and religation, are equally applicable.

Therefore, in a preferred form, this embodiment relates to live attenuated bacteria in which the gene encoding the ApxIV toxin comprises a mutation.

Well-defined mutations involving the deletion of fragments of the *apxIV* gene or even the whole gene, or the insertion of heterologous DNA-fragments, when compared to classically induced mutations, have the advantage that they will not revert to the wild-type situation.

Thus, in a more preferred form, this embodiment refers to live attenuated bacteria in which the gene encoding the ApxIV toxin comprises an insertion and/or a deletion.

Given the large amount of vaccines given nowadays to pigs, it is clear that combined administration of several vaccines would be desirable, if only for reasons of decreased vaccination costs. It is therefore very attractive to use live attenuated vaccine strains as a recombinant carrier for heterologous genes, encoding antigens selected from other pathogenic micro-organisms or viruses. Administration of such a recombinant carrier has the advantage that after administration of such a carrier, immunity is induced against two or more diseases at the same time. The live attenuated bacteria provide a very suitable carrier for heterologous genes, since the gene encoding the ApxIV toxin can be used as an insertion site for such heterologous genes. The use of the *apxIV* gene as an insertion site has the advantage that at the same time the *apxIV* gene is inactivated, and the newly introduced heterologous gene can be expressed in accordance with the homologous *Actinobacillus pleuropneumoniae* genes. The construction of such recombinant carriers can be done routinely, using standard molecular biology techniques such as homologous recombination. Therefore, in an even more preferred embodiment, the live attenuated bacteria of the species *Actinobacillus pleuropneumoniae* that do not produce a functional ApxIV toxin, have a heterologous gene inserted in the *apxIV* gene. Such a heterologous gene can, as mentioned above, e.g. be a gene encoding an antigen selected from other pathogenic micro-organisms or viruses. Another possibility is to insert a gene encoding a protein involved in triggering the immune system, such as an interleukine or an interferone.

In a still even more preferred form, the heterologous gene encodes one or more antigens selected from the group consisting of Porcine Reproductive Respiratory Syndrome (PRRS) virus, Pseudorabies virus, Porcine Influenza virus, Porcine Parvovirus, Transmissible Gastroenteritis virus, rotavirus, *Escherichia coli, Erysipelothrixrhusiopathiae, Pasteurella multocida, Bordetella bronchiseptica, Haemophilus parasuis* and *Streptococcus suis.*

There is however a serious pitfall in expression of heterologous genes in recombinant carriers: it is known that several proteins are toxic if they are expressed in heterologous bacteria. Therefore, genes encoding such proteins can never be introduced in heterologous carriers, since successful recombinants will eventually die as a result of the expression a certain amount of the heterologous gene. The P2-protein of *Haemophilus influenzae,* to name just one example, can simply not be expressed in *E. coli.* (Munson et al., Infect. & Immun. 57: 88-94 (1989)). It was unexpectedly found, that although the *apxIV* gene is efficiently expressed *in vivo (see Example 5),* it is not expressed *in vitro* (see Example 4). This was concluded from the failure to show the presence of ApxIV toxin in *in vitro* grown *A. pleuropneumoniae* cultures. This means that ApxIV expression is switched on or off, depending on the environment in which *A. pleuropneumoniae* is grown. This feature offers an unexpected advantage over known live recombinant carriers: if the expression of the heterologous gene is brought under the control of the *apxIV* promoter, the live attenuated P. pleuropneumoniae carrier according to the invention can be grown in vitro to high densities, regardless the inserted heterologous gene, since the foreign gene will not be expressed under these conditions. After administering a number of bacteria to the host, the expression of the heterologous gene will start and at some time during replication or after the death of the bacterium it will become available to the immune system of the host. The heterologous gene to be expressed can be functionally linked to the *apxIV* promoter by e.g. replacing the coding sequence of the apxIV gene by the coding region of the heterologous gene. It is not necessary to replace the whole *apxIV* gene: it suffices to replace the ATG-codon of ApxIV by the coding region of the heterologous gene including its stop-codon. It is also possible to express a heterologous gene under the influence of the *apxIV* promoter by making a fusion construct. This can be made by inserting the heterologous gene in frame with the *apxIV* reading frame downstream of the *apxIV* ATG codon.

The wording "functionally linked to the *apxIV* promoter" means that transcription of the heterologous gene starts at the apxIV promoter.

It goes without saying that each location of the inserted heterologous gene in which it is functionally linked to the *apxIV* promotor falls within the scope of the invention.

Therefore, the most preferred form of this embodiment relates to live attenuated bacteria, carrying a heterologous gene that is functionally linked to the promotor region of the *apxIV* gene.

The surprising finding that the native *apxIV* promotor is a switchable promotor that is switched off *in vitro* and switched on *in vivo* makes this promotor a very versatile expression tool both in it's natural host and as a heterologous promotor in other bacteria. When used as a heterologous promotor in other bacteria, the DNA comprising the promotor can be isolated from its host and transferred to a bacterium other than *Actinobacillus pleuropneumoniae.* Another option that has now become feasible, is the cloning of several copies of the *apxIV* promotor each controlling the expression of another gene. This can be done in the host bacterium *Actinobacillus pleuropneumoniae,* but this principle of multiple copies is equally applicable to other bacteria. As mentioned above, the promotor can be used for the selective *in vivo* expression of one or more heterologous genes encoding antigens selected from other pathogenic micro-organisms or viruses. The promotor can also be used for the expression of a heterologous DNA sequence encoding a cytokine such as an interleukin, Tumor Necrosis Factor or an interferon. Several cytokines, e.g. interferons are known to play an important role as immune modulators. Thus it may be advantageous to express such genetic information under the control of the *apxIV* promotor.

Therefore, another embodiment relates to a nucleotide sequence harbouring the promotor controling the expression of the *apxIV* gene.

The switchable promotor that in the native situation controls the expression of the *apxIV* gene, was now found to be located in the DNA fragment between position 451 and 1132 of SEQ ID NO: 5. It is clear, that those parts of this DNA fragment that are not essential promotor elements need not necessarily be present in the fragment. Thus, shorter fragments of this DNA fragment in which the promotor activity is retained, are equally suitable for the expression of heterologous genes. Therefore, a more preferred form of this embodiment relates to a nucleotide sequence comprising the DNA fragment from position 451 to 1132 of SEQ ID NO: 5 or a subfragment thereof still having promotor activity.

Bacterial promotors all share two consensus regions, the so-called -10 and the -35 region. Although the flanking sequence of these consensus regions may to a certain extend influence the efficiency of the promotor, it can be advantageous to use only that part of the promotor region that comprises the DNA fragment between -35 and the ATG codon. This DNA fragment is located between position 617 and position 641 of SEQ ID NO: 5. Therefore, in a more preferred form of this embodiment the invention relates to a nucleotide sequence comprising the DNA fragment from position 617 to 641 of SEQ ID NO: 5.

The present invention relates to ApxIV toxin as a subunit vaccine component.

Subunit vaccines will most probably comprise the three known Apx-toxins. This was mentioned above. Since it was unexpectedly found, that the ApxIV toxin is however present in all *A. pleuropneumoniae* serotypes as mentioned above, it is a desirable additional component of subunit vaccines: neutralising antibodies raised against the ApxIV toxin provide protection against the ApxIV toxin produced by each and every *Actinobacillus pleuropneumoniae* strain, regardless the serotype. Therefore, another embodiment of the invention relates to subunit vaccines for the protection of animals against infection with a bacterium of the species *Actinobacillus pleuropneumoniae,* that comprise purified ApxIV toxin. The ApxIV toxin can be administered alone, or in combination with any or all of the toxins Apxl, -II and -III mentioned above and/or e.g. in combination with Outer Membrane Proteins (OMPs) of *Actinobacillus pleuropneumoniae.* Such vaccines can easily be prepared by admixing ApxIV toxin in an amount sufficient to induce an immune response, and a pharmaceutically acceptable carrier. Production of the ApxIV toxin is possible by introducing the *apxIV* gene in a suitable expression vector, expression of the gene and isolation of the toxin. Many versatile expression systems are known in the art, such as bacterial expression systems, baculovirus expression systems and mammalian cell expression systems. In Example 3 it is described how to obtain the ApxIV toxin by expression of the gene in E. coli.

Still another embodiment of the invention relates to live attenuated vaccines comprising live attenuated bacteria as described above for the protection of animals against infection with a bacterium of the species *Actinobacillus pleuropneumoniae.* Such vaccines can be obtained by admixing live attenuated bacteria with a pharmaceutically acceptable carrier. These vaccines comprise at least an immunogenically effective amount of the live attenuated producing bacterium according to the invention. Immunogenically effective means that the amount of live attenuated bacterium administered at the moment of vaccination is sufficient to induce in the host an effective immune response to virulent forms of the RTX-toxin producing bacterium. The useful dosage to be administered will vary depending on the age, weight and animal vaccinated, the mode of administration and the type of pathogen against which vaccination is sought. The vaccine may comprise any dose of bacteria, sufficient to evoke an immune response. Doses ranging between 10³ and 10¹⁰ bacteria are e.g. very suitable doses.

The pharmaceutically acceptable carrier may be as simple as water, but it may e.g. also comprise culture fluid in which the bacteria were cultured. Another suitable carrier is e.g. a solution of physiological salt concentration. Other examples of pharmaceutically acceptable carriers or diluents useful in the present invention include stabilisers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).

Optionally, one or more compounds having adjuvant activity may be added to the vaccine. Adjuvantia are non-specific stimulators of the immune system. They enhance the immune response of the host to the invading pathogen. Examples of adjuvantia known in the art are Freunds Complete and Incomplete adjuvans, vitamin E, non-ionic block polymers, muramyldipeptides, ISCOMs (immune stimulating complexes, cf. for instance European Patent EP 109942), Saponins, mineral oil, vegetable oil, and Carbopol (a homopolymer). Adjuvantia, specially suitable for mucosal application are e.g. the E. coli heat-labile toxin (LT) or Cholera toxin (CT).

Other suitable adjuvants are for example aluminium hydroxide, phosphate or oxide, oil-emulsions (e.g. of Bayol F (R) or Marcol 52 (R), saponins or vitamin-E solubilisate.

Therefore, in a preferred form, the vaccines according to the present invention comprise an adjuvant.

For administration to animals, the vaccine according to the presentation can be given inter alia intranasally, intradermally, subcutaneously, by aerosol or intramuscularly.

There are several ways to store both subunits and live organisms. Storage in a refrigerator is e.g. a well-known method. Also often used is storage at -70°C in a buffer containing glycerol. Bacteria can also be kept in liquid nitrogen. Freeze-drying is another way of conservation. Freeze-dried bacteria can be stored and kept viable for many years. Storage temperatures for freeze-dried bacteria may well be above zero degrees, without being detrimental to the viability. Freeze-drying is equaiiy applicable for subunits.

Freeze-drying can be done according to all well-known standard freeze-drying procedures. Optional beneficial additives, such as e.g. skimmed milk, trehalose, gelatin or bovine serum albumin can be added in the freeze-drying process.

Therefore, in a more preferred embodiment, the vaccine according to the present invention is in a freeze-dried form.

In an even more preferred form of this embodiment, the vaccine additionally comprises one or more antigens selected from other pathogenic micro-organisms or viruses.

Such a vaccine can be obtained by adding one or more antigens selected from other pathogenic bacteria or viruses to the live attenuated bacterium according to the invention and a pharmaceutically acceptable carrier as described above.

Of course, it is possible to add not only one or more antigens, but also one or more of the whole pathogens as such, in an inactivated or live form.

It can alternatively be obtained by cloning the genetic information encoding one or more antigens selected from other pathogenic micro-organisms or viruses into the live attenuated bacterium, using known recombinant DNA technology as described above.

Such vaccines are of course less stressing for the animal to be vaccinated than separate vaccinations with each of the pathogens, both from a medical and a physical point of view.

In a still even more preferred form, these antigens are selected from the group consisting of Porcine Reproductive Respiratory Syndrome (PRRS) virus, Pseudo-rabies virus, Porcine Influenza virus, Porcine Parvovirus, Transmissible Gastroenteritis virus, rotavirus, *Escherichia coli, Erysipelothrix rhusiopathiae, Pasteurella multocida, Bordetella bronchiseptica, Haemophilus parasuis* and *Streptococcus suis.*

The invention also describes methods for the preparation of a live attenuated bacterium of the species *Actinobacillus pleuropneumoniae* that is not capable of producing a functional ApxIV toxin. These method comprise the introduction of a mutation in the gene encoding the apxIV protein. Both classical mutation techniques, using mutagenic agents, and recombinant DNA techniques well-known in the art for insertion, replacement or deletion of genetic information from the apxIV gene are applicable.

In a preferred form, the above mentioned methods are used for the introduction of a deletion.

Methods for the preparation of a live attenuated vaccine, that comprise admixing bacteria according to the invention with a pharmaceutically acceptable carrier are also described by the invention.

Also falling within the scope of the invention are methods for the preparation of a subunit vaccine. Such methods comprise the mixing of purified ApxIV toxin with a pharmaceutically acceptable carrier.

Another generally acknowledged problem in the field of vaccination with live vaccines is the following: the presence of antibodies against a certain pathogen in the serum of a host animal indicates that the host has been infected with the pathogen, either in a virulent or attenuated form. It is however impossible to discriminate between field-infected animals and animals vaccinated with a live vaccine strain. The live attenuated *Actinobacillus pleuropneumoniae* according to the present invention offers a solution to this problem as follows:
As described in Example 3, the *apxIV* gene of *Actinobacillus pleuropneumoniae* serotype 1 has been isolated and expressed in a heterologous host cell. This expression product was subjected to PAAGE-gelelectroforesis and then used for Western-blotting. The blots were incubated with convalescent serum obtained from a deliberately *Actinobacillus pleuropneumoniae*-infected pigs, and sera from field-strains. It was found, that the *apxIV* gene is expressed *in vivo* in all *Actinobacillus pleuropneumoniae* field strains tested. This implicates, that pigs infected with *Actinobacillus pleuropneumoniae* will always have antibodies against the strain with which they were infected, regardless the serotype of the infectious strain.

The live attenuated bacteria according to the present invention can, due to the deletion of the *apxIV* gene, no longer make ApxIV toxin. Therefore animals vaccinated with a live attenuated *Actinobacillus pleuropneumoniae* strain according to the invention will not have antibodies against ApxIV toxin in their serum.

In a comparative test, e.g. an ELISA test, such sera will therefore react with all immunogenic *Actinobacillus pleuropneumoniae*-proteins such as e.g. Apxl, II and/or III, but not with ApxIV. Sera from pigs infected with an *Actinobacillus pleuropneumoniae* field strain however will react with all immunogenic *Actinobacillus pleuropneumoniae*-proteins, including ApxIV. Therefore, the live attenuated *Actinobacillus pleuropneumoniae* according to the present invention turns out to be a very suitable marker vaccine, i.e. a vaccine strain that can be discriminated from a field strain.

A diagnostic test for the discrimination between vaccine strains and field strains can be a simple ELISA-test in which purified ApxIV toxin is coated to the wall of the wells of an ELISA-plate. Incubation with serum from pigs to be tested, followed by e.g. incubation with a labelled anti-pig antibody can then reveal the presence or absence of antibodies against ApxIV toxin.

Another example of a diagnostic test system is e.g. the incubation of a Western blot comprising purified ApxIV toxin with serum of pigs to be tested, followed by detection of specific anti-ApxIV antibodies.

Therefore, diagnostic test for the discrimination between sera from pigs infected with *Actinobacillus pleuropneumoniae* field strains and from pigs vaccinated with a vaccine comprising live attenuated vaccine *Actinobacillus pleuropneumoniae* strains according to the invention, that comprise purified ApxIV toxin. also fall within the scope of the invention.

Still another problem seen in pig health care is the following: It is difficult to determine in a both quick and unambiguous manner if a pig is infected with *Actinobacillus pleuropneumoniae* or *A. suis,* or possibly a combination of both. Diagnostic tests for the specific detection of A. suis are currently not available. This is mainly due to the fact that A. pleuropneumoniae and A. suis share so many antigens. As an example may serve, that two highly antigenic Apx-toxins; Apxl and Apxll have highly conserved homologues in e.g. A. suis (Van Ostaayen et al., submitted for publication).

The known RTX-genes, encoding the Apxl, -II and -III toxins or homologues are found in practically all members of the genus *Actinobacillus,* such as *A. pleuropneumoniae, A. suis, A. rossii* and *A. equuli.* Thus, it was initially assumed by the inventors, that the new RTX-toxin ApxIV would also be common to all members of the genus *Actinobacillus.*

It was however found after testing a the swine-pathogenic *Actinobacillus,* again surprisingly in contrast with the known three RTX-genes, that this novel RTX-gene *apxIV* is only present in the swine-pathogen *Actinobacillus pleuropneumoniae.* It is absent in all other common swine pathogenic *Actinobacillus* species, and therefore it is also absent in *Actinobacillus suis.* See Example 6 and 7.

Therefore, it was surprisingly noticed that the presence of antibodies against ApxIV in the serum of a pig is a quick and unambiguous proof that the pig has been infected with A. pleuropneumoniae, and not with A. suis or any other swine-pathogen *Actinobacillus* species.

Thus the present invention also provides a diagnostic test based on the presence or absence of antibodies against ApxIV, and therefore a discriminating test for specifically distinguishing an infection with *A. pleuropneumoniae* from an infection with *A. suis* Such a test can e.g. be an ELISA test that comprises in separate wells the Apxl and -II toxins, present in both *A. pleuropneumoniae* and *A. suis* and the purified ApxIV toxin. Serum from A. suis-infected animals will react only with the wells comprising the Apxl and -II whereas *A. pleuropneumoniae*-infected animals will also react with the well comprising the purified ApxIV toxin.

### Example 1:

### Cloning and analysis of the apxIV gene of A. pleuropneumoniae serotype 1.

Standard molecular biological procedures (plasmid DNA isolation, restriction digestion, agarose gel electrophoresis, Southern blotting, ligation, transformation, electroporation) were, unless stated otherwise, essentially performed as described in Sambrook et al. (Molecular Cloning, A Laboratory Manual, Cold Spring Harbor, N.Y., 1989) or Ausubel et al., (Current Protocols in Molecular Biology. John Wiley & Sons, N.Y., 1987). PCR was performed essentially as described in Innis et al., (PCR protocols, A guide to Methods and Applications, Academic Press Inc., San Diego, 1990). Chromosomal DNA isolation was performed according to Pitcher et al., (Lett. Appl. Microbiol., 8;151-156, 1989). The origin of all *A. pleuropneumoniae* reference strains (serotype 1: strain 4074; serotype 2: strain S1536: serotype 3: S1421; serotype 4 M62; serotype 5a: K17; serotype 5b: L20; serotype 6: femø; serotype 7: WF83; serotype 8: 405; serotype 9: CVI13261; serotype 10: 13039; serotype 11: 56153 and serotype 12: 8329) is described by Frey and Nicolet, (J. Clin. Microbiol., 28;232-236, 1990). *A. pleuropneumoniae* serotype 3 strain HV114 is a field isolate (i.e. one of the serotype 3 strains tested in Beck et al., J. Clin. Microbiol., 32;2749-2754, 1994). Other *Actinobacillus* strains used; *A*. *rossii:* ATCC 27072; *A. equuli:* ATCC 19392; *A. suis:* ATCC 15558. *Pasteurella haemolytica* type 1 strain ATCC 14003 was used.

*E. coli* host strains used: XL1-blue (Stratagene, La Yolla, Ca; genotype: *recA1 endA1 gyrA96 thi-1 hsdR17 supE44 relA1 lac* [F' *proAB lacl^{q}ZDM15* Tn10 (Tet^{r})]) and HMS174(DE3) (AMS Biotechnology Ltd, Switzerland; genotype: F⁻ *recA* (*r_{K12-} m*_{*K12*+}) rif^{r} IDE3).

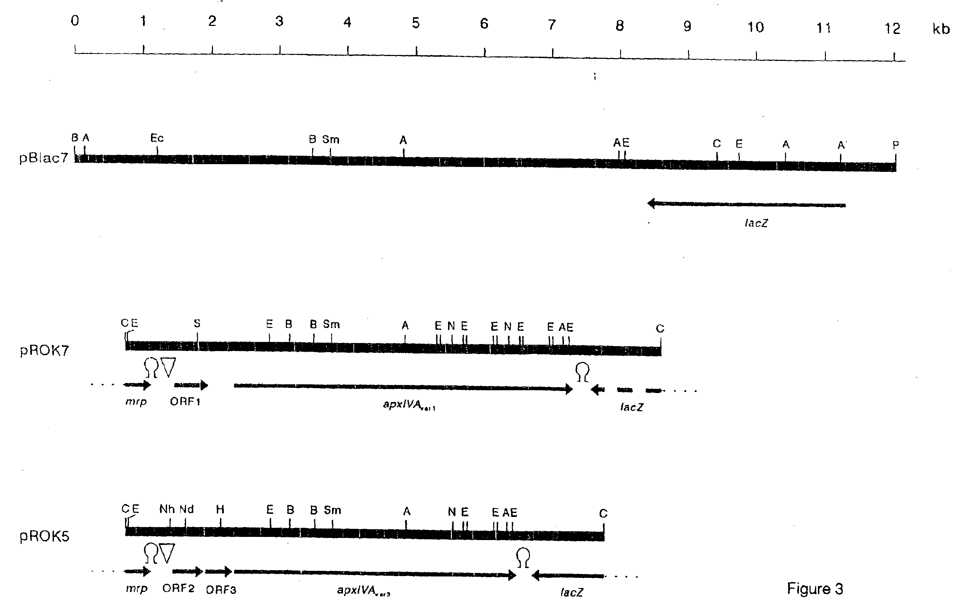

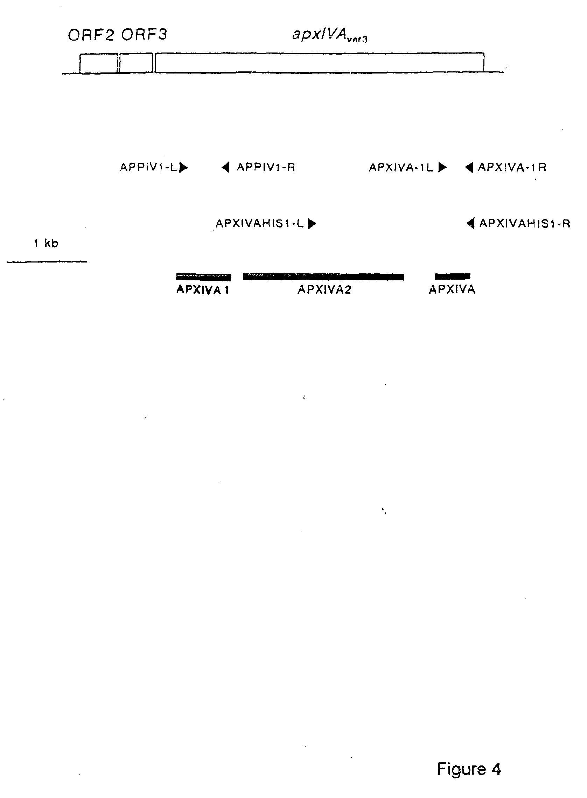

On the basis of the preliminary sequence data obtained from the thesis of T.J. Anderson (University of Guelph, 1995), two primers, designated APXIVA-1L (5'-TGGCACTGACGGTGATGA-3') and APXIVA-1R (5'-GGCCATCGACTCAACCAT-3'), were synthesised. These primers were used in a PCR amplification, with chromosomal DNA from *A. pleuropneumoniae* serotype 3 strain HV114 and serotype 1 reference strain 4074 as a template. With both strains a fragment of 442 bp was amplified. The fragment derived from the serotype 3 chromosomal DNA was labelled with Digoxigenin-11-dUTP (Boehringer Mannheim) according to the protocol of the manufacturer (this fragment was designated probe APXIVA, see fig. 4). The labelled probe was subsequently used to hybridize a Southern blot containing Clal digested chromosomal DNA from strain 4074. The probe hybridised with a fragment of approximately 8.0 kb. The *apx*I*V* gene from serotype 1 strain 4074 was isolated by ligating *Cla*I digested chromosomal DNA into *Cla*I digested pBluescript II SK⁻ (Stratagene USA). *E. coli* strain XL1-blue was transformed with the ligated DNA and transformants were selected on an LB plate with 100 mg/ml of ampicillin. Clones harbouring the *apx*I*V* were selected by colony hybridisation of a nitrocellulose replica of the plate with the APXIVA probe. Thus, a plasmid designated pROK7 was isolated and shown to harbour a *Cla*I insert of approximately 8 kb. The first 6736 bp of the *Cla*I insert were sequenced (SEQID 1) and an open reading frame of 4971 nucleotides was identified encoding a protein of 1657 amino acid residues (SEQID 2) with a predicted size of approximately 186 kD. The gene was designated *apxIV_var1* (see fig. 3).

### Example 2:

### Cloning and analysis of the apxIV gene of A. pleuropneumoniae serotype 3.

The labelled probe APXIVA (mentioned in example 1) was used to hybridize a Southern blot containing *Cla*I digested chromosomal DNA from strain HV114. The probe hybridised with a fragment of approximately 7.0 kb. The isolated chromosomal DNA from HV114 was digested with *Cla*I, and ligated with *Cla*I digested pBluescript II SK⁻ (Stratagene USA). *E*. *coli* strain XL1-blue was transformed with the ligated DNA and transformants were selected on an LB plate with 100 mg/ml of ampicillin. Clones harbouring the *apx*I*V* were selected by colony hybridisation of a nitrocellulose replica of the plate with the APXIVA probe. Thus, a plasmid designated pROK5 was isolated and shown to harbour a *Cla*I insert of approximately 7 kb. The insert was analysed by sequence analysis (SEQID 3). An open reading frame of 4146 bp was identified encoding a protein of 1382 amino acid residues (SEQID 4), with a predicted size of approximately 154 kD. The gene was designated *apxIV_var3* (see fig. 3) .

### Example 3:

### EXPRESSION OF ApxIV var3-polyhistidine fusion proteins in E. coli

From plasmid pROK5, a deletion clone was made which contains the 3' end of the *apxIV* gene, starting at the *Bam*HI site (nucleotide No. 2747 in SEQ ID No: 3) up to the *Cla*I site at the end of the insert downstream of the *apxIV* gene. This plasmid was designated pROK1. Using oligonucleotides APXIVAHIS1-L (5'-AGCCATATGGGCGATTTAAATTTCAG-3') and APXIVAHIS1-R (5'-TATGGATCCTCCGTGCTTCTGAGC-3') and DNA from plasmid pROK1 as a template, a DNA fragment of 2.1 kb was amplified (see fig. 4) containing the region from bp 3520 to 5643 in *apxIV*_*var3* (SEQID 3) flanked with *Nde*I and *Bam*HI restriction sites at the 5' and 3' end respectively. After cloning of the *Nde*I/*Bam*HI digested PCR fragment in expression vector pETHIS-1, digested with the same enzymes, a plasmid designated pJFFapxlV6/10his-1 was obtained. Plasmid pETHIS-1 is a derivative of pET14b (Novagen Inc., Madison, Wi.) where the multiple cloning site has been extended and a region encoding a histidine decamer has been inserted. Consequently, The pJFFapxIV6/10his-1 plasmid contains a translational fusion encoding a histidine hexamer, followed by amino acid residues 653 up to 1360 from SEQID 4, followed by a histidine decamer, under the control of a T7 promoter. The plasmid was transferred to *E*. *coli* strain HMS174(DE3) with pLysS, which contains an IPTG inducible T7 RNA polymerase gene as well as the T7 lysozyme gene for increased stability. The strain was grown in LB medium containing 25 mg/ml of chloramphenicol and 100 mg/ml of ampicillin, up to an OD₆₅₀ of 0.5, and induced with isopropyl-b-D-thiogalactopyranoside at a concentration of 10 mM. After the addition of IPTG, the cells were incubated at 37°C for 2.5 hours, the cells were harvested by centrifugation, and fusion protein with the expected size of 80 kD was isolated in the form of inclusion bodies. The inclusion bodies were solubilized in a solution of 6M guanidine hydrochloride, 300 mM NaCl and 50 mM NaH₂PO₄ at pH 8.0 and the 80 kD fusion protein was further purified by Immobilised Metal Affinity Chromatography (IMAC) (Schmitt et al., Molecular Biology Reports 18;223-230, 1993) using Ni²⁺ chelated columns (Qiagen AG, Basel). Pure protein was eluted from the column at pH 5.0. Pooled fractions were dialysed against a solution of 300 mM NaCl and 50 mM NaH₂PO₄ at pH 8.0. A rabbit was immunised with 500 mg of the polyhistidine fusion product, mixed with 1 volume of Complete Freunds Adjuvant (Difco Labs, Detroit, Ml). A booster dose of the same amount, mixed with incomplete Fre- und Adjuvant was given 3 weeks later. Four weeks after the booster, the rabbit was bled and a hyperimmune serum comprising anti-ApxIV toxin antibodies , designated serum 522-409, was obtained.

### Example 4:

### Expression of apxIV genes in in vitro grown A. pleuropneumoniae

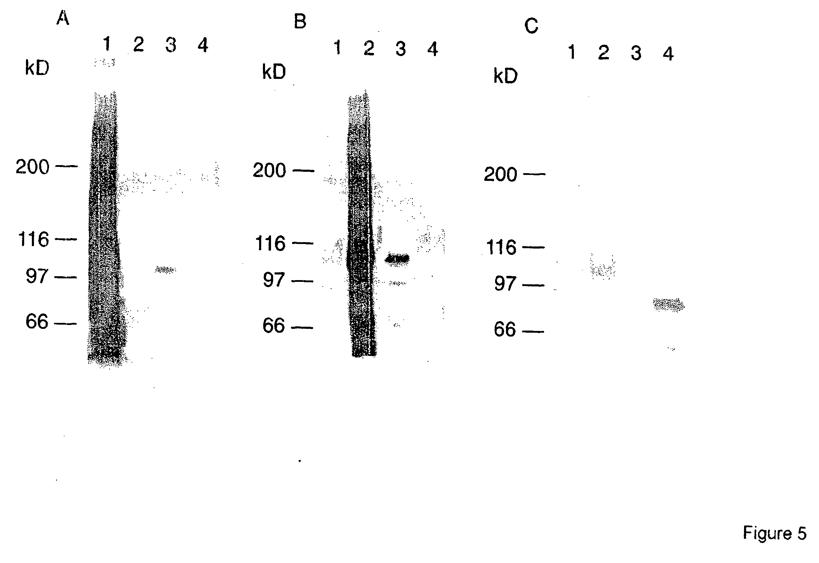

The *A. pleuropneumoniae* reference strain from serotype 1 was grown in Columbia broth supplemented with 10 mg/ml of b-NAD and harvested as described (Beck et al., J. Clin. Microbiol., 32;2749-2754, 1994). Adjacent to lanes comprising ApxIA, ApxIIA and ApxIVA-polyhistidine fusion proteins the concentrated culture supernatant was separated by polyacrylamide gel electrophoresis (Laemmli, Nature 227:680-685, 1970) and subjected to a Western blotting procedure (Towbin et al., Proc. Natl. Acad. Sci. USA 76:4350-4354, 1979). The Western blot was reacted with anti-ApxIA- and anti-ApxIIA monoclonal antibodies as described by Beck et al., (J. Clin. Microbiol., 32;2749-2754, 1994), and with anti-ApxIV serum 522-409 (see example 3). The isolated RTX toxin fraction of serotype 1 clearly contains ApxlA and ApxIIA. The presence of ApxIVA could not be demonstrated (see fig. 5).

### Example 5:

### Expression of apxIV genes in A. pleuropneumoniae in vivo during infection

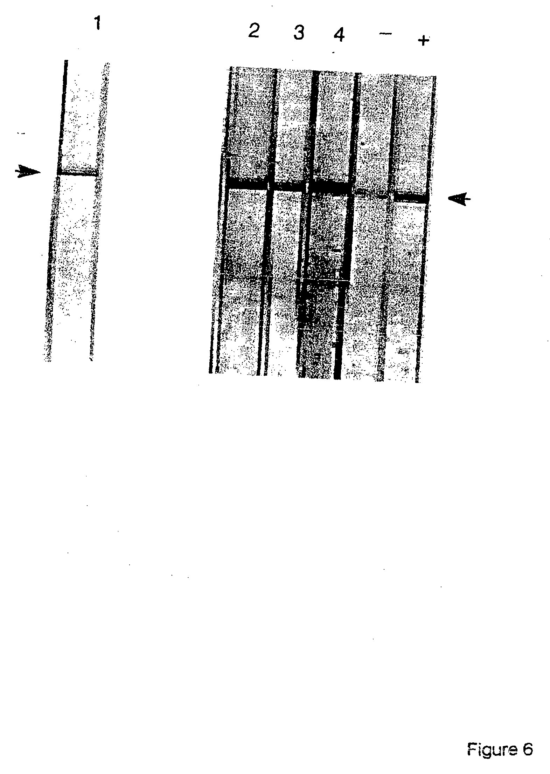

A polyacrylamide gel containing the 80 kD polyhistidine-ApxIV_var3 fusion protein (see example 3) was transferred to a nitrocellulose membrane. The membrane was divided into strips which were reacted with (100-fold dilutions of) convalescent field sera against serotype 1 or sera from a pig, experimentally infected with the serotype 1 reference strain (Frey and Nicolet, Vet. Microbiol., 28;61-73, 1991). The reaction was visualised using alkaline phosphatase-labelled conjugate against rabbit IgG (Kirkegaard Perry Inc., Gaithers-burg, Md.) and NBT (4-Nitrobluetetrazolium chloride) and BCIP (5-Bromo-4-chloro-3-indolyl-phosphate) colour development (see fig. 6). The serotype 1 field sera, as well as serum from the experimentally infected pig react with the 80 kD polyhistidine-ApxIV_var3 protein. This indicates that the ApxIV protein actually is expressed, is antigenic and induces anti-ApxIV toxin antibodies during *A. pleuropneumoniae* infection in pigs.

### Example 6:

### Presence of apxIV genes in all A. pleuropneumoniae serotypes and the absence thereof in non-pleuropneumoniae Actinobacillus-strains using Southern blotting

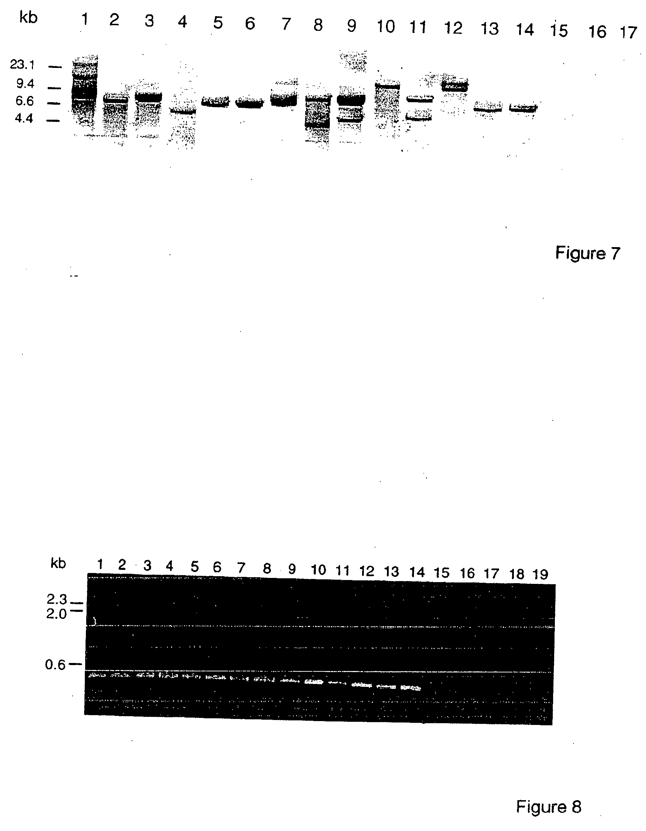

To investigate the presence of the *apx*I*V* gene in the various *A.pleuropneumoniae* serotypes and related bacteria, three probes were made (see fig. 4). Probe APXIVA is described in example 1. Probe APXIVA2 contains the 2015 bp DNA fragment between the *Bam*HI and *Nru*I sites. The 758 bp probe APPIVA1 was made by PCR amplification with oligos APPIV1-L (5'-GGGACAGTGGCTCAATTAAG-3') and (APPIV1-R (5'-AGCTGTAAACTCCACCAACG-3'). All probes were labelled with Digoxigenin-11-dUTP (Boehringer Mannheim) according to the protocol of the manufacturer and hybridised with Southern blots containing *Cla*I digested chromosomal DNA of all *A. pleuropneumoniae* reference strains and the HV114 field strain, *Actinobacillus suis* (ATCC 15558), *Actinobacillus rossii* (ATCC 27072) and *Actinobacillus equuli* (ATCC 19392). All three probes react similarly (see fig. 7 for the results with the APXIVA2 probe). All *A. pleuropneumoniae* strains react, whereas no hybridisation is observed with the *A*. *suis, A. equuli* and *A. rossii* strains.

### Example 7:

### Presence of apxIV genes in A. pleuropneumoniae and related strains using PCR amplification

With 50 ng of chromosomal DNA from the various *A. pleuropneumoniae* serotypes, other *Actinobacillus* species and *P. haemolytica* as templates, and primers APXIVA-1 L (5'-TGGCACTGACGGTGATGA-3') and APXIVA-1R (5'-GGCCATCGACTCAACCAT-3') PCR amplification was performed. After analysis of the products on an agarose gel, products with the expected size of 442 bp were observed in all *A. pleuropneumoniae* samples, but in none of the other *Actinobacillus* species (fig. 8). This indicates that in addition to the results in example 6, also PCR could be used to discriminate *A. pleuropneumoniae* from other *Actinobacillus* species.

### Example 8:

### Overexpression of ApxIV-var1 polyhistidine fusion protein.

Starting with plasmid pROK-7 (see example 1) as a template and oligonucleotides APX4/115-L (5'-CGCCATATGACAAAATTAACTATGCAAG) and APX4116-R (5'-CGCGAAT TCAGCGACACAAGAGATAT) as PCR-primers, a PCR fragment was amplified. A sufficient amount of this fragment was then digested with restriction enzymes Ndel and EcoRI and cloned in expression vector pETHIS-1, digested with the same enzymes as described in Example 3. From the resulting plasmid, designated pJFFApxIVA1His1, a 206 kD polyhistidine fusion protein (MW determined in PAGE) of 1841 amino acid residues was overexpressed in E. coli as described in Example 3. The protein is encoded in the coding region spanning nucleic acid no. 1132 to 6546 as depicted in SEQ ID NO: 5. The amino acid sequence of the protein in given in SEQ ID NO: 6. In Western blot this product was shown to react with specific anti-ApxIV serum 522-409 (antiserum described in example 3).

### Example 9:

### Protection of mice by vaccination with ApxIV against APP challenge.

The 206 kD polyhistidine-ApxIV fusion protein as described in Example 8 and a comparable 108 kD polyhistidine-ApxIA-fusion protein, both derived from serotype 1 reference strain 4074 genomic material, were overexpressed as described in example 3. The cell pellet of induced E. coli cells was resuspended into PBS buffer (1 g. cell pellet in 6 ml buffer) and sonicated on ice for two times 45 seconds on ice for lysis of the cells. After centrifugation for 20 minutes at 22.000 x g at 4°C, the supernatant was discarded and the resulting pellet was washed with a solution of 3M urea (pH 6.3). The urea was removed after centrifugation for 20 minutes at 22.000 x g, and the resulting pellet was solubilized in 6M GuanidiniumHCl (pH 8.0). The protein samples were standardised by specific protein content after densitometry of PAGE gels. The samples were diluted with PBS and formulated with an oil adjuvant.

Three groups of 15 mice each, were intraperitoneally immunised with the ApxIV antigen (36.3 microgram), ApxIA antigen (36.3 microgram), or the adjuvant alone. The vaccines were administered in a volume of 0.4 ml. Twenty-four days after the first vaccination, each group of mice was split in two groups of 7 and 8 mice which were boosted with half the amount of antigen. The groups of 7 mice were boosted intraperitoneally in a volume of 0.2 ml and the groups of 8 mice were vaccinated intramuscularly with 0.1 ml in each hind leg. Thirteen days after the booster, the mice were challenged intraperitoneally with 1.5 10⁸ cfu of a virulent serotype 1 strain.

### Example 10:

### Poreforming capacity of ApxIV:

Freshly induced E. coli cells expressing ApxIV were used as the source of protein for testing poreformation in artificial lipid bilayers as described by Maier et al., Infect. Immun., 64; 4415-4423(1996). The methods used for black lipid bilayer experiments have been described previously (Benz et al.; Biochim. Biophys. Acta 511: 305-319 (1978)). Membranes were formed from a 1% solution of asolectin (soybean lecitin type IV-S from Sigma, St. Louis MO) in n-decane. Zero current membrane potentials were measured with a Keithley 610 C electrometer 5-10 min. after a 10-fold salt gradient was established across the membranes (Benz et al.; Biochim. Biophys. Acta 551: 238-247 (1979)). The presence of ApxIV resulted in a high frequency of pore formation in the presence of 0.5 % cholesterol, with an average single channel conductance (G) of 4 nS.

These results indicate that the ApxIV induces pores into artificial bilayers and is therefore toxic to eukaryotic cells and thus is a virulence factor for A. pleuropneumoniae.

### Legend to the figures:

Figure 1: Comparison of ApxIVAvar1 and ApxIVAvar3 (var stands for serotype). Graphic representation of the different features found in the C-terminal end. Dashed boxes represent the direct repeat regions in ApxIVA. Bold vertical bars indicate the position of glycine rich nonapeptides, and DNA polymerase family B signatures are indicated by black triangles. The amino acid sequence YSDSANSKK represents the spacer sequence in the ApxIVAvar3 gene. The sequence segment of ApxIVAvar1 which is deleted in ApxIVAvar3 is also indicated in the figure.
figure 2: Alignment of the amino acid sequences of direct repeat 1 (A), direct repeat 2 (B) and direct repeat 3 (C) of ApxIVAvar1 and ApxIVAvar3. The copies of each of the direct repeats 1, 2 and 3 are labeled by letters to distinguish them for the sequence comparison. Variant residues are shown in bold letters.
Figure 3: Partial restriction maps of pBLac7 (Thesis of T.J. Anderson University of Guelph, 1995), pROK7 and pROK5. The different open reading frames (ORF's) are indicated by arrows. The interrupted arrow of *lacZ* in pROK7 indicates partial sequencing of the gene. Potential *rho*-independent transcription terminators are indicated (W). Potential transcription start sites are indicated by a triangle. Restriction sites: A= *Asp*700; B= *Bam*HI; C= *Cla*I*;* E= *Eco*RV; Ec= *Eco*RI; H= *Hin*dIII; N= *Nru*I*;* Nd= *Nde*I; Nh= *Nhe*I; P= *Pst*I; S= *Spe*I; Sm= *Sma*I.
Figure 4: Location of the various oligonucleotides and probes on the map of the *apxIVAvar3* gene.
Figure 5: Expression of ApxIV in *in vitro* cultivated serotype 1 reference strain 4074. Panel A was reacted with the anti-ApxlA monoclonal antibody, panel B with anti-ApxIIA monoclonal antibody and panel C was reacted with anti-ApxIVA serum 522-409. Lane 1 contains ApxIA-polyhistidine fusion protein, lane 2 contains ApxIIA-polyhistidine fusion protein, lane 3 contains strain 4074 concentrated culture supernatant, lane 4 contains ApxIVA polyhistidine fusion protein.
Figure 6: Immunoblot showing the reactivities of sera from pigs which were experimentally infected with the reference strain of serotype 1 (lane 1) or pig sera from serotype 1 field infections (lane 2-4) with the 80 kD polyhistidine-ApxIV fusion protein. As a positive control (+), serum 522-409 was used, as a negative control (-), polyclonal rabbit serum against Apxl and ApxII (Frey et al., Infect. Immun., 57;2050-2056, 1989) was used as a 1000-fold diiution.
Figure 7: Southern blot of *Cla*I digested genomic DNA hybridised with probe APXIVA2. Lanes 1-13: *A. pleuropneumoniae* reference strains; 1: serotype 1; 2: serotype 2; 3: serotype 3; 4: serotype 4; 5: serotype 5a; 6: serotype 5b; 7: serotype 6; 8: serotype 7; 9: serotype 8; 10: serotype 9; 11: serotype 10; 12: serotype 11; 13: serotype 12; 14: HV114 field strain; 15: *A. suis* (ATCC 15558); 16: *A. rossii* (ATCC 27072); 17: *A. equuli* (ATCC 19392). Molecular size markers are indicated (in kilobasepairs) on the left.
Figure 8: PCR amplification of *apxIV* using primers APXIVA-1 L and APXIVA-1 R. Lane assignments: lanes 1 to 13 contain the *A. pleuropneumoniae* reference strains from serotypes 1, 2, 3, 4, 5a, 5b, 6, 7, 8, 9, 10, 11 and 12 respectively; lane 14: strain HV114; lane 15: *A. suis* ATCC 15558; lane 16: *A. rossii* ATCC 27072; lane 17: *A. equuli* ATCC 19392; lane 18: *A. lignieresii* ATCC 49236; lane 19: *P. haemolytica* type 1 ATCC 14003. Molecular size markers (in kilobasepairs) are indicated on the left.

## Claims

1. Subunit vaccine for the protection of animals against infection with a bacterium of the species *Actinobacillus pleuropneumoniae,* **characterised in that** said vaccine comprises purified ApxIV toxin and a pharmaceutically acceptable carrier.

2. Vaccine according to claim 1, **characterised in that** it comprises an adjuvant.

3. Vaccine according to claim 1 or 2, **characterised in that** the vaccine is in a freeze-dried form.

4. Vaccine according to claims 1-3, **characterised in that** it additionally comprises one or more antigens from pig-pathogenic micro-organisms or viruses.

5. Vaccine according to claim 4, **characterised in that** it additionally comprises one or more antigens selected from the group consisting of Porcine Reproductive Respiratory Syndrome (PRRS) virus, Pseudorabies virus, Porcine Influenza virus, Porcine Parvovirus, Transmissible Gastroenteritis virus, rotavirus, *Escherichia coli, Erysipelothirx rhusiopathiae, Pasteurella multocida, Bordetella bronchiseptica, Haemophilus parasuis* and *Streptococcus suis.*

6. Method for the preparation of a vaccine according to claim 1-5, said method comprising admixing purified ApxIV toxin with a pharmaceutically acceptable carrier.

7. Diagnostic test for distinguishing *Actinobacillus pleuropneumoniae* infection in pigs from *A. suis* infection in pigs, **characterised in that** said test comprises purified ApxIV toxin.

8. A method of detecting the presence or absence of all serotypes of *Actinobacillus pleuropneumoniae* in pigs comprising the steps of:
- providing purified ApxIV toxin,
- providing a sample from a pig, wherein the sample is serum,
- incubating the ApxIV toxin with the sample, and
- detecting the presence or absence of antibodies against the ApxIV toxin.

9. A diagnostic test for distinguishing between pigs infected with field strains of *Actinobacillus pleuropneumnoiae* and pigs vaccinated with a vaccine having live attenuated bacterium of the species *Actinobacillus pleuropneumnoiae,* wherein the vaccine produces no functional ApxIV toxin, the test comprising purified ApxIV toxin coated to wells of an ELISA-plate.

10. The diagnostic test of claim 9 further comprising a labeled anti-pig antibody that can reveal the presence or absence of antibodies against ApxIV toxin.

## Patentansprüche

1. Spaltimpfstoff für den Schutz von Tieren gegen Infektion mit einem Bakterium der Art *Actinobacillus pleuropneumoniae,* **dadurch gekennzeichnet, dass** der Impfstoff aufgereinigtes ApxIV-Toxin und einen pharmazeutisch unbedenklichen Träger umfasst.

2. Impfstoff nach Anspruch 1, **dadurch gekennzeichnet, dass** er ein Adjuvans umfasst.

3. Impfstoff nach Anspruch 1 oder 2, **dadurch gekennzeichnet, dass** der Impfstoff in gefriergetrockneter Form vorliegt.

4. Impfstoff nach den Ansprüchen 1-3, **dadurch gekennzeichnet, dass** er zusätzlich ein oder mehrere Antigene von schweinepathogenen Mikroorganismen oder Viren umfasst.

5. Impfstoff nach Anspruch 4, **dadurch gekennzeichnet, dass** er weiterhin ein oder mehrere Antigene aus der Gruppe PRRS-Virus (Virus des porcinen reproduktiven und respiratorischen Syndroms), Aujeszky-Virus, Schweinegrippevirus, porcines Parvovirus, Transmissible-Gastroenteritis-Virus, Rotavirus, *Escherichia coli, Erysipelothirx rhusiopathiae, Pasteurella multocida, Bordetella bronchiseptica, Haemophilus parasuis* und *Streptococcus suis* umfasst.

6. Verfahren zur Herstellung eines Impfstoffs nach den Ansprüchen 1-5, wobei das Verfahren umfasst, dass man aufgereinigtes ApxIV-Toxin mit einem pharmazeutisch unbedenklichen Träger vermischt.

7. Diagnostischer Test zum Unterscheiden von *Actinobacillus-pleuropneumoniae*-Infektion beim Schwein und A. suis-Infektion beim Schwein, **dadurch gekennzeichnet, dass** der Test aufgereinigtes ApxIV-Toxin umfasst.

8. Verfahren zum Nachweisen des Vorliegens bzw. Fehlens von allen *Actinobacillus-pleuropneumoniae-*Serotypen beim Schwein, umfassend die folgenden Schritte:
- Bereitstellen von aufgereinigtem ApxIV-Toxin,
- Bereitstellen einer Probe von einem Schwein, wobei es sich bei der Probe um Serum handelt,
- Inkubieren des ApxIV-Toxins mit der Probe und
- Nachweisen des Vorliegens oder Fehlens von Antikörpern gegen das ApxIV-Toxin.

9. Diagnostischer Test zum Unterscheiden zwischen mit Feldstämmen von *Actinobacillus pleuropneumnoiae* infizierten Schweinen und mit einem Impfstoff mit lebenden attenuierten Bakterien der Art *Actinobacillus pleuropneumnoiae* geimpften Schweinen, wobei der Impfstoff kein funktionelles ApxIV-Toxin produziert, wobei der Test mit aufgereinigtem ApxIV-Toxin beschichtete Näpfchen einer ELISA-Platte umfasst.

10. Diagnostischer Test nach Anspruch 9, der weiterhin einen markierten anti-Schwein-Antikörper, mit dem das Vorhandensein oder Fehlen von Antikörpern gegen ApxIV-Toxin aufgezeigt werden kann, umfasst.

## Revendications

1. Vaccin sous-unitaire destiné à la protection d'animaux contre une infection par une bactérie issue de l'espèce *Actinobacillus pleuropneumoniae,* **caractérisé en ce que** ledit vaccin comprend une toxine ApxIV purifiée et un véhicule pharmaceutiquement acceptable.

2. Vaccin selon la revendication 1, **caractérisé en ce qu'**il comprend un adjuvant.

3. Vaccin selon la revendication 1 ou 2, **caractérisé en ce que** le vaccin est sous forme lyophilisée.

4. Vaccin selon les revendications 1-3, **caractérisé en ce qu'**il comprend en outre un ou plusieurs antigènes issus de microorganismes ou de virus pathogènes du porc.

5. Vaccin selon la revendication 4, **caractérisé en ce qu'**il comprend en outre un ou plusieurs antigènes choisis dans le groupe constitué par le virus du Syndrome Reproducteur et Respiratoire Porcin (PRRS), le virus de Pseudo-rage, l'Influenzavirus Porcin, le Parvovirus Porcin, le virus de Gastroentérite Transmissible, le rotavirus, *Escherichia coli, Erysipelothrix rhusiopathiae, Pasteurella multocida, Bordetella bronchiseptica, Haemophilus parasuis* et *Streptococcus suis.*

6. Méthode de préparation d'un vaccin selon les revendications 1-5, ladite méthode comprenant le mélange d'une toxine ApxIV purifiée et d'un véhicule pharmaceutiquement acceptable.

7. Test de diagnostic destiné à distinguer une infection par *Actinobacillus pleuropneumoniae* chez le porc d'une infection par *A*. *suis* chez le porc, **caractérisé en ce que** ledit test comprend une toxine ApxIV purifiée.

8. Méthode de détection de la présence ou de l'absence de tous les sérotypes d'*Actinobacillus pleuropneumoniae* chez le porc, comprenant les étapes consistant à :
- fournir de la toxine ApxIV purifiée,
- fournir un échantillon issu d'un porc, où l'échantillon est du sérum,
- incuber la toxine ApxIV avec l'échantillon, et
- détecter la présence ou l'absence d'anticorps contre la toxine ApxIV.

9. Test de diagnostic afin de distinguer des porcs infectés par des souches sauvages d'*Actinobacillus pleuropneumoniae,* de porcs vaccinés par un vaccin ayant une bactérie atténuée vivante de l'espèce *Actinobacillus pleuropneumoniae,* où le vaccin ne produit aucune toxine ApxIV fonctionnelle, le test comprenant de la toxine ApxIV purifiée revêtue sur des puits d'une plaque ELISA.

10. Test de diagnostic selon la revendication 9, comprenant en outre un anticorps anti-porc marqué pouvant révéler la présence ou l'absence d'anticorps contre la toxine ApxIV.


### SEQUENCE LISTING

(1) GENERAL INFORMATION:
   (i) APPLICANT:
      (A) NAME: AKZO Nobel N.V.
      (B) STREET: Velperweg 76
      (C) CITY: Arnhem
      (E) COUNTRY: The Netherlands
      (F) POSTAL CODE (ZIP): 6824 BM
   (ii) TITLE OF INVENTION: Live attenuated Actinobacillus pleuropneumoniae
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 6736 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Actinobacillus pleuropneumoniae
      (B) STRAIN: 4074 (serotype 1 reference strain)
   (vii) IMMEDIATE SOURCE:
      (B) CLONE: pROK7
   (ix) FEATURE:
      (A) NAME/KEY: CDS
      (B) LOCATION:1576..6549
      (D) OTHER INFORMATION:/codon start= 1576
         /function= "RTX toxin"
         /product= "ApxIV-var1"
         /gene= "apxIV_var1"
         /number= 1
   (xi) SEQUENCE DESCRIPTION: SEQ ID NO: 1:
   ATCGATATGC CGCCGGGTAC GGGCGATATC CAACTTACTC TTTCGCAACA AATTCCGGTT      60
   ACCGGTGCGG TGGTGGTAAC CACTCCGCAA GATATTGCGT TATTAGATGC GGTGAAAGGT     120
   ATTTCAATGT TCCAAAAAGT GTCGGTACCG GTCTTAGGTA TCATTGAAAA TATGAGCGTA     180
   CATATCTGCC AAAATTGCGG TCACCACGAA GATATTTTCG GCACCGGCGG TGCGGAGAAA     240
   GTGGCGAAGA AATACGGTAC TAAAGTATTA GGACAAATGC CGTTGCATAT TCGCTTACGT     300
   CAAGATTTGG ATGCCGGCAC ACCGACCGTC GTTGCGGCAC CGGAACACGA AACCAGCCGA     360
   GCCTATATTG AATTAGCGGC AAAAGTCGCT TCGGAATTAT ACTGGCAAGG TTCGGTTATC    420
   CCGTCTGAAA TTATGATTCG TGAAGTAAAA TAAGTTTTAA TAACCACGAA AACACAAAGA    480
   ACACAAGCGG TAGAATTTGC AGAAAAATTT GCAAATCCTA CCGCTTTTTT ATTAGTACGA    540
   TTCGCTGTTG GACTGCTATT TGATTTGGTT TGTCAGGATA TTATGTTATT GTAATGAAAT    600
   GTTAGTGAAT TATTTTTATT AATTTGAAAG GAAACAAAAT GAAAATAAAA AAACGTTACA    660
   TTGCGCTGTT GGTCTTAGGT GTCGTTATCA GCTATGCCTG GTATCAAAAT TATCAATGGG    720
   AACAGCTGAT GTTAAGCGGT TATTGTGAAA AGGACGGAAG TTATTTTGAT GATAGGCATA    780
   CGAAGCAAGA ACTGATTGAT AGGGCAATTA ACTATATGCT GGAGCATCAA TCTAAAAAAA    840
   CATACGATGC TTATACTGAT GAACCTTTAG AAATAAAACC ATATTTAACA ATAGAGGAAT    900
   TTAAAAAACT CAATCCAAAT TGTTGTGAAA TTACCTCATG GCCAGCAGAT GCAGTTCCAC    960
   AAGATTGGGA TGTTCGTGTG GAAGGTAAGG CATATAGGTA TGTAATCGTA AAATATTTAA   1020
   GAACCTTAGC AAATAGAGAA CCTGAACGAT GGGAAACTAG TATTGTTTTT GATAATTGCG   1080
   GCAATCCTAA AAGAGCAAGC TACTTATATT ATTTAAAGAG AGAAATTTAT TATGACAAAA   1140
   TTAACTATGC AAGATGTGAC CAATTTATAT TTATATAAAA CGAAAACTCT ACCTAAAGAT   1200
   AGATTGGATG ATTCACTTAT TTCTGAAATA GGAAAAGGAG ATGATGATAT TGATAGAAAA   1260
   GAATTTATGG TGGGGCCGGG ACGTTTTGTG ACCGCTGATA ACTTTAGCGT TGTAAGAGAT   1320
   TTTTTTAATG CTGGGAAATC ACGCATTATT GCGCCGCAAG TCCCGCCTAT TCGTTCACAG   1380
   CAGGAAAAAA TCTTGGTCGG TTTAAAACCG GGCAAATATT CCAAAGCGCA GATATTGGAA   1440
   ATGCTGGGTT ATACGAAAGG CGGAGAAGTG GTAAATGGCA TGTTTGCCGG TGAAGTCCAG   1500
   ACATTAGGCT TTTATGACGA TGGCAAAGGG GATTTACTCG AACGCGCCTA TATCTGGAAT   1560
   ACCACAGGAT TTAAA ATG AGC GAC AAT GCC TTT TTT GTT ATA GAA GAA TCA    1611
                    Met Ser Asp Asn Ala Phe Phe Val Ile Glu Glu Ser
                      1               5                  10
   GGC AAA CGC TAT ATT GAA AAC TTT GGT ATT GAA CCT CTT GGT AAG CAA     1659
   Gly Lys Arg Tyr Ile Glu Asn Phe Gly Ile Glu Pro Leu Gly Lys Gln
            15                  20                  25
   GAA GAT TTT GAT TTT GTC GGC GGC TTT TGG TCT AAC TTA GTG AAT CGT     1707
   Glu Asp Phe Asp Phe Val Gly Gly Phe Trp Ser Asn Leu Val Asn Arg
        30                  35                  40
   GGT TTG GAA AGT ATT ATC GAC CCA TCC GGT ATC GGT GGA ACG GTA AAC     1755
   Gly Leu Glu Ser Ile Ile Asp Pro Ser Gly Ile Gly Gly Thr Val Asn
    45                  50                  55                  60
   CTT AAC TTT ACC GGC GAG GTG GAA ACC TAC ACG TTA GAC GAA ACA AGG     1803
   Leu Asn Phe Thr Gly Glu Val Glu Thr Tyr Thr Leu Asp Glu Thr Arg
                    65                  70                  75
   TTT AAA GCG GAA GCG GCG AAG AAA AGC CAT TGG AGT TTA GTG AAT GCG     1851
   Phe Lys Ala Glu Ala Ala Lys Lys Ser His Trp Ser Leu Val Asn Ala
                80                  85                  90
   GCG AAA GTA TAC GGC GGT TTA GAC CAA ATT ATT AAA AAA CTA TGG GAC     1899
   Ala Lys Val Tyr Gly Gly Leu Asp Gln Ile Ile Lys Lys Leu Trp Asp
            95                 100                 105
   AGT GGC TCA ATT AAG CAT TTA TAT CAA GAT AAA GAT ACG GGC AAA TTA      1947
   Ser Gly Ser Ile Lys His Leu Tyr Gln Asp Lys Asp Thr Gly Lys Leu
       110                 115                 120
   AAA CCG ATT ATT TAC GGC ACG GCC GGC AAC GAC AGT AAG ATT GAA GGC      1995
   Lys Pro Ile Ile Tyr Gly Thr Ala Gly Asn Asp Ser Lys Ile Glu Gly
   125                 130                 135                 140
   ACT AAA ATC ACC CGT AGG ATT GCG GGT AAA GAA GTT ACG CTT GAT ATT      2043
   Thr Lys Ile Thr Arg Arg Ile Ala Gly Lys Glu Val Thr Leu Asp Ile
                   145                 150                 155
   GCC AAT CAG AAA ATT GAA AAA GGC GTG TTA GAG AAA TTG GGG CTG TCT      2091
   Ala Asn Gln Lys Ile Glu Lys Gly Val Leu Glu Lys Leu Gly Leu Ser
               160                 165                 170
   GTT AGT GGT TCG GAT ATC ATT AAA TTG TTG TTT GGA GCA TTG ACT CCA      2139
   Val Ser Gly Ser Asp Ile Ile Lys Leu Leu Phe Gly Ala Leu Thr Pro
           175                 180                 185
   ACT TTA AAT AGA ATG TTG CTA TCA CAA CTT ATC CAG TCT TTT TCC GAT      2187
   Thr Leu Asn Arg Met Leu Leu Ser Gln Leu Ile Gln Ser Phe Ser Asp
       190                 195                 200
   AGC TTG GCT AAA CTT GAT AAT CCC TTA GCC CCT TAC ACT AAA AAT GGC      2235
   Ser Leu Ala Lys Leu Asp Asn Pro Leu Ala Pro Tyr Thr Lys Asn Gly
   205                 210                 215                 220
   GTG GTT TAT GTC ACC GGC AAA GGG AAT GAT GTG CTT AAA GGA ACT GAA      2283
   Val Val Tyr Val Thr Gly Lys Gly Asn Asp Val Leu Lys Gly Thr Glu
                   225                 230                 235
   CAT GAG GAT TTG TTT CTC GGT GGT GAG GGG AAT GAT ACT TAT TAT GCG      2331
   His Glu Asp Leu Phe Leu Gly Gly Glu Gly Asn Asp Thr Tyr Tyr Ala
               240                 245                 250
   AGA GTA GGC GAT ACA ATT GAA GAC GCC GAC GGC AAA GGT AAA GTC TAT      2379
   Arg Val Gly Asp Thr Ile Glu Asp Ala Asp Gly Lys Gly Lys Val Tyr
           255                 260                 265
   TTT GTG AGA GAA AAA GGG GTA CCT AAG GCG GAT CCT AAG CGG GTA GAG      2427
   Phe Val Arg Glu Lys Gly Val Pro Lys Ala Asp Pro Lys Arg Val Glu
       270                 275                 280
   TTT AGC GAG TAC ATA ACG AAA GAA GAA ATA AAA GAG GTT GAA AAG GGG      2475
   Phe Ser Glu Tyr Ile Thr Lys Glu Glu Ile Lys Glu Val Glu Lys Gly
   285                 290                 295                 300
   TTA TTA ACT TAC GCA GTT TTA GAA AAT TAT AAT TGG GAA GAG AAA ACG      2523
   Leu Leu Thr Tyr Ala Val Leu Glu Asn Tyr Asn Trp Glu Glu Lys Thr
                   305                 310                 315
   GCG ACT TTC GCT CAT GCG ACT ATG CTT AAT GAG CTT TTT ACT GAT TAT      2571
   Ala Thr Phe Ala His Ala Thr Met Leu Asn Glu Leu Phe Thr Asp Tyr
               320                 325                 330
   ACT AAT TAT CGT TAT GAA GTT AAA GGA CTA AAA TTG CCC GCC GTT AAA      2619
   Thr Asn Tyr Arg Tyr Glu Val Lys Gly Leu Lys Leu Pro Ala Val Lys
           335                 340                 345
   AAG TTA AAA AGT CCG TTG GTG GAG TTT ACA GCT GAT TTA TTA ACT GTT      2667
   Lys Leu Lys Ser Pro Leu Val Glu Phe Thr Ala Asp Leu Leu Thr Val
       350                 355                 360
   ACG CCT ATT GAC GAA AAC GGA AAA GCA CTT AGC GAA AAA AGT ATT ACG      2715
   Thr Pro Ile Asp Glu Asn Gly Lys Ala Leu Ser Glu Lys Ser Ile Thr
   365                 370                 375                 380
   GTT AAA AAT TTT AAA AAT GGT GAT TTA GGA ATA AGG TTG TTG GAT CCT     2763
   Val Lys Asn Phe Lys Asn Gly Asp Leu Gly Ile Arg Leu Leu Asp Pro
               385                 390                 395
   AAT AGC TAT TAT TAT TTC CTT GAA GGC CAA GAT ACG GGT TTT TAT GGT     2811
   Asn Ser Tyr Tyr Tyr Phe Leu Glu Gly Gln Asp Thr Gly Phe Tyr Gly
           400                 405                 410
   CCT GCT TTT TAT ATT GAA CGA AAA AAC GGT GGC GGC GCT AAA AAT AAC     2859
   Pro Ala Phe Tyr Ile Glu Arg Lys Asn Gly Gly Gly Ala Lys Asn Asn
       415                 420                 425
   TCG TCG GGA GCA GGA AAT AGC AAA GAT TGG GGC GGG AAC GGG CAT GGA     2907
   Ser Ser Gly Ala Gly Asn Ser Lys Asp Trp Gly Gly Asn Gly His Gly
       430                 435                 440
   AAT CAC CGA AAT AAT GCC TCC GAC CTG AAT AAA CCG GAC GGA AAT AAT     2955
   Asn His Arg Asn Asn Ala Ser Asp Leu Asn Lys Pro Asp Gly Asn Asn
   445                 450                 455                 460
   GGG AAT AAC CAA AAT AAC GGA AGC AAT CAA GAT AAT CAT AGC GAT GTG     3003
   Gly Asn Asn Gln Asn Asn Gly Ser Asn Gln Asp Asn His Ser Asp Val
                   465                 470                 475
   AAT GCG CCA AAT AAC CCG GGA CGT AAC TAT GAT ATT TAC GAT CCT TTA     3051
   Asn Ala Pro Asn Asn Pro Gly Arg Asn Tyr Asp Ile Tyr Asp Pro Leu
               480                 485                 490
   GCT TTA GAT TTA GAT GGA GAT GGG CTT GAA ACC GTG TCG ATG AAC GGG     3099
   Ala Leu Asp Leu Asp Gly Asp Gly Leu Glu Thr Val Ser Met Asn Gly
           495                 500                 505
   CGA CAA GGC GCG TTA TTC GAT CAT GAA GGA AAA GGT ATT CGT ACC GCA     3147
   Arg Gln Gly Ala Leu Phe Asp His Glu Gly Lys Gly Ile Arg Thr Ala
       510                 515                 520
   ACG GGC TGG CTC GCT GCG GAT GAC GGT TTT TTA GTG TTA GAT CGT AAC     3195
   Thr Gly Trp Leu Ala Ala Asp Asp Gly Phe Leu Val Leu Asp Arg Asn
   525                 530                 535                 540
   CAA GAC GGC ATT ATT AAT GAT ATA AGC GAG TTA TTT AGT AAT AAA AAT     3243
   Gln Asp Gly Ile Ile Asn Asp Ile Ser Glu Leu Phe Ser Asn Lys Asn
                   545                 550                 555
   CAA CTT TCC GAC GGC AGT ATT TCT GCA CAC GGT TTT GCG ACA TTA GCC     3291
   Gln Leu Ser Asp Gly Ser Ile Ser Ala His Gly Phe Ala Thr Leu Ala
               560                 565                 570
   GAT TTG GAT ACA AAC CAA GAT CAG CGT ATC GAC CAA AAT GAT AAG CTG     3339
   Asp Leu Asp Thr Asn Gln Asp Gln Arg Ile Asp Gln Asn Asp Lys Leu
           575                 580                 585
   TTT TCT AAA CTC CAA ATT TGG CGG GAT TTA AAT CAA AAC GGT TTT AGT     3387
   Phe Ser Lys Leu Gln Ile Trp Arg Asp Leu Asn Gln Asn Gly Phe Ser
       590                 595                 600
   GAA GCG AAT GAG CTG TTT AGC TTA GAA AGT TTG AAT ATT AAA TCT TTA     3435
   Glu Ala Asn Glu Leu Phe Ser Leu Glu Ser Leu Asn Ile Lys Ser Leu
   605                 610                 615                 620
   CAT ACC GCC TAT GAA GAG CGT AAT GAT TTT CTA GCG GGC AAT AAT ATC     3483
   His Thr Ala Tyr Glu Glu Arg Asn Asp Phe Leu Ala Gly Asn Asn Ile
                   625                 630                 635
   CTT GCT CAG CTT GGG AAG TAT GAA AAA ACG GAC GGT ACT TTT GCA CAA     3531
   Leu Ala Gln Leu Gly Lys Tyr Glu Lys Thr Asp Gly Thr Phe Ala Gln
               640                 645                 650
   ATG GGC GAT TTA AAT TTC AGT TTT AAC CCG TTT TAT AGC CGA TTT ACC    3579
   Met Gly Asp Leu Asn Phe Ser Phe Asn Pro Phe Tyr Ser Arg Phe Thr
       655                 660                     665
   GAA GCG TTA AAT TTA ACC GAG CAA CAA CGT CGC ACA ATT AAT CTA ACC    3627
   Glu Ala Leu Asn Leu Thr Glu Gln Gln Arg Arg Thr Ile Asn Leu Thr
       670                 675                     680
   GGC ACC GGT CGG GTT CGG GAT TTG CGT GAA GCC GCC GCA CTT TCT GAG    3675
   Gly Thr Gly Arg Val Arg Asp Leu Arg Glu Ala Ala Ala Leu Ser Glu
   685                 690                     695                 700
   GAG TTG GCT GCT TTA TTA CAA CAG TAC ACT AAG GCC TCC GAT TTT CAG    3723
   Glu Leu Ala Ala Leu Leu Gln Gln Tyr Thr Lys Ala Ser Asp Phe Gln
                   705                     710                 715
   GCA CAA CGA GAA TTA TTG CCT GCC ATT TTA GAT AAA TGG GCG GCA ACG    3771
   Ala Gln Arg Glu Leu Leu Pro Ala Ile Leu Asp Lys Trp Ala Ala Thr
               720                     725                 730
   GAT TTA CAG TAT CAA CAT TAT GAT AAA ACA TTA CTT AAA ACG GTA GAA    3819
   Asp Leu Gln Tyr Gln His Tyr Asp Lys Thr Leu Leu Lys Thr Val Glu
           735                     740                 745
   AGT ACC GAT AGT AGT GCT TCT GTC GTT AGA GTC ACG CCT TCT CAA TTA    3867
   Ser Thr Asp Ser Ser Ala Ser Val Val Arg Val Thr Pro Ser Gln Leu
       750                 755                     760
   AGT AGT ATA CGC AAT GCA AAG CAT GAT CCT ACC GTT ATG CAA AAC TTT    3915
   Ser Ser Ile Arg Asn Ala Lys His Asp Pro Thr Val Met Gln Asn Phe
   765                 770                     775                 780
   GAA CAG AGT AAG GCA AAA ATT GCG ACT TTA AAT TCG CTC TAC GGG TTA    3963
   Glu Gln Ser Lys Ala Lys Ile Ala Thr Leu Asn Ser Leu Tyr Gly Leu
                   785                     790                 795
   AAT ATC GAT CAA CTT TAT TAC ACG ACG GAT AAA GAC ATT CGC TAT ATT    4011
   Asn Ile Asp Gln Leu Tyr Tyr Thr Thr Asp Lys Asp Ile Arg Tyr Ile
               800                     805                 810
   ACT GAT AAA GTG AAT AAT ATG TAT CAA ACA ACC GTA GAA CTT GCC TAC    4059
   Thr Asp Lys Val Asn Asn Met Tyr Gln Thr Thr Val Glu Leu Ala Tyr
           815                     820                 825
   CGT TCT TTA CTT TTA CAA ACG CGT TTG AAG AAA TAT GTT TAT AGC GTT    4107
   Arg Ser Leu Leu Leu Gln Thr Arg Leu Lys Lys Tyr Val Tyr Ser Val
       830                 835                     840
   AAT GCG AAA CAA TTC GAA GGG AAA TGG GTA ACC GAT TAT TCT CGT ACT    4155
   Asn Ala Lys Gln Phe Glu Gly Lys Trp Val Thr Asp Tyr Ser Arg Thr
   845                 850                     855                 860
   GAA GCC TTA TTT AAC TCT ACT TTT AAA CAA TCG CCT GAA AAT GCA TTA    4203
   Glu Ala Leu Phe Asn Ser Thr Phe Lys Gln Ser Pro Glu Asn Ala Leu
                   865                     870                 875
   TAT GAT TTA AGC GAA TAC CTT TCT TTC TTT AAC GAT CCT ACG GAA TGG    4251
   Tyr Asp Leu Ser Glu Tyr Leu Ser Phe Phe Asn Asp Pro Thr Glu Trp
               880                     885                 890
   AAA GAA GGG CTA TTA CTG TTA AGC CGT TAT ATA GAT TAT GCT AAA GCA    4299
   Lys Glu Gly Leu Leu Leu Leu Ser Arg Tyr Ile Asp Tyr Ala Lys Ala
           895                     900                 905
   CAA GGA TTT TAT GAA AAC TGG GCG GCT ACT TCT AAC TTA ACT ATT GCC    4347
   Gln Gly Phe Tyr Glu Asn Trp Ala Ala Thr Ser Asn Leu Thr Ile Ala
       910                 915                     920
   CGT TTA AGA GAG GCT GGA GTA ATT TTT GCA GAA TCG ACG GAT TTA AAA      4395
   Arg Leu Arg Glu Ala Gly Val Ile Phe Ala Glu Ser Thr Asp Leu Lys
   925                 930                 935                 940
   GGC GAT GAA AAA AAT AAT ATT TTG TTA GGT AGC CAA AAA GAT AAT AAC      4443
   Gly Asp Glu Lys Asn Asn Ile Leu Leu Gly Ser Gln Lys Asp Asn Asn
                   945                 950                 955
   TTA TCG GGT AGT GCA GGT GAT GAT CTA CTT ATC GGC GGA GAG GGT AAT      4491
   Leu Ser Gly Ser Ala Gly Asp Asp Leu Leu Ile Gly Gly Glu Gly Asn
               960                 965                 970
   GAT ACG TTA AAA GGC AGC TAC GGT GCA GAC ACC TAT ATC TTT AGC AAA      4539
   Asp Thr Leu Lys Gly Ser Tyr Gly Ala Asp Thr Tyr Ile Phe Ser Lys
           975                 980                 985
   GGA CAC GGA CAG GAT ATC GTT TAT GAA GAT ACC AAT AAT GAT AAC CGC      4587
   Gly His Gly Gln Asp Ile Val Tyr Glu Asp Thr Asn Asn Asp Asn Arg
       990                 995                 1000
   GCA AGA GAT ATC GAC ACC TTA AAA TTT ACC GAT GTG AAT TAT GCG GAA      4635
   Ala Arg Asp Ile Asp Thr Leu Lys Phe Thr Asp Val Asn Tyr Ala Glu
   1005                1010                1015                1020
   GTG AAG TTT CGA CGA GTA GAT AAT GAC TTA ATG TTA TTC GGT TAT CAT      4683
   Val Lys Phe Arg Arg Val Asp Asn Asp Leu Met Leu Phe Gly Tyr His
                   1025                1030                1035
   GAT ACG GAT TCG GTC ACG GTA AAA TCC TTC TAC AGC CAT GTA GAT TAT      4731
   Asp Thr Asp Ser Val Thr Val Lys Ser Phe Tyr Ser His Val Asp Tyr
               1040                1045                1050
   CAA TTT GAC AAA TTG GAG TTT GCT GAC CGC AGT ATA ACT CGC GAT GAA      4779
   Gln Phe Asp Lys Leu Glu Phe Ala Asp Arg Ser Ile Thr Arg Asp Glu
           1055                1060                1065
   CTG ATT AAA GCA GGG CTT CAT CTA TAC GGC ACC GAT GGC AAT GAT GAT      4827
   Leu Ile Lys Ala Gly Leu His Leu Tyr Gly Thr Asp Gly Asn Asp Asp
       1070                1075                1080
   ATA AAG GAT CAT GCG GAT TGG GAC AGC ATT TTG GAA GGC GGC AAA GGC      4875
   Ile Lys Asp His Ala Asp Trp Asp Ser Ile Leu Glu Gly Gly Lys Gly
   1085                1090                1095                1100
   AAC GAT ATT CTA AGA GGT GGC TAC GGT GCG GAC ACC TAT ATC TTT AGC      4923
   Asn Asp Ile Leu Arg Gly Gly Tyr Gly Ala Asp Thr Tyr Ile Phe Ser
                   1105                1110                1115
   AAA GGA CAC GGA CAG GAT ATC GTT TAT GAA GAT ACC AAT AAT GAT AAC      4971
   Lys Gly His Gly Gln Asp Ile Val Tyr Glu Asp Thr Asn Asn Asp Asn
               1120                1125                1130
   CGC GCA AGA GAT ATC GAC ACC TTA AAA TTT ACT GAT GTG AAT TAT GCG      5019
   Arg Ala Arg Asp Ile Asp Thr Leu Lys Phe Thr Asp Val Asn Tyr Ala
           1135                1140                1145
   GAA GTG AAA TTC CGA CGA GTA GAT AAT GAC TTA ATG TTA TTC GGT TAT      5067
   Glu Val Lys Phe Arg Arg Val Asp Asn Asp Leu Met Leu Phe Gly Tyr
       1150                1155                1160
   CAT GAT ACG GAT TCG GTC ACG ATA AAA TCC TTC TAC AAC CAT GTA GAT      5115
   His Asp Thr Asp Ser Val Thr Ile Lys Ser Phe Tyr Asn His Val Asp
   1165                1170                1175                1180
   TAT CAA TTT GAC AAA TTG GAA TTT GCT GAC CGC AGT ATA ACT CGT GAT      5163
   Tyr Gln Phe Asp Lys Leu Glu Phe Ala Asp Arg Ser Ile Thr Arg Asp
                   1185                1190                1195
   GAA CTA GGT AAA CAA GGT ATG GCA TTA TTT GGC ACT GAC GGT GAT GAT      5211
   Glu Leu Gly Lys Gln Gly Met Ala Leu Phe Gly Thr Asp Gly Asp Asp
               1200                1205                1210
   AAT ATC AAC GAC TGG GGA CGT AAC TCG GTG ATT GAT GCC GGT GCG GGT      5259
   Asn Ile Asn Asp Trp Gly Arg Asn Ser Val Ile Asp Ala Gly Ala Gly
           1215                1220                1225
   AAT GAT ACG GTT AAT GGC GGT AAT GGC GAT GAC ACC CTC ATC GGC GGC      5307
   Asn Asp Thr Val Asn Gly Gly Asn Gly Asp Asp Thr Leu Ile Gly Gly
       1230                1235                1240
   AAA GGT AAT GAT ATT CTA AGA GGT GGC TAC GGT GCG GAC ACC TAT ATC      5355
   Lys Gly Asn Asp Ile Leu Arg Gly Gly Tyr Gly Ala Asp Thr Tyr Ile
   1245                1250                1255                1260
   TTT AGC AAA GGA CAC GGA CAG GAT ATC GTT TAT GAA GAT ACC AAT AAT      5403
   Phe Ser Lys Gly His Gly Gln Asp Ile Val Tyr Glu Asp Thr Asn Asn
                   1265                1270                1275
   GAT AAC CGC GCA AGA GAT ATC GAC ACC TTA AAA TTT ACC GAT GTG AAT      5451
   Asp Asn Arg Ala Arg Asp Ile Asp Thr Leu Lys Phe Thr Asp Val Asn
               1280                1285                1290
   TAT GCG GAA GTG AAA TTC CGA CGA GTA GAT AAT GAC TTA ATG TTA TTC      5499
   Tyr Ala Glu Val Lys Phe Arg Arg Val Asp Asn Asp Leu Met Leu Phe
           1295                1300                1305
   GGT TAT CAT GAT ACG GAT TCG GTC ACG GTA AAA TCC TTC TAC AGC CAT      5547
   Gly Tyr His Asp Thr Asp Ser Val Thr Val Lys Ser Phe Tyr Ser His
       1310                1315                1320
   GTA GAT TAT CAA TTT GAC AAA TTG GAG TTT GCT GAC CGC AGT ATA ACT      5595
   Val Asp Tyr Gln Phe Asp Lys Leu Glu Phe Ala Asp Arg Ser Ile Thr
   1325                1330                1335                1340
   CGC GAT GAA CTG ATT AAA GCA GGG CTT CAT CTA TAC GGC ACC GAT GGC      5643
   Arg Asp Glu Leu Ile Lys Ala Gly Leu His Leu Tyr Gly Thr Asp Gly
                   1345                1350                1355
   AAT GAT GAT ATA AAG GAT CAT GCG GAT TGG GAC AGC ATT TTG GAA GGC      5691
   Asn Asp Asp Ile Lys Asp His Ala Asp Trp Asp Ser Ile Leu Glu Gly
               1360                1365                1370
   GGC AAA GGC AAC GAT ATT CTA AGA GGT GGC TAC GGT GCG GAC ACC TAT      5739
   Gly Lys Gly Asn Asp Ile Leu Arg Gly Gly Tyr Gly Ala Asp Thr Tyr
           1375                1380                1385
   ATC TTT AGC AAA GGA CAC GGA CAG GAT ATC GTT TAT GAA GAT ACC AAT      5787
   Ile Phe Ser Lys Gly His Gly Gln Asp Ile Val Tyr Glu Asp Thr Asn
       1390                1395                1400
   AAT GAT AAC CGA GCA AGA GAT ATC GAC ACC TTA AAA TTT ACT GAT GTG      5835
   Asn Asp Asn Arg Ala Arg Asp Ile Asp Thr Leu Lys Phe Thr Asp Val
   1405                1410                1415                1420
   AAT TAT GCG GAA GTG AAA TTC CGA CGA GTA GAT AAT GAC TTA ATG TTA      5883
   Asn Tyr Ala Glu Val Lys Phe Arg Arg Val Asp Asn Asp Leu Met Leu
                   1425                1430                1435
   TTC GGT TAT CAT GAT ACG GAT TCG GTC ACG ATA AAA TCC TTC TAC AAC      5931
   Phe Gly Tyr His Asp Thr Asp Ser Val Thr Ile Lys Ser Phe Tyr Asn
               1440                1445                1450
   CAT GTA GAT TAT CAA TTT GAC AAA TTG GAA TTT GCT GAC CGC AGT ATA      5979
   His Val Asp Tyr Gln Phe Asp Lys Leu Glu Phe Ala Asp Arg Ser Ile
           1455                1460                1465
   ACT CGT GAT GAA CTA GGT AAA CAA GGT ATG GCA TTA TTT GGC ACT GAC      6027
   Thr Arg Asp Glu Leu Gly Lys Gln Gly Met Ala Leu Phe Gly Thr Asp
       1470                1475                1480
   GGT GAT GAT AAT ATC AAC GAC TGG GGA CGT AAC TCG GTG ATT GAT GCC      6075
   Gly Asp Asp Asn Ile Asn Asp Trp Gly Arg Asn Ser Val Ile Asp Ala
   1485                1490                1495                1500
   GGT GCG GGT AAT GAT ACG GTT AAT GGC GGT AAT GGC GAT GAC ACC CTC      6123
   Gly Ala Gly Asn Asp Thr Val Asn Gly Gly Asn Gly Asp Asp Thr Leu
                   1505                1510                1515
   ATC GGC GGC AAA GGT AAT GAT ATT CTA AGA GGT GGC TAC GGT GCG GAC      6171
   Ile Gly Gly Lys Gly Asn Asp Ile Leu Arg Gly Gly Tyr Gly Ala Asp
               1520                1525                1530
   ACC TAT ATC TTT AGC AAA GGA CAC GGA CAG GAT ATC GTT TAT GAA GAT      6219
   Thr Tyr Ile Phe Ser Lys Gly His Gly Gln Asp Ile Val Tyr Glu Asp
           1535                1540                1545
   ACC AAT AAT GAT AAC CGC GCA AGA GAT ATC GAC ACC TTA AAA TTT ACT      6267
   Thr Asn Asn Asp Asn Arg Ala Arg Asp Ile Asp Thr Leu Lys Phe Thr
       1550                1555                1560
   GAT ATT AAT TTA TCC GAA CTT TGG TTT AGC CGA GAA AAT AAC GAT TTG      6315
   Asp Ile Asn Leu Ser Glu Leu Trp Phe Ser Arg Glu Asn Asn Asp Leu
   1565                1570                1575                1580
   ATT ATT AAA TCA TTA TTA AGT GAG GAT AAA GTC ACG GTT CAA AAT TGG      6363
   Ile Ile Lys Ser Leu Leu Ser Glu Asp Lys Val Thr Val Gln Asn Trp
                   1585                1590                1595
   TAT TCA CAC CAA GAT CAT AAA ATA GAA AAT ATT CGT TTA TCG AAT GAG      6411
   Tyr Ser His Gln Asp His Lys Ile Glu Asn Ile Arg Leu Ser Asn Glu
               1600                1605                1610
   CAA ACG TTG GTG AGC ACT CAG GTG GAG AAG ATG GTT GAG TCG ATG GCC      6459
   Gln Thr Leu Val Ser Thr Gln Val Glu Lys Met Val Glu Ser Met Ala
           1615                1620                1625
   GGC TTT GCT CAG AAG CAC GGA GGA GAG ATA TCT CTT GTG TCG CTT GAA      6507
   Gly Phe Ala Gln Lys His Gly Gly Glu Ile Ser Leu Val Ser Leu Glu
       1630                1635                1640
   GAG GTA AAA CAA TAT ATC AAT AGC TTA ACA GCT GCT TTA TAA              6549
   Glu Val Lys Gln Tyr Ile Asn Ser Leu Thr Ala Ala Leu  *
   1645                1650                1655
   CATACGAAAG AAATCGGCAC AGTTTTTTTG AACTGTGCCG ATTTGATTTT AGTGTAAGAA    6609
   TATAGCCTGA TTTTAAGAAA TTTACTCTTG GCTAATAACT ATTTCCCATT TTATAAGTTA    6669
   TTGACGGATG GTTTTATCAA ATATGAGATC AAATCTTATT TTAAATTCGC TTTCCATTAA    6729
   GCGATAT                                                              6736
(2) INFORMATION FOR SEQ ID NO: 2:
   (i) SEQUENCE CHARACTERISTICS:
      (A) LENGTH: 1658 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Ser Asp Asn Ala Phe Phe Val Ile Glu Glu Ser Gly Lys Arg Tyr
   1 5 10 15
   Ile Glu Asn Phe Gly Ile Glu Pro Leu Gly Lys Gln Glu Asp Phe Asp
   20 25 30
   Phe Val Gly Gly Phe Trp Ser Asn Leu Val Asn Arg Gly Leu Glu Ser
   35 40 45
   Ile Ile Asp Pro Ser Gly Ile Gly Gly Thr Val Asn Leu Asn Phe Thr
   50 55 60
   Gly Glu Val Glu Thr Tyr Thr Leu Asp Glu Thr Arg Phe Lys Ala Glu
   65 70 75 80
   Ala Ala Lys Lys Ser His Trp Ser Leu Val Asn Ala Ala Lys Val Tyr
   85 90 95
   Gly Gly Leu Asp Gln Ile Ile Lys Lys Leu Trp Asp Ser Gly Ser Ile
   100 105 110
   Lys His Leu Tyr Gln Asp Lys Asp Thr Gly Lys Leu Lys Pro Ile Ile
   115 120 125
   Tyr Gly Thr Ala Gly Asn Asp Ser Lys Ile Glu Gly Thr Lys Ile Thr
   130 135 140
   Arg Arg Ile Ala Gly Lys Glu Val Thr Leu Asp Ile Ala Asn Gln Lys
   145 150 155 160
   Ile Glu Lys Gly Val Leu Glu Lys Leu Gly Leu Ser Val Ser Gly Ser
   165 170 175
   Asp Ile Ile Lys Leu Leu Phe Gly Ala Leu Thr Pro Thr Leu Asn Arg
   180 185 190
   Met Leu Leu Ser Gln Leu Ile Gln Ser Phe Ser Asp Ser Leu Ala Lys
   195 200 205
   Leu Asp Asn Pro Leu Ala Pro Tyr Thr Lys Asn Gly Val Val Tyr Val
   210 215 220
   Thr Gly Lys Gly Asn Asp Val Leu Lys Gly Thr Glu His Glu Asp Leu
   225 230 235 240
   Phe Leu Gly Gly Glu Gly Asn Asp Thr Tyr Tyr Ala Arg Val Gly Asp
   245 250 255
   Thr Ile Glu Asp Ala Asp Gly Lys Gly Lys Val Tyr Phe Val Arg Glu
   260 265 270
   Lys Gly Val Pro Lys Ala Asp Pro Lys Arg Val Glu Phe Ser Glu Tyr
   275 280 285
   Ile Thr Lys Glu Glu Ile Lys Glu Val Glu Lys Gly Leu Leu Thr Tyr
   290 295 300
   Ala Val Leu Glu Asn Tyr Asn Trp Glu Glu Lys Thr Ala Thr Phe Ala
   305 310 315 320
   His Ala Thr Met Leu Asn Glu Leu Phe Thr Asp Tyr Thr Asn Tyr Arg
   325 330 335
   Tyr Glu Val Lys Gly Leu Lys Leu Pro Ala Val Lys Lys Leu Lys Ser
   340 345 350
   Pro Leu Val Glu Phe Thr Ala Asp Leu Leu Thr Val Thr Pro Ile Asp
   355 360 365
   Glu Asn Gly Lys Ala Leu Ser Glu Lys Ser Ile Thr Val Lys Asn Phe
   370 375 380
   Lys Asn Gly Asp Leu Gly Ile Arg Leu Leu Asp Pro Asn Ser Tyr Tyr
   385 390 395 400
   Tyr Phe Leu Glu Gly Gln Asp Thr Gly Phe Tyr Gly Pro Ala Phe Tyr
   405 410 415
   Ile Glu Arg Lys Asn Gly Gly Gly Ala Lys Asn Asn Ser Ser Gly Ala
   420 425 430
   Gly Asn Ser Lys Asp Trp Gly Gly Asn Gly His Gly Asn His Arg Asn
   435 440 445
   Asn Ala Ser Asp Leu Asn Lys Pro Asp Gly Asn Asn Gly Asn Asn Gln
   450 455 460
   Asn Asn Gly Ser Asn Gln Asp Asn His Ser Asp Val Asn Ala Pro Asn
   465 470 475 480
   Asn Pro Gly Arg Asn Tyr Asp Ile Tyr Asp Pro Leu Ala Leu Asp Leu
   485 490 495
   Asp Gly Asp Gly Leu Glu Thr Val Ser Met Asn Gly Arg Gln Gly Ala
   500 505 510
   Leu Phe Asp His Glu Gly Lys Gly Ile Arg Thr Ala Thr Gly Trp Leu
   515 520 525
   Ala Ala Asp Asp Gly Phe Leu Val Leu Asp Arg Asn Gln Asp Gly Ile
   530 535 540
   Ile Asn Asp Ile Ser Glu Leu Phe Ser Asn Lys Asn Gln Leu Ser Asp
   545 550 555 560
   Gly Ser Ile Ser Ala His Gly Phe Ala Thr Leu Ala Asp Leu Asp Thr
   565 570 575
   Asn Gln Asp Gln Arg Ile Asp Gln Asn Asp Lys Leu Phe Ser Lys Leu
   580 585 590
   Gln Ile Trp Arg Asp Leu Asn Gln Asn Gly Phe Ser Glu Ala Asn Glu
   595 600 605
   Leu Phe Ser Leu Glu Ser Leu Asn Ile Lys Ser Leu His Thr Ala Tyr
   610 615 620
   Glu Glu Arg Asn Asp Phe Leu Ala Gly Asn Asn Ile Leu Ala Gln Leu
   625 630 635 640
   Gly Lys Tyr Glu Lys Thr Asp Gly Thr Phe Ala Gln Met Gly Asp Leu
   645 650 655
   Asn Phe Ser Phe Asn Pro Phe Tyr Ser Arg Phe Thr Glu Ala Leu Asn
   660 665 670
   Leu Thr Glu Gln Gln Arg Arg Thr Ile Asn Leu Thr Gly Thr Gly Arg
   675 680 685
   Val Arg Asp Leu Arg Glu Ala Ala Ala Leu Ser Glu Glu Leu Ala Ala
   690 695 700
   Leu Leu Gln Gln Tyr Thr Lys Ala Ser Asp Phe Gln Ala Gln Arg Glu
   705 710 715 720
   Leu Leu Pro Ala Ile Leu Asp Lys Trp Ala Ala Thr Asp Leu Gln Tyr
   725 730 735
   Gln His Tyr Asp Lys Thr Leu Leu Lys Thr Val Glu Ser Thr Asp Ser
   740 745 750
   Ser Ala Ser Val Val Arg Val Thr Pro Ser Gln Leu Ser Ser Ile Arg
   755 760 765
   Asn Ala Lys His Asp Pro Thr Val Met Gln Asn Phe Glu Gln Ser Lys
   770 775 780
   Ala Lys Ile Ala Thr Leu Asn Ser Leu Tyr Gly Leu Asn Ile Asp Gln
   785 790 795 800
   Leu Tyr Tyr Thr Thr Asp Lys Asp Ile Arg Tyr Ile Thr Asp Lys Val
   805 810 815
   Asn Asn Met Tyr Gln Thr Thr Val Glu Leu Ala Tyr Arg Ser Leu Leu
   820 825 830
   Leu Gln Thr Arg Leu Lys Lys Tyr Val Tyr Ser Val Asn Ala Lys Gln
   835 840 845
   Phe Glu Gly Lys Trp Val Thr Asp Tyr Ser Arg Thr Glu Ala Leu Phe
   850 855 860
   Asn Ser Thr Phe Lys Gln Ser Pro Glu Asn Ala Leu Tyr Asp Leu Ser
   865 870 875 880
   Glu Tyr Leu Ser Phe Phe Asn Asp Pro Thr Glu Trp Lys Glu Gly Leu
   885 890 895
   Leu Leu Leu Ser Arg Tyr Ile Asp Tyr Ala Lys Ala Gln Gly Phe Tyr
   900 905 910
   Glu Asn Trp Ala Ala Thr Ser Asn Leu Thr Ile Ala Arg Leu Arg Glu
   915 920 925
   Ala Gly Val Ile Phe Ala Glu Ser Thr Asp Leu Lys Gly Asp Glu Lys
   930 935 940
   Asn Asn Ile Leu Leu Gly Ser Gln Lys Asp Asn Asn Leu Ser Gly Ser
   945 950 955 960
   Ala Gly Asp Asp Leu Leu Ile Gly Gly Glu Gly Asn Asp Thr Leu Lys
   965 970 975
   Gly Ser Tyr Gly Ala Asp Thr Tyr Ile Phe Ser Lys Gly His Gly Gln
   980 985 990
   Asp Ile Val Tyr Glu Asp Thr Asn Asn Asp Asn Arg Ala Arg Asp Ile
   995 1000 1005
   Asp Thr Leu Lys Phe Thr Asp Val Asn Tyr Ala Glu Val Lys Phe Arg
   1010 1015 1020
   Arg Val Asp Asn Asp Leu Met Leu Phe Gly Tyr His Asp Thr Asp Ser
   1025 1030 1035 1040
   Val Thr Val Lys Ser Phe Tyr Ser His Val Asp Tyr Gln Phe Asp Lys
   1045 1050 1055
   Leu Glu Phe Ala Asp Arg Ser Ile Thr Arg Asp Glu Leu Ile Lys Ala
   1060 1065 1070
   Gly Leu His Leu Tyr Gly Thr Asp Gly Asn Asp Asp Ile Lys Asp His
   1075 1080 1085
   Ala Asp Trp Asp Ser Ile Leu Glu Gly Gly Lys Gly Asn Asp Ile Leu
   1090 1095 1100
   Arg Gly Gly Tyr Gly Ala Asp Thr Tyr Ile Phe Ser Lys Gly His Gly
   1105 1110 1115 1120
   Gln Asp Ile Val Tyr Glu Asp Thr Asn Asn Asp Asn Arg Ala Arg Asp
   1125 1130 1135
   Ile Asp Thr Leu Lys Phe Thr Asp Val Asn Tyr Ala Glu Val Lys Phe
   1140 1145 1150
   Arg Arg Val Asp Asn Asp Leu Met Leu Phe Gly Tyr His Asp Thr Asp
   1155 1160 1165
   Ser Val Thr Ile Lys Ser Phe Tyr Asn His Val Asp Tyr Gln Phe Asp
   1170 1175 1180
   Lys Leu Glu Phe Ala Asp Arg Ser Ile Thr Arg Asp Glu Leu Gly Lys
   1185 1190 1195 1200
   Gln Gly Met Ala Leu Phe Gly Thr Asp Gly Asp Asp Asn Ile Asn Asp
   1205 1210 1215
   Trp Gly Arg Asn Ser Val Ile Asp Ala Gly Ala Gly Asn Asp Thr Val
   1220 1225 1230
   Asn Gly Gly Asn Gly Asp Asp Thr Leu Ile Gly Gly Lys Gly Asn Asp
   1235 1240 1245
   Ile Leu Arg Gly Gly Tyr Gly Ala Asp Thr Tyr Ile Phe Ser Lys Gly
   1250 1255 1260
   His Gly Gln Asp Ile Val Tyr Glu Asp Thr Asn Asn Asp Asn Arg Ala
   1265 1270 1275 1280
   Arg Asp Ile Asp Thr Leu Lys Phe Thr Asp Val Asn Tyr Ala Glu Val
   1285 1290 1295
   Lys Phe Arg Arg Val Asp Asn Asp Leu Met Leu Phe Gly Tyr His Asp
   1300 1305 1310
   Thr Asp Ser Val Thr Val Lys Ser Phe Tyr Ser His Val Asp Tyr Gln
   1315 1320 1325
   Phe Asp Lys Leu Glu Phe Ala Asp Arg Ser Ile Thr Arg Asp Glu Leu
   1330 1335 1340
   Ile Lys Ala Gly Leu His Leu Tyr Gly Thr Asp Gly Asn Asp Asp Ile
   1345 1350 1355 1360
   Lys Asp His Ala Asp Trp Asp Ser Ile Leu Glu Gly Gly Lys Gly Asn
   1365 1370 1375
   Asp Ile Leu Arg Gly Gly Tyr Gly Ala Asp Thr Tyr Ile Phe Ser Lys
   1380 1385 1390
   Gly His Gly Gln Asp Ile Val Tyr Glu Asp Thr Asn Asn Asp Asn Arg
   1395 1400 1405
   Ala Arg Asp Ile Asp Thr Leu Lys Phe Thr Asp Val Asn Tyr Ala Glu
   1410 1415 1420
   Val Lys Phe Arg Arg Val Asp Asn Asp Leu Met Leu Phe Gly Tyr His
   1425 1430 1435 1440
   Asp Thr Asp Ser Val Thr Ile Lys Ser Phe Tyr Asn His Val Asp Tyr
   1445 1450 1455
   Gln Phe Asp Lys Leu Glu Phe Ala Asp Arg Ser Ile Thr Arg Asp Glu
   1460 1465 1470
   Leu Gly Lys Gln Gly Met Ala Leu Phe Gly Thr Asp Gly Asp Asp Asn
   1475 1480 1485
   Ile Asn Asp Trp Gly Arg Asn Ser Val Ile Asp Ala Gly Ala Gly Asn
   1490 1495 1500
   Asp Thr Val Asn Gly Gly Asn Gly Asp Asp Thr Leu Ile Gly Gly Lys
   1505 1510 1515 1520
   Gly Asn Asp Ile Leu Arg Gly Gly Tyr Gly Ala Asp Thr Tyr Ile Phe
   1525 1530 1535
   Ser Lys Gly His Gly Gln Asp Ile Val Tyr Glu Asp Thr Asn Asn Asp
   1540 1545 1550
   Asn Arg Ala Arg Asp Ile Asp Thr Leu Lys Phe Thr Asp Ile Asn Leu
   1555 1560 1565
   Ser Glu Leu Trp Phe Ser Arg Glu Asn Asn Asp Leu Ile Ile Lys Ser
   1570 1575 1580
   Leu Leu Ser Glu Asp Lys Val Thr Val Gln Asn Trp Tyr Ser His Gln
   1585 1590 1595 1600
   Asp His Lys Ile Glu Asn Ile Arg Leu Ser Asn Glu Gln Thr Leu Val
   1605 1610 1615
   Ser Thr Gln Val Glu Lys Met Val Glu Ser Met Ala Gly Phe Ala Gln
   1620 1625 1630
   Lys His Gly Gly Glu Ile Ser Leu Val Ser Leu Glu Glu Val Lys Gln
   1635 1640 1645
   Tyr Ile Asn Ser Leu Thr Ala Ala Leu *
   1650 1655
(2) INFORMATION FOR SEQ ID NO: 3:
   (i) SEQUENCE CHARACTERISTICS:
      (A) LENGTH: 7004 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Actinobacillus pleuropneumoniae
      (B) STRAIN: HV114 (serotype 3 field strain)
   (vii) IMMEDIATE SOURCE:
      (B) CLONE: pROK5
   (ix) FEATURE:
      (A) NAME/KEY: CDS
      (B) LOCATION:1566..5714
      (D) OTHER INFORMATION:/codon start= 1566
         /function= "RTX-toxin"
         /product= "ApxIV_var3"
         /gene= "apxIV_var3"
         /number= 1
   (xi) SEQUENCE DESCRIPTION: SEQ ID NO: 3:
   ATCGATATGC CGCCGGGTAC GGGCGATATC CAACTTACTC TTTCGCAACA AATTCCGGTT      60
   ACCGGTGCGG TAGTGGTAAC TACTCCGCAA GATATTGCGT TATTAGATGC GGTGAAAGGT     120
   ATTTCAATGT TCCAAAAAGT GTCGGTACCG GTCTTAGGTA TCATTGAAAA TATGAGCGTG     180
   CATATCTGCC AAAATTGCGG TCACCACGAA GATATTTTCG GCACCGGCGG TGCGGAGAAA     240
   GTGGCGAAGA AATACGGTAC TAAAGTATTA GGACAAATGC CGTTGCATAT TCGCTTACGT     300
   CAAGATTTGG ATGCCGGCAC ACCGACCGTC GTTGCGGCAC CGGAACACGA CACCAGCAGA     360
   GCCTATATTG AATTAGCGGC AAAAGTCGCT TCGGAATTAT ACTGGCAAGG TTCGGTTATC     420
   CCGTCTGAAA TTATGATTCG TGAAGTAAAA TAAGCCTACA TAACCACGGA ATACCAGATA     480
   ACACAGAAGG AAAACAAGCG GTAGAATTTG CAGAAAAAGT TGCAAATTCT ACCGCTTTTT     540
   TATTAGTACG ATTCGCTGTT GGACTGCCAT TTGATTTGGT TTGTCAGGAT ATTATGTTAT     600
   TGTAATGAAA TGTTAGTGAA TTATTTTTAT TAATTTGAAA GGAGACAAAA TGAAAATAAA   660
   AAAACGTTAC ATTGCGCTGC TAGCTTTAGG CAGTGTTATT GGCTATGCCT GGTATCAAAA     720
   TTATCAATGG GAACAGTTGA TGTTAAGTGG CTATTGTGAA AAGGACGGAA GCTATTGTGA     780
   TGATAGGCAT ACGAAGCAGG AACTGATTGA TAGGGCAATT AACTATGTGC TGGAAAATCA     840
   AATTCAACAG ACATATGAAG GTGATGACCT TGTGGATATA AAACAATATT CAACAATAGA     900
   GGAATTTAAA AAACTAAATC CGAATTGTTG TAAGGTAGAT TCTTGGCCGG ATGATGCTGT     960
   TCGTGAGGAT GCTGATTTAC AGCGAGAGGG CAAAGCGTAT AAATACGTAA AAGTCAAATA    1020
   TTTAAGAACC TATTTAGCGA ATAGAGAACC TGAACAATGG GAAAATTACA TAGTATTTGA    1080
   TAATTGCAGT GGAATTAAAG AAAGACACCA ACTGTATTAA AAATAGATTA GATGGAGACA 1140
   ACACGATGAC AAAACTAACT ATCCAAGATG TGACTAATTT ATATTTATAT AAGCAAAGAA 1200
   CTTTACCTAC GGATAGGTTA GATGATTCGC TTATTAGCAA AACAGGAAAA GGGGAAAATA 1260
   TTGATAAAAA GGAATTTATG GCGGGGCCGG GACGTTTTGT GACGGCCGAT AATTTTAGTG 1320
   TTGTAAAAGA CTTTTTTACT GCAAAGGATT CATTAATAAA CCTAAGCTTG CAGACTCGTA 1380
   TATTAGCGAA TTTAAAGCCG GGCAAATATT CCAAAGCGCA GATATTAGAA ATGTTGGGCT 1440
   ATACGAAAAA TGGAGAAAAG GTAGATGGCA TGTTTACCGG TGAAGTCCAG ACATTAGGCT 1500
   TTTATGACGA TGGCAAAGGG GATTTACTCG AACGCGCCTA TATCTGAAAT ACCACAGGAT 1560
   TTAAA ATG AGC GAC AAT GCC TTT TTT GTT ATA GAA GAA TCA GGC AAA      1607
         Met Ser Asp Asn Ala Phe Phe Val Ile Glu Glu Ser Gly Lys
             1660                1665                1670
   CGC TAT ATT GAA AAC TTT GGT ATT GAA CCT CTT GGT AAG CAA GAA GAT     1655
   Arg Tyr Ile Glu Asn Phe Gly Ile Glu Pro Leu Gly Lys Gln Glu Asp
           1675                1680                1685
   TTT GAT TTT GTC GGC GGC TTT TGG TCT AAC TTA GTG AAT CGT GGT TTG     1703
   Phe Asp Phe Val Gly Gly Phe Trp Ser Asn Leu Val Asn Arg Gly Leu
       1690                1695                1700
   GAA AGT ATT ATC GAC CCA TCC GGT ATC GGT GGA ACG GTA AAC CTT AAC     1751
   Glu Ser Ile Ile Asp Pro Ser Gly Ile Gly Gly Thr Val Asn Leu Asn
   1705                1710                1715                1720
   TTT ACC GGC GAG GTG GAA ACC TAC ACG TTA GAC GAA ACA AGG TTT AAA     1799
   Phe Thr Gly Glu Val Glu Thr Tyr Thr Leu Asp Glu Thr Arg Phe Lys
               1725                1730                1735
   GCG GAA GCG GCG AAG AAA AGC CAT TGG AGT TTA GTG AAT GCG GCG AAA     1847
   Ala Glu Ala Ala Lys Lys Ser His Trp Ser Leu Val Asn Ala Ala Lys
               1740                1745                1750
   GTA TAC GGC GGT TTA GAC CAA ATT ATT AAA AAA CTA TGG GAC AGT GGC     1895
   Val Tyr Gly Gly Leu Asp Gln Ile Ile Lys Lys Leu Trp Asp Ser Gly
           1755                1760                1765
   TCA ATT AAG CAT TTA TAT CAA GAT AAA GAT ACG GGC AAA TTA AAA CCG     1943
   Ser Ile Lys His Leu Tyr Gln Asp Lys Asp Thr Gly Lys Leu Lys Pro
       1770                1775                1780
   ATT ATT TAC GGC ACG GCC GGC AAC GAC AGT AAG ATT GAA GGC ACT AAA     1991
   Ile Ile Tyr Gly Thr Ala Gly Asn Asp Ser Lys Ile Glu Gly Thr Lys
   1785                1790                1795                1800
   ATC ACC CGT AGG ATT GCG GGT AAA GAA GTT ACG CTT GAT ATT GCC AAT     2039
   Ile Thr Arg Arg Ile Ala Gly Lys Glu Val Thr Leu Asp Ile Ala Asn
               1805                1810                1815
   CAG AAA ATT GAA AAA GGC GTG TTA GAG AAA TTG GGG CTG TCT GTT AGT     2087
   Gln Lys Ile Glu Lys Gly Val Leu Glu Lys Leu Gly Leu Ser Val Ser
               1820                1825                1830
   GGT TCG GAT ATC ATT AAA TTG TTG TTT GGA GCA TTG ACT CCA ACT TTA     2135
   Gly Ser Asp Ile Ile Lys Leu Leu Phe Gly Ala Leu Thr Pro Thr Leu
           1835                1840                1845
   AAT AGA ATG TTG CTA TCA CAA CTT ATC CAG TCT TTT TCC GAT AGC TTG     2183
   Asn Arg Met Leu Leu Ser Gln Leu Ile Gln Ser Phe Ser Asp Ser Leu
       1850                1855                1860
   GCT AAA CTT GAT AAT CCC TTA GCC CCT TAC ACT AAA AAT GGC GTG GTT     2231
   Ala Lys Leu Asp Asn Pro Leu Ala Pro Tyr Thr Lys Asn Gly Val Val
   1865                1870                1875                1880
   TAT GTC ACC GGC AAA GGG AAT GAT GTG CTT AAA GGA ACT GAA CAT GAG     2279
   Tyr Val Thr Gly Lys Gly Asn Asp Val Leu Lys Gly Thr Glu His Glu
                   1885                1890                1895
   GAT TTG TTT CTC GGT GGT GAG GGG AAT GAT ACT TAT TAT GCG AGA GTA     2327
   Asp Leu Phe Leu Gly Gly Glu Gly Asn Asp Thr Tyr Tyr Ala Arg Val
               1900                1905                1910
   GGC GAT ACA ATT GAA GAC GCC GAC GGC AAA GGT AAA GTC TAT TTT GTG     2375
   Gly Asp Thr Ile Glu Asp Ala Asp Gly Lys Gly Lys Val Tyr Phe Val
           1915                1920                1925
   AGA GAA AAA GGG GTA CCT AAG GCG GAT CCT AAG CGG GTA GAG TTT AGC     2423
   Arg Glu Lys Gly Val Pro Lys Ala Asp Pro Lys Arg Val Glu Phe Ser
       1930                1935                1940
   GAG TAC ATA ACG AAA GAA GAA ATA AAA GAG GTT GAA AAG GGG TTA TTA     2471
   Glu Tyr Ile Thr Lys Glu Glu Ile Lys Glu Val Glu Lys Gly Leu Leu
   1945                1950                1955                1960
   ACT TAC GCA GTT TTA GAA AAT TAT AAT TGG GAA GAG AAA ACG GCG ACT     2519
   Thr Tyr Ala Val Leu Glu Asn Tyr Asn Trp Glu Glu Lys Thr Ala Thr
                   1965                1970                1975
   TTC GCT CAT GCG ACT ATG CTT AAT GAG CTT TTT ACT GAT TAT ACT AAT     2567
   Phe Ala His Ala Thr Met Leu Asn Glu Leu Phe Thr Asp Tyr Thr Asn
               1980                1985                1990
   TAT CGT TAT GAA GTT AAA GGA CTA AAA TTG CCC GCC GTT AAA AAG TTA     2615
   Tyr Arg Tyr Glu Val Lys Gly Leu Lys Leu Pro Ala Val Lys Lys Leu
           1995                2000                2005
   AAA AGT CCG TTG GTG GAG TTT ACA GCT GAT TTA TTA ACT GTT ACG CCT     2663
   Lys Ser Pro Leu Val Glu Phe Thr Ala Asp Leu Leu Thr Val Thr Pro
       2010                2015                2020
   ATT GAC GAA AAC GGA AAA GCA CTT AGC GAA AAA AGT ATT ACG GTT AAA     2711
   Ile Asp Glu Asn Gly Lys Ala Leu Ser Glu Lys Ser Ile Thr Val Lys
   2025                2030                2035                2040
   AAT TTT AAA AAT GGT GAT TTA GGA ATA AGG TTG TTG GAT CCT AAT AGC     2759
   Asn Phe Lys Asn Gly Asp Leu Gly Ile Arg Leu Leu Asp Pro Asn Ser
                   2045                2050                2055
   TAT TAT TAT TTC CTT GAA GGC CAA GAT ACG GGT TTT TAT GGT CCT GCT     2807
   Tyr Tyr Tyr Phe Leu Glu Gly Gln Asp Thr Gly Phe Tyr Gly Pro Ala
               2060                2065                2070
   TTT TAT ATT GAA CGA AAA AAC GGT GGA GGC TCT AAA AAT AAC TCG TCG     2855
   Phe Tyr Ile Glu Arg Lys Asn Gly Gly Gly Ser Lys Asn Asn Ser Ser
           2075                2080                2085
   GGA GCA GGA AAT AGC AAA GAT TGG GGC GGG AAC GGG CAT GGA AAT CAC     2903
   Gly Ala Gly Asn Ser Lys Asp Trp Gly Gly Asn Gly His Gly Asn His
       2090                2095                2100
   CGA AAT AAT GCC TCC GAC CTG AAT AAA CCG GAC GGA AAT AAT GGG AAT     2951
   Arg Asn Asn Ala Ser Asp Leu Asn Lys Pro Asp Gly Asn Asn Gly Asn
   2105                2110                2115                2120
   AAC CAA AAT AAC GGA AGC AAT CAA GAT AAT CAT AGC GAT GTG AAT GCG     2999
   Asn Gln Asn Asn Gly Ser Asn Gln Asp Asn His Ser Asp Val Asn Ala
                   2125                2130                2135
   CCA AAT AAC CCG GGA CGT AAC TAT GAT ATT TAC GAT CCT TTA GCT TTA     3047
   Pro Asn Asn Pro Gly Arg Asn Tyr Asp Ile Tyr Asp Pro Leu Ala Leu
               2140                2145                2150
   GAT TTA GAT GGA GAT GGG CTT GAA ACC GTG TCG ATG AAC GGG CGA CAA     3095
   Asp Leu Asp Gly Asp Gly Leu Glu Thr Val Ser Met Asn Gly Arg Gln
           2155                2160                2165
   GGC GCG TTA TTC GAT CAT GAA GGA AAA GGT ATT CGT ACC GCA ACG GGC     3143
   Gly Ala Leu Phe Asp His Glu Gly Lys Gly Ile Arg Thr Ala Thr Gly
       2170                2175                2180
   TGG CTC GCT GCG GAT GAC GGT TTT TTA GTG TTA GAT CGT AAC CAA GAC     3191
   Trp Leu Ala Ala Asp Asp Gly Phe Leu Val Leu Asp Arg Asn Gln Asp
   2185                2190                2195                2200
   GGC ATT ATT AAT GAT ATA AGC GAG TTA TTT AGT AAT AAA AAT CAA CTT     3239
   Gly Ile Ile Asn Asp Ile Ser Glu Leu Phe Ser Asn Lys Asn Gln Leu
                   2205                2210                2215
   TCC GAC GGG AGT ATT TCT GCA CAC GGT TTT GCG ACA TTA GCC GAT TTG     3287
   Ser Asp Gly Ser Ile Ser Ala His Gly Phe Ala Thr Leu Ala Asp Leu
               2220                2225                2230
   GAT ACA AAC CAA GAT CAG CGT ATC GAC CAA AAT GAT AAG CTG TTT TCT     3335
   Asp Thr Asn Gln Asp Gln Arg Ile Asp Gln Asn Asp Lys Leu Phe Ser
           2235                2240                2245
   AAA CTC CAA ATT TGG CGG GAT TTA AAT CAA AAC GGT TTT AGT GAA GCG     3383
   Lys Leu Gln Ile Trp Arg Asp Leu Asn Gln Asn Gly Phe Ser Glu Ala
       2250                2255                2260
   AAT GAG CTG TTT AGC TTA GAA AGT TTG AAT ATT AAA TCT TTA CAT ACC     3431
   Asn Glu Leu Phe Ser Leu Glu Ser Leu Asn Ile Lys Ser Leu His Thr
   2265                2270                2275                2280
   GCC TAT GAA GAG CGT AAT GAT TTT CTA GCG GGC AAT AAT ATC CTT GCT     3479
   Ala Tyr Glu Glu Arg Asn Asp Phe Leu Ala Gly Asn Asn Ile Leu Ala
                   2285                2290                2295
   CAG CTT GGG AAG TAT GAA AAA ACG GAC GGT ACT TTT GGA CAA ATG GGC     3527
   Gln Leu Gly Lys Tyr Glu Lys Thr Asp Gly Thr Phe Gly Gln Met Gly
               2300                2305                2310
   GAT TTA AAT TTC AGT TTT AAC CCG TTT TAT AGC CGA TTT ACC GAA GCG     3575
   Asp Leu Asn Phe Ser Phe Asn Pro Phe Tyr Ser Arg Phe Thr Glu Ala
           2315                2320                2325
   TTA AAT TTA ACC GAG CAA CAA CGT CGC ACA ATT AAT CTA ACC GGC ACC     3623
   Leu Asn Leu Thr Glu Gln Gln Arg Arg Thr Ile Asn Leu Thr Gly Thr
       2330                2335                2340
   GGT CGG GTT CGG GAT TTG CGT GAA GCC GCC GCA CTT TCT GAG GAG TTG     3671
   Gly Arg Val Arg Asp Leu Arg Glu Ala Ala Ala Leu Ser Glu Glu Leu
   2345                2350                2355                2360
   GCT GCT TTA TTA CAA CAG TAC ACT AAG GGC TCC GAT TTT CAG GCA CAA     3719
   Ala Ala Leu Leu Gln Gln Tyr Thr Lys Gly Ser Asp Phe Gln Ala Gln
                   2365                2370                2375
   CGA GAA TTA TTG CCT GCC ATT TTA GAT AAA TGG GCG GCA ACG GAT TTA     3767
   Arg Glu Leu Leu Pro Ala Ile Leu Asp Lys Trp Ala Ala Thr Asp Leu
               2380                2385                2390
   CAG TAT CAA CAT TAT GAT AAA ACA TTA CTT AAA ACG GTA GAA AGT ACC     3815
   Gln Tyr Gln His Tyr Asp Lys Thr Leu Leu Lys Thr Val Glu Ser Thr
           2395                2400                2405
   GAT AGT AGT GCT TCT GTC GTT AGA GTC ACG CCT TCT CAA TTA AGT AGT      3863
   Asp Ser Ser Ala Ser Val Val Arg Val Thr Pro Ser Gln Leu Ser Ser
       2410                2415                2420
   ATA CGC AAT GTA AAG CAT GAT CCT ACC GTT ATG CAA AAC TGT GAA CAA      3911
   Ile Arg Asn Val Lys His Asp Pro Thr Val Met Gln Asn Cys Glu Gln
   2425                2430                2435                2440
   AGT AAG GCA AAA ATT GCG ACT TTA AAT TCG CTC TAC GGG TTA AAT ATT      3959
   Ser Lys Ala Lys Ile Ala Thr Leu Asn Ser Leu Tyr Gly Leu Asn Ile
                   2445                2450                2455
   GAT CAA CTT TAT TAT ACG ACG GAT AAA GAC ATT CGT TAT ATT ACT GAC      4007
   Asp Gln Leu Tyr Tyr Thr Thr Asp Lys Asp Ile Arg Tyr Ile Thr Asp
               2460                2465                2470
   AAA GTG AAT AAT ATG TAT CAA ACA ACC GGA GAA CTC GGC TAT CGT TCT      4055
   Lys Val Asn Asn Met Tyr Gln Thr Thr Gly Glu Leu Gly Tyr Arg Ser
           2475                2480                2485
   TTA CTT TTA CAA ACG CGT GTG AAG AAA TAT GTT TAT AGC GTT AAT GCG      4103
   Leu Leu Leu Gln Thr Arg Val Lys Lys Tyr Val Tyr Ser Val Asn Ala
       2490                2495                2500
   AAA CAA TTC GAA GGG AAA TGG GTA GCC GAT TAT TCT CGT ACT GAA GCC      4151
   Lys Gln Phe Glu Gly Lys Trp Val Ala Asp Tyr Ser Arg Thr Glu Ala
   2505                2510                2515                2520
   TTA TTT AAC TCT ACT TAT AAA CAA TCG CCC GAA AAT GTA TTA TAT GAT      4199
   Leu Phe Asn Ser Thr Tyr Lys Gln Ser Pro Glu Asn Val Leu Tyr Asp
                   2525                2530                2535
   TTA CGC GAA TAC CTT TCT TTC TAT AAC GAC CCT ACG GAA TGG AAA GAA      4247
   Leu Arg Glu Tyr Leu Ser Phe Tyr Asn Asp Pro Thr Glu Trp Lys Glu
               2540                2545                2550
   GGG CTA TTA CTG TTA AGC CGT TAT ATA GAT TAT GCT AAA GCA CAA GGA      4295
   Gly Leu Leu Leu Leu Ser Arg Tyr Ile Asp Tyr Ala Lys Ala Gln Gly
           2555                2560                2565
   TTT TAT GAA AAC TGG GCG GCT ACT TCT AAC TTA ACT ATT GCC CGT TTA      4343
   Phe Tyr Glu Asn Trp Ala Ala Thr Ser Asn Leu Thr Ile Ala Arg Leu
       2570                2575                2580
   AGA GAG GCT GGA GTA ATT TGT GCA GAA TCG ACG GAT TTA AAA GGC GAT      4391
   Arg Glu Ala Gly Val Ile Cys Ala Glu Ser Thr Asp Leu Lys Gly Asp
   2585                2590                2595                2600
   GAA AAA AAT AAT ATT GTG TTA GGT AGC CAA AAA GAT AAT AAC TTA TCG      4439
   Glu Lys Asn Asn Ile Val Leu Gly Ser Gln Lys Asp Asn Asn Leu Ser
                   2605                2610                2615
   GGT AGT GCA GGT GAT GAT CTA CTT ATC GGC GGA GAG GGT AAT GAT ACG      4487
   Gly Ser Ala Gly Asp Asp Leu Leu Ile Gly Gly Glu Gly Asn Asp Thr
               2620                2625                2630
   TTA AAA GGC AGC TAC GGT GCA GAC ACC TAT ATC TTT AGC AAA GGG CAT      4535
   Leu Lys Gly Ser Tyr Gly Ala Asp Thr Tyr Ile Phe Ser Lys Gly His
           2635                2640                2645
   GGA CAA GAT GTA ATT TAT GAA TAT TCC GAC AGT GCA AAC TCT AAA AAA      4583
   Gly Gln Asp Val Ile Tyr Glu Tyr Ser Asp Ser Ala Asn Ser Lys Lys
       2650                2655                2660
   GAT ATT GAT ACC TTA AAA TTT ACC GAT GTG AAT TAT GCG GAA GTG AAG      4631
   Asp Ile Asp Thr Leu Lys Phe Thr Asp Val Asn Tyr Ala Glu Val Lys
   2665                2670                2675                2680
   TTT CGA CGA GTA GAT AAT GAC TTA ATG TTA TTC GGT TAT CAT GAT ACG     4679
   Phe Arg Arg Val Asp Asn Asp Leu Met Leu Phe Gly Tyr His Asp Thr
                   2685                2690                2695
   GAT TCG GTC ACG GTA AAA TCC TTC TAC AGC CAT GTA GAT TAT CAA TTT     4727
   Asp Ser Val Thr Val Lys Ser Phe Tyr Ser His Val Asp Tyr Gln Phe
               2700                2705                2710
   GAC AAA TTG GAG TTT GCT GAC CGC AGT ATA ACT CGC GAT GAA CTG ATT     4775
   Asp Lys Leu Glu Phe Ala Asp Arg Ser Ile Thr Arg Asp Glu Leu Ile
           2715                2720                2725
   AAA GCA GGG CTT CAT CTA TAC GGC ACC GAT GGC AAT GAT GAT ATA AAG     4823
   Lys Ala Gly Leu His Leu Tyr Gly Thr Asp Gly Asn Asp Asp Ile Lys
       2730                2735                2740
   GAT CAT GCG GAT TGG GAC AGC ATT GTG GAA GGC GGC AAA GGC AAC GAT     4871
   Asp His Ala Asp Trp Asp Ser Ile Val Glu Gly Gly Lys Gly Asn Asp
   2745                2750                2755                2760
   ATT CTA AGA GGT GGC TAC GGT GCG GAC ACC TAT ATC TTT AGC AAA GGA     4919
   Ile Leu Arg Gly Gly Tyr Gly Ala Asp Thr Tyr Ile Phe Ser Lys Gly
                   2765                2770                2775
   CAC GGA CAG GAT ATC GTT TAT GAA GAT ACC AAT AAT GAT AAC CGA GCA     4967
   His Gly Gln Asp Ile Val Tyr Glu Asp Thr Asn Asn Asp Asn Arg Ala
               2780                2785                2790
   AGA GAT ATC GAC ACC TTA ACA TTT ACT GAT GTG AAT TAT GCG GAA GTG     5015
   Arg Asp Ile Asp Thr Leu Thr Phe Thr Asp Val Asn Tyr Ala Glu Val
           2795                2800                2805
   AAA TTC CGA CGA GTA GAT AAT GAC TTA ATG TTA TTC GGT TAT CAT GAT     5063
   Lys Phe Arg Arg Val Asp Asn Asp Leu Met Leu Phe Gly Tyr His Asp
       2810                2815                2820
   ACG GAT TCG GTC ACG ATA AAA TCC TTC TAC AAC CAT GTA GAT TAT CAA     5111
   Thr Asp Ser Val Thr Ile Lys Ser Phe Tyr Asn His Val Asp Tyr Gln
   2825                2830                2835                2840
   TGT GAC AAA TTG GAC TTT GCT GAC CGC AGT ATA ACT CGT GAT GAA CTA     5159
   Cys Asp Lys Leu Asp Phe Ala Asp Arg Ser Ile Thr Arg Asp Glu Leu
                   2845                2850                2855
   GGT AAA CAA GGT ATG GCA TTA TTT GGC ACT GAC GGC GAT GAT AAT ATC     5207
   Gly Lys Gln Gly Met Ala Leu Phe Gly Thr Asp Gly Asp Asp Asn Ile
               2860                2865                2870
   AAC GAC TGG GGA CGT AAC TCG GTG ATT GAT GCC GGT GCG GGT AAT GAT     5255
   Asn Asp Trp Gly Arg Asn Ser Val Ile Asp Ala Gly Ala Gly Asn Asp
           2875                2880                2885
   ACG GTT AAT GGC GGT AAT GGC GAT GAC ACC CTC ATC GGC GGC AAA GGT     5303
   Thr Val Asn Gly Gly Asn Gly Asp Asp Thr Leu Ile Gly Gly Lys Gly
       2890                2895                2900
   AAT GAT ATT CTA AGA GGT GGC TAC GGT GCG GAC ACC TAT ATC TTT AGC     5351
   Asn Asp Ile Leu Arg Gly Gly Tyr Gly Ala Asp Thr Tyr Ile Phe Ser
   2905                2910                2915                2920
   AAA GGA CAC GGA CAG GAT ATC GTT TAT GAA GAT ACC AAT AAT GAT AAC     5399
   Lys Gly His Gly Gln Asp Ile Val Tyr Glu Asp Thr Asn Asn Asp Asn
                   2925                2930                2935
   CGC GCA AGA GAT ATC GAC ACC TTA AAA TTT ACT GAT ATT AAT TTA TCC     5447
   Arg Ala Arg Asp Ile Asp Thr Leu Lys Phe Thr Asp Ile Asn Leu Ser
               2940                2945                2950
   GAA CTT TGG TTT AGC CGA GAA AAT AAC GAT TTG ATT ATT AAA TCA TTA      5495
   Glu Leu Trp Phe Ser Arg Glu Asn Asn Asp Leu Ile Ile Lys Ser Leu
             2955                2960                2965
   TTA AGT GAG GAT AAA GTC ACG GTT CAA AAT TGG TAT TCA CAC CAA GAT      5543
   Leu Ser Glu Asp Lys Val Thr Val Gln Asn Trp Tyr Ser His Gln Asp
        2970                2975                2980
   CAT AAA ATA GAA AAT ATT CGT TTA TCG AAT GAG CAA ATG TTG GTG AGC      5591
   His Lys Ile Glu Asn Ile Arg Leu Ser Asn Glu Gln Met Leu Val Ser
   2985                2990                2995                3000
   ACT CAG GTG GAG AAG ATG GTT GAG TCG ATG GCC GGC TTT GCT CAG AAG      5639
   Thr Gln Val Glu Lys Met Val Glu Ser Met Ala Gly Phe Ala Gln Lys
                   3005                3010                3015
   CAC GGA GGA GAG ATA TCT CTT CTG TCG CCT GAA GAG GTA AAA CAA TAT      5687
   His Gly Gly Glu Ile Ser Leu Leu Ser Pro Glu Glu Val Lys Gln Tyr
              3020                3025                3030
   ATC AAT AGC TTA ACA GCT GCT TTA TAA CATACGAAAG AAATCGGCAC            5734
   Ile Asn Ser Leu Thr Ala Ala Leu  *
        3035                3040
   AGTTTTTGTG AACTGTGCCG ATTTGATTTT AGTGTAAGAA TATAGCCTGA TTTTAAGAAA    5794
   TTTACTCTTG GCTAATAACT ATTTCCCATT TTATAAGTTA TTGACGGATG GTTTTATCAA    5854
   ATATGAGATC AAATCTTATT TTAAATTCGC TTTCCATTAA GCGATATTGA TCTTTTAAGT    5914
   TTGGGGCCGC ATGAGTTTGG AACCGATACC ACTCATTGTG GGAATCAATA CACAATACGC    5974
   TGTAATCGGA CTCTTGCAGT TCATAATAAT GCTTTCTCTC CGTTAATTCT TCTTGCGTAT    6034
   ATGGCGAGAG ATTAAAGCTG AATGGCTGGT TCGCACTAAC AAACAGGTTC TCCGATTTCA    6094
   GATATTCACA ACCGTAATGG CTACCGGTTT CCTGCGGTTT TACATAATTG GTATGATTTT    6154
   GTTTAGCTGT TATACGGTAG ATGCCTAATT GTGGTAAATT GCGTGTGTCA ATATAGCTTT    6214
   CTTGTTCTCC GTAACCGAAA TACTCAATGG CGTTTTCTGT TTTAGCTAAG AAGAAACGTA    6274
   AGCCGAAGCG GGGTAAATAC GGTAATTCGA TCGGGCGAAT AGCGTTAATT TCAACCGAAA    6334
   GTTGTCCGTC ATTGAAGATA CGATAACGAA TATCCAGTGT TAAAATGCGA CCGCGAGAAA    6394
   TTGACACAAT TGCAGATTTT ACTGAAAATT CGACCGCTTG TTCGCTTTGC TGCCACTGAA    6454
   TTTCATACGC TCTGGTATAG GCTTTATCGT AGCCGGCATT TTGGCACGCC TCACGAATGA    6514
   GGCGATCATT GTCGGTTGGC GCACGCCAAA TATTAAAATC TAACGATTGT TGGATAATCG    6574
   CTTTACCGGC TTTTTCAATA CGGGTGAAAA TCCCTTTCTG TTTATCTAAT TGATAACTAA    6634
   ATTGACCGTT GTGTACGTTA ATGTGGAAGC GATCTTCTTG TACTTCAAAT GCACTGTTCT    6694
   CAATTGTGAA TTGTGGTAAT ACTAATTTAT TTTCGCTAAA TAAATTGAGC TGCTCGAAGC    6754
   CAAGTGAATG TGCTTCGTCT AATAATTCGA CCGCGGTATT TAAGCGATAA TTTAAATTCA    6814
   GTAGCCATAA ATGCCCGTTA TTTTTTGGTA ACTCAATCGG TAATACTACG CTGCCGTGCG    6874
   GTTGGCAAGA AACGGATAAA TTCCCACCGC TTGTCACCAC GCCGTTTTCG ACAAATTCGT    6934
   AATCAATCGT TAAATAATCG GCAAGATCAG TGAAATCCAA GTAGTTGTGG ATCACAATTT    6994
   GGTTATCGAT                                                           7004
(2) INFORMATION FOR SEQ ID NO: 4:
   (i) SEQUENCE CHARACTERISTICS:
      (A) LENGTH: 1383 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Ser Asp Asn Ala Phe Phe Val Ile Glu Glu Ser Gly Lys Arg Tyr
   1               5                   10                  15
   Ile Glu Asn Phe Gly Ile Glu Pro Leu Gly Lys Gln Glu Asp Phe Asp
               20                  25                  30
   Phe Val Gly Gly Phe Trp Ser Asn Leu Val Asn Arg Gly Leu Glu Ser
           35                  40                  45
   Ile Ile Asp Pro Ser Gly Ile Gly Gly Thr Val Asn Leu Asn Phe Thr
       50                  55                  60
   Gly Glu Val Glu Thr Tyr Thr Leu Asp Glu Thr Arg Phe Lys Ala Glu
   65                  70                  75                  80
   Ala Ala Lys Lys Ser His Trp Ser Leu Val Asn Ala Ala Lys Val Tyr
                   85                  90                  95
   Gly Gly Leu Asp Gln Ile Ile Lys Lys Leu Trp Asp Ser Gly Ser Ile
               100                 105                 110
   Lys His Leu Tyr Gln Asp Lys Asp Thr Gly Lys Leu Lys Pro Ile Ile
           115                 120                 125
   Tyr Gly Thr Ala Gly Asn Asp Ser Lys Ile Glu Gly Thr Lys Ile Thr
       130                 135                 140
   Arg Arg Ile Ala Gly Lys Glu Val Thr Leu Asp Ile Ala Asn Gln Lys
   145                 150                 155                 160
   Ile Glu Lys Gly Val Leu Glu Lys Leu Gly Leu Ser Val Ser Gly Ser
                   165                 170                 175
   Asp Ile Ile Lys Leu Leu Phe Gly Ala Leu Thr Pro Thr Leu Asn Arg
               180                 185                 190
   Met Leu Leu Ser Gln Leu Ile Gln Ser Phe Ser Asp Ser Leu Ala Lys
           195                 200                 205
   Leu Asp Asn Pro Leu Ala Pro Tyr Thr Lys Asn Gly Val Val Tyr Val
       210                 215                 220
   Thr Gly Lys Gly Asn Asp Val Leu Lys Gly Thr Glu His Glu Asp Leu
   225                 230                 235                 240
   Phe Leu Gly Gly Glu Gly Asn Asp Thr Tyr Tyr Ala Arg Val Gly Asp
                   245                 250                 255
   Thr Ile Glu Asp Ala Asp Gly Lys Gly Lys Val Tyr Phe Val Arg Glu
               260                 265                 270
   Lys Gly Val Pro Lys Ala Asp Pro Lys Arg Val Glu Phe Ser Glu Tyr
           275                 280                 285
   Ile Thr Lys Glu Glu Ile Lys Glu Val Glu Lys Gly Leu Leu Thr Tyr
       290                 295                 300
   Ala Val Leu Glu Asn Tyr Asn Trp Glu Glu Lys Thr Ala Thr Phe Ala
   305                 310                 315                 320
   His Ala Thr Met Leu Asn Glu Leu Phe Thr Asp Tyr Thr Asn Tyr Arg
                   325                 330                 335
   Tyr Glu Val Lys Gly Leu Lys Leu Pro Ala Val Lys Lys Leu Lys Ser
               340                 345                 350
   Pro Leu Val Glu Phe Thr Ala Asp Leu Leu Thr Val Thr Pro Ile Asp
           355                 360                 365
   Glu Asn Gly Lys Ala Leu Ser Glu Lys Ser Ile Thr Val Lys Asn Phe
       370                 375                 380
   Lys Asn Gly Asp Leu Gly Ile Arg Leu Leu Asp Pro Asn Ser Tyr Tyr
   385                 390                 395                 400
   Tyr Phe Leu Glu Gly Gln Asp Thr Gly Phe Tyr Gly Pro Ala Phe Tyr
                   405                 410                 415
   Ile Glu Arg Lys Asn Gly Gly Gly Ser Lys Asn Asn Ser Ser Gly Ala
               420                 425                 430
   Gly Asn Ser Lys Asp Trp Gly Gly Asn Gly His Gly Asn His Arg Asn
           435                 440                 445
   Asn Ala Ser Asp Leu Asn Lys Pro Asp Gly Asn Asn Gly Asn Asn Gln
       450                 455                 460
   Asn Asn Gly Ser Asn Gln Asp Asn His Ser Asp Val Asn Ala Pro Asn
   465                 470                 475                 480
   Asn Pro Gly Arg Asn Tyr Asp Ile Tyr Asp Pro Leu Ala Leu Asp Leu
                   485                 490                 495
   Asp Gly Asp Gly Leu Glu Thr Val Ser Met Asn Gly Arg Gln Gly Ala
               500                 505                 510
   Leu Phe Asp His Glu Gly Lys Gly Ile Arg Thr Ala Thr Gly Trp Leu
           515                 520                 525
   Ala Ala Asp Asp Gly Phe Leu Val Leu Asp Arg Asn Gln Asp Gly Ile
       530                 535                 540
   Ile Asn Asp Ile Ser Glu Leu Phe Ser Asn Lys Asn Gln Leu Ser Asp
   545                 550                 555                 560
   Gly Ser Ile Ser Ala His Gly Phe Ala Thr Leu Ala Asp Leu Asp Thr
                   565                 570                 575
   Asn Gln Asp Gln Arg Ile Asp Gln Asn Asp Lys Leu Phe Ser Lys Leu
               580                 585                 590
   Gln Ile Trp Arg Asp Leu Asn Gln Asn Gly Phe Ser Glu Ala Asn Glu
           595                 600                 605
   Leu Phe Ser Leu Glu Ser Leu Asn Ile Lys Ser Leu His Thr Ala Tyr
       610                 615                 620
   Glu Glu Arg Asn Asp Phe Leu Ala Gly Asn Asn Ile Leu Ala Gln Leu
   625                 630                 635                 640
   Gly Lys Tyr Glu Lys Thr Asp Gly Thr Phe Gly Gln Met Gly Asp Leu
                   645                 650                 655
   Asn Phe Ser Phe Asn Pro Phe Tyr Ser Arg Phe Thr Glu Ala Leu Asn
               660                 665                 670
   Leu Thr Glu Gln Gln Arg Arg Thr Ile Asn Leu Thr Gly Thr Gly Arg
           675                 680                 685
   Val Arg Asp Leu Arg Glu Ala Ala Ala Leu Ser Glu Glu Leu Ala Ala
   690 695 700
   Leu Leu Gln Gln Tyr Thr Lys Gly Ser Asp Phe Gln Ala Gln Arg Glu
   705 710 715 720
   Leu Leu Pro Ala Ile Leu Asp Lys Trp Ala Ala Thr Asp Leu Gln Tyr
   725 730 735
   Gln His Tyr Asp Lys Thr Leu Leu Lys Thr Val Glu Ser Thr Asp Ser
   740 745 750
   Ser Ala Ser Val Val Arg Val Thr Pro Ser Gln Leu Ser Ser Ile Arg
   755 760 765
   Asn Val Lys His Asp Pro Thr Val Met Gln Asn Cys Glu Gln Ser Lys
   770 775 780
   Ala Lys Ile Ala Thr Leu Asn Ser Leu Tyr Gly Leu Asn Ile Asp Gln
   785 790 795 800
   Leu Tyr Tyr Thr Thr Asp Lys Asp Ile Arg Tyr Ile Thr Asp Lys Val
   805 810 815
   Asn Asn Met Tyr Gln Thr Thr Gly Glu Leu Gly Tyr Arg Ser Leu Leu
   820 825 830
   Leu Gln Thr Arg Val Lys Lys Tyr Val Tyr Ser Val Asn Ala Lys Gln
   835 840 845
   Phe Glu Gly Lys Trp Val Ala Asp Tyr Ser Arg Thr Glu Ala Leu Phe
   850 855 860
   Asn Ser Thr Tyr Lys Gln Ser Pro Glu Asn Val Leu Tyr Asp Leu Arg
   865 870 875 880
   Glu Tyr Leu Ser Phe Tyr Asn Asp Pro Thr Glu Trp Lys Glu Gly Leu
   885 890 895
   Leu Leu Leu Ser Arg Tyr Ile Asp Tyr Ala Lys Ala Gln Gly Phe Tyr
   900 905 910
   Glu Asn Trp Ala Ala Thr Ser Asn Leu Thr Ile Ala Arg Leu Arg Glu
   915 920 925
   Ala Gly Val Ile Cys Ala Glu Ser Thr Asp Leu Lys Gly Asp Glu Lys
   930 935 940
   Asn Asn Ile Val Leu Gly Ser Gln Lys Asp Asn Asn Leu Ser Gly Ser
   945 950 955 960
   Ala Gly Asp Asp Leu Leu Ile Gly Gly Glu Gly Asn Asp Thr Leu Lys
   965 970 975
   Gly Ser Tyr Gly Ala Asp Thr Tyr Ile Phe Ser Lys Gly His Gly Gln
   980 985 990
   Asp Val Ile Tyr Glu Tyr Ser Asp Ser Ala Asn Ser Lys Lys Asp Ile
   995 1000 1005
   Asp Thr Leu Lys Phe Thr Asp Val Asn Tyr Ala Glu Val Lys Phe Arg
   1010 1015 1020
   Arg Val Asp Asn Asp Leu Met Leu Phe Gly Tyr His Asp Thr Asp Ser
   1025 1030 1035 1040
   Val Thr Val Lys Ser Phe Tyr Ser His Val Asp Tyr Gln Phe Asp Lys
   1045 1050 1055
   Leu Glu Phe Ala Asp Arg Ser Ile Thr Arg Asp Glu Leu Ile Lys Ala
   1060 1065 1070
   Gly Leu His Leu Tyr Gly Thr Asp Gly Asn Asp Asp Ile Lys Asp His
   1075 1080 1085
   Ala Asp Trp Asp Ser Ile Val Glu Gly Gly Lys Gly Asn Asp Ile Leu
   1090 1095 1100
   Arg Gly Gly Tyr Gly Ala Asp Thr Tyr Ile Phe Ser Lys Gly His Gly
   1105 1110 1115 1120
   Gln Asp Ile Val Tyr Glu Asp Thr Asn Asn Asp Asn Arg Ala Arg Asp
   1125 1130 1135
   Ile Asp Thr Leu Thr Phe Thr Asp Val Asn Tyr Ala Glu Val Lys Phe
   1140 1145 1150
   Arg Arg Val Asp Asn Asp Leu Met Leu Phe Gly Tyr His Asp Thr Asp
   1155 1160 1165
   Ser Val Thr Ile Lys Ser Phe Tyr Asn His Val Asp Tyr Gln Cys Asp
   1170 1175 1180
   Lys Leu Asp Phe Ala Asp Arg Ser Ile Thr Arg Asp Glu Leu Gly Lys
   1185 1190 1195 1200
   Gln Gly Met Ala Leu Phe Gly Thr Asp Gly Asp Asp Asn Ile Asn Asp
   1205 1210 1215
   Trp Gly Arg Asn Ser Val Ile Asp Ala Gly Ala Gly Asn Asp Thr Val
   1220 1225 1230
   Asn Gly Gly Asn Gly Asp Asp Thr Leu Ile Gly Gly Lys Gly Asn Asp
   1235 1240 1245
   Ile Leu Arg Gly Gly Tyr Gly Ala Asp Thr Tyr Ile Phe Ser Lys Gly
   1250 1255 1260
   His Gly Gln Asp Ile Val Tyr Glu Asp Thr Asn Asn Asp Asn Arg Ala
   1265 1270 1275 1280
   Arg Asp Ile Asp Thr Leu Lys Phe Thr Asp Ile Asn Leu Ser Glu Leu
   1285 1290 1295
   Trp Phe Ser Arg Glu Asn Asn Asp Leu Ile Ile Lys Ser Leu Leu Ser
   1300 1305 1310
   Glu Asp Lys Val Thr Val Gln Asn Trp Tyr Ser His Gln Asp His Lys
   1315 1320 1325
   Ile Glu Asn Ile Arg Leu Ser Asn Glu Gln Met Leu Val Ser Thr Gln
   1330 1335 1340
   Val Glu Lys Met Val Glu Ser Met Ala Gly Phe Ala Gln Lys His Gly
   1345 1350 1355 1360
   Gly Glu Ile Ser Leu Leu Ser Pro Glu Glu Val Lys Gln Tyr Ile Asn
   1365 1370 1375
   Ser Leu Thr Ala Ala Leu *
   1380
(2) INFORMATION FOR SEQ ID NO: 5:
   (i) SEQUENCE CHARACTERISTICS:
      (A) LENGTH: 6736 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Actinobacillus pleuropneumoniae
      (B) STRAIN: 4074 (serotype 1 reference strain)
   (vii) IMMEDIATE SOURCE:
      (B) CLONE: pROK7
   (ix) FEATURE:
      (A) NAME/KEY: CDS
      (B) LOCATION:1132..6549
      (C) IDENTIFICATION METHOD: experimental
      (D) OTHER INFORMATION:/codon start= 1132
         /function= "RTX toxin"
         /product= "ApxIV"
         /evidence= EXPERIMENTAL
         /gene= "ApxIV_v1"
   (ix) FEATURE:
      (A) NAME/KEY: CDS
      (B) LOCATION:639..1178
      (D) OTHER INFORMATTON:/codon_start= 639
      /function= "unknown"
      /product= "ORF1"
      /gene= "orf1"
   (ix) FEATURE:
      (A) NAME/KEY: CDS
      (B) LOCATION:1..453
      (D) OTHER INFORMATION:/partial
         /product= "Met-G"
         /gene= "mrp"
         /standard name= "mrp"
         /label= mrp
   (ix) FEATURE:
      (A) NAME/KEY: -10 signal
      (B) LOCATION:617..623
      (D) OTHER INFORMATION:/standard_ name= "-10"
         /label= -10_s
   (ix) FEATURE:
      (A) NAME/KEY: -35 signal
      (B) LOCATION:594..599
      (D) OTHER INFORMATION:/standard_name= "-35 s"
      (D) OTHER INFORMATION: name= "-35_ s"
         /label= -35_s
   (ix) FEATURE:
      (A) NAME/KEY: promoter
      (B) LOCATION:454..1131
      (D) OTHER INFORMATION:/function= "Promoter"
         /standard name= "promoter ApxIV"
         /label= promoter
   (xi) SEQUENCE DESCRIPTION: SEQ ID NO: 5:
   ATC GAT ATG CCG CCG GGT ACG GGC GAT ATC CAA CTT ACT CTT TCG CAA      48
   Ile Asp Met Pro Pro Gly Thr Gly Asp Ile Gln Leu Thr Leu Ser Gln
     1             5                  10                  15
   CAA ATT CCG GTT ACC GGT GCG GTG GTG GTA ACC ACT CCG CAA GAT ATT      96
   Gln Ile Pro Val Thr Gly Ala Val Val Val Thr Thr Pro Gln Asp Ile
                20                  25                  30
   GCG TTA TTA GAT GCG GTG AAA GGT ATT TCA ATG TTC CAA AAA GTG TCG     144
   Ala Leu Leu Asp Ala Val Lys Gly Ile Ser Met Phe Gln Lys Val Ser
            35                  40                  45
   GTA CCG GTC TTA GGT ATC ATT GAA AAT ATG AGC GTA CAT ATC TGC CAA     192
   Val Pro Val Leu Gly Ile Ile Glu Asn Met Ser Val His Ile Cys Gln
        50                  55                  60
   AAT TGC GGT CAC CAC GAA GAT ATT TTC GGC ACC GGC GGT GCG GAG AAA    240
   Asn Cys Gly His His Glu Asp Ile Phe Gly Thr Gly Gly Ala Glu Lys
    65                  70                  75                  80
   GTG GCG AAG AAA TAC GGT ACT AAA GTA TTA GGA CAA ATG CCG TTG CAT    288
   Val Ala Lys Lys Tyr Gly Thr Lys Val Leu Gly Gln Met Pro Leu His
                    85                  90                  95
   ATT CGC TTA CGT CAA GAT TTG GAT GCC GGC ACA CCG ACC GTC GTT GCG    336
   Ile Arg Leu Arg Gln Asp Leu Asp Ala Gly Thr Pro Thr Val Val Ala
               100                 105                 110
   GCA CCG GAA CAC GAA ACC AGC CGA GCC TAT ATT GAA TTA GCG GCA AAA    384
   Ala Pro Glu His Glu Thr Ser Arg Ala Tyr Ile Glu Leu Ala Ala Lys
           115                 120                 125
   GTC GCT TCG GAA TTA TAC TGG CAA GGT TCG GTT ATC CCG TCT GAA ATT    432
   Val Ala Ser Glu Leu Tyr Trp Gln Gly Ser Val Ile Pro Ser Glu Ile
       130                 135                 140
   ATG ATT CGT GAA GTA AAA TAA GTTTTAATAA CCACGAAAAC ACAAAGAACA       483
   Met Ile Arg Glu Val Lys  *
   145                 150
   CAAGCGGTAG AATTTGCAGA AAAATTTGCA AATCCTACCG CTTTTTTATT AGTACGATTC  543
   GCTGTTGGAC TGCTATTTGA TTTGGTTTGT CAGGATATTA TGTTATTGTA ATGAAATGTT  603
   AGTGAATTAT TTTTATTAAT TTGAAAGGAA ACAAAATGAA AATAAAAAAA CGTTACATTG  663
   CGCTGTTGGT CTTAGGTGTC GTTATCAGCT ATGCCTGGTA TCAAAATTAT CAATGGGAAC  723
   AGCTGATGTT AAGCGGTTAT TGTGAAAAGG ACGGAAGTTA TTTTGATGAT AGGCATACGA  783
   AGCAAGAACT GATTGATAGG GCAATTAACT ATATGCTGGA GCATCAATCT AAAAAAACAT  843
   ACGATGCTTA TACTGATGAA CCTTTAGAAA TAAAACCATA TTTAACAATA GAGGAATTTA  903
   AAAAACTCAA TCCAAATTGT TGTGAAATTA CCTCATGGCC AGCAGATGCA GTTCCACAAG  963
   ATTGGGATGT TCGTGTGGAA GGTAAGGCAT ATAGGTATGT AATCGTAAAA TATTTAAGAA  1023
   CCTTAGCAAA TAGAGAACCT GAACGATGGG AAACTAGTAT TGTTTTTGAT AATTGCGGCA  1083
   ATCCTAAAAG AGCAAGCTAC TTATATTATT TAAAGAGAGA AATTTATT ATG ACA AAA   1140
                                                        Met Thr Lys
                                                          1
   TTA ACT ATG CAA GAT GTG ACC AAT TTA TAT TTA TAT AAA ACG AAA ACT   1188
   Leu Thr Met Gln Asp Val Thr Asn Leu Tyr Leu Tyr Lys Thr Lys Thr
         5                  10                  15
   CTA CCT AAA GAT AGA TTG GAT GAT TCA CTT ATT TCT GAA ATA GGA AAA   1236
   Leu Pro Lys Asp Arg Leu Asp Asp Ser Leu Ile Ser Glu Ile Gly Lys
    20                  25                  30                  35
   GGA GAT GAT GAT ATT GAT AGA AAA GAA TTT ATG GTG GGG CCG GGA CGT   1284
   Gly Asp Asp Asp Ile Asp Arg Lys Glu Phe Met Val Gly Pro Gly Arg
                    40                  45                  50
   TTT GTG ACC GCT GAT AAC TTT AGC GTT GTA AGA GAT TTT TTT AAT GCT   1332
   Phe Val Thr Ala Asp Asn Phe Ser Val Val Arg Asp Phe Phe Asn Ala
                55                  60                  65
   GGG AAA TCA CGC ATT ATT GCG CCG CAA GTC CCG CCT ATT CGT TCA CAG   1380
   Gly Lys Ser Arg Ile Ile Ala Pro Gln Val Pro Pro Ile Arg Ser Gln
            70                  75                  80
   CAG GAA AAA ATC TTG GTC GGT TTA AAA CCG GGC AAA TAT TCC AAA GCG   1428
   Gln Glu Lys Ile Leu Val Gly Leu Lys Pro Gly Lys Tyr Ser Lys Ala
        85                  90                  95
   CAG ATA TTG GAA ATG CTG GGT TAT ACG AAA GGC GGA GAA GTG GTA AAT   1476
   Gln Ile Leu Glu Met Leu Gly Tyr Thr Lys Gly Gly Glu Val Val Asn
   100                 105                 110                 115
   GGC ATG TTT GCC GGT GAA GTC CAG ACA TTA GGC TTT TAT GAC GAT GGC   1524
   Gly Met Phe Ala Gly Glu Val Gln Thr Leu Gly Phe Tyr Asp Asp Gly
                   120                 125                 130
   AAA GGG GAT TTA CTC GAA CGC GCC TAT ATC TGG AAT ACC ACA GGA TTT   1572
   Lys Gly Asp Leu Leu Glu Arg Ala Tyr Ile Trp Asn Thr Thr Gly Phe
               135                 140                 145
   AAA ATG AGC GAC AAT GCC TTT TTT GTT ATA GAA GAA TCA GGC AAA CGC   1620
   Lys Met Ser Asp Asn Ala Phe Phe Val Ile Glu Glu Ser Gly Lys Arg
           150                 155                 160
   TAT ATT GAA AAC TTT GGT ATT GAA CCT CTT GGT AAG CAA GAA GAT TTT     1668
   Tyr Ile Glu Asn Phe Gly Ile Glu Pro Leu Gly Lys Gln Glu Asp Phe
       165                 170                 175
   GAT TTT GTC GGC GGC TTT TGG TCT AAC TTA GTG AAT CGT GGT TTG GAA     1716
   Asp Phe Val Gly Gly Phe Trp Ser Asn Leu Val Asn Arg Gly Leu Glu
   180                 185                 190                 195
   AGT ATT ATC GAC CCA TCC GGT ATC GGT GGA ACG GTA AAC CTT AAC TTT     1764
   Ser Ile Ile Asp Pro Ser Gly Ile Gly Gly Thr Val Asn Leu Asn Phe
                   200                 205                 210
   ACC GGC GAG GTG GAA ACC TAC ACG TTA GAC GAA ACA AGG TTT AAA GCG     1812
   Thr Gly Glu Val Glu Thr Tyr Thr Leu Asp Glu Thr Arg Phe Lys Ala
               215                 220                 225
   GAA GCG GCG AAG AAA AGC CAT TGG AGT TTA GTG AAT GCG GCG AAA GTA     1860
   Glu Ala Ala Lys Lys Ser His Trp Ser Leu Val Asn Ala Ala Lys Val
           230                 235                 240
   TAC GGC GGT TTA GAC CAA ATT ATT AAA AAA CTA TGG GAC AGT GGC TCA     1908
   Tyr Gly Gly Leu Asp Gln Ile Ile Lys Lys Leu Trp Asp Ser Gly Ser
       245                 250                 255
   ATT AAG CAT TTA TAT CAA GAT AAA GAT ACG GGC AAA TTA AAA CCG ATT     1956
   Ile Lys His Leu Tyr Gln Asp Lys Asp Thr Gly Lys Leu Lys Pro Ile
   260                 265                 270                 275
   ATT TAC GGC ACG GCC GGC AAC GAC AGT AAG ATT GAA GGC ACT AAA ATC     2004
   Ile Tyr Gly Thr Ala Gly Asn Asp Ser Lys Ile Glu Gly Thr Lys Ile
                   280                 285                 290
   ACC CGT AGG ATT GCG GGT AAA GAA GTT ACG CTT GAT ATT GCC AAT CAG     2052
   Thr Arg Arg Ile Ala Gly Lys Glu Val Thr Leu Asp Ile Ala Asn Gln
               295                 300                 305
   AAA ATT GAA AAA GGC GTG TTA GAG AAA TTG GGG CTG TCT GTT AGT GGT     2100
   Lys Ile Glu Lys Gly Val Leu Glu Lys Leu Gly Leu Ser Val Ser Gly
           310                 315                 320
   TCG GAT ATC ATT AAA TTG TTG TTT GGA GCA TTG ACT CCA ACT TTA AAT     2148
   Ser Asp Ile Ile Lys Leu Leu Phe Gly Ala Leu Thr Pro Thr Leu Asn
       325                 330                 335
   AGA ATG TTG CTA TCA CAA CTT ATC CAG TCT TTT TCC GAT AGC TTG GCT     2196
   Arg Met Leu Leu Ser Gln Leu Ile Gln Ser Phe Ser Asp Ser Leu Ala
   340                 345                 350                 355
   AAA CTT GAT AAT CCC TTA GCC CCT TAC ACT AAA AAT GGC GTG GTT TAT     2244
   Lys Leu Asp Asn Pro Leu Ala Pro Tyr Thr Lys Asn Gly Val Val Tyr
                   360                 365                 370
   GTC ACC GGC AAA GGG AAT GAT GTG CTT AAA GGA ACT GAA CAT GAG GAT     2292
   Val Thr Gly Lys Gly Asn Asp Val Leu Lys Gly Thr Glu His Glu Asp
               375                 380                 385
   TTG TTT CTC GGT GGT GAG GGG AAT GAT ACT TAT TAT GCG AGA GTA GGC     2340
   Leu Phe Leu Gly Gly Glu Gly Asn Asp Thr Tyr Tyr Ala Arg Val Gly
           390                 395                 400
   GAT ACA ATT GAA GAC GCC GAC GGC AAA GGT AAA GTC TAT TTT GTG AGA     2388
   Asp Thr Ile Glu Asp Ala Asp Gly Lys Gly Lys Val Tyr Phe Val Arg
       405                 410                 415
   GAA AAA GGG GTA CCT AAG GCG GAT CCT AAG CGG GTA GAG TTT AGC GAG     2436
   Glu Lys Gly Val Pro Lys Ala Asp Pro Lys Arg Val Glu Phe Ser Glu
   420                 425                 430                 435
   TAC ATA ACG AAA GAA GAA ATA AAA GAG GTT GAA AAG GGG TTA TTA ACT     2484
   Tyr Ile Thr Lys Glu Glu Ile Lys Glu Val Glu Lys Gly Leu Leu Thr
                   440                 445                 450
   TAC GCA GTT TTA GAA AAT TAT AAT TGG GAA GAG AAA ACG GCG ACT TTC     2532
   Tyr Ala Val Leu Glu Asn Tyr Asn Trp Glu Glu Lys Thr Ala Thr Phe
               455                 460                 465
   GCT CAT GCG ACT ATG CTT AAT GAG CTT TTT ACT GAT TAT ACT AAT TAT     2580
   Ala His Ala Thr Met Leu Asn Glu Leu Phe Thr Asp Tyr Thr Asn Tyr
           470                 475                 480
   CGT TAT GAA GTT AAA GGA CTA AAA TTG CCC GCC GTT AAA AAG TTA AAA     2628
   Arg Tyr Glu Val Lys Gly Leu Lys Leu Pro Ala Val Lys Lys Leu Lys
       485                 490                 495
   AGT CCG TTG GTG GAG TTT ACA GCT GAT TTA TTA ACT GTT ACG CCT ATT      2676
   Ser Pro Leu Val Glu Phe Thr Ala Asp Leu Leu Thr Val Thr Pro Ile
   500                 505                 510                 515
   GAC GAA AAC GGA AAA GCA CTT AGC GAA AAA AGT ATT ACG GTT AAA AAT      2724
   Asp Glu Asn Gly Lys Ala Leu Ser Glu Lys Ser Ile Thr Val Lys Asn
                   520                 525                 530
   TTT AAA AAT GGT GAT TTA GGA ATA AGG TTG TTG GAT CCT AAT AGC TAT      2772
   Phe Lys Asn Gly Asp Leu Gly Ile Arg Leu Leu Asp Pro Asn Ser Tyr
               535                 540                 545
   TAT TAT TTC CTT GAA GGC CAA GAT ACG GGT TTT TAT GGT CCT GCT TTT      2820
   Tyr Tyr Phe Leu Glu Gly Gln Asp Thr Gly Phe Tyr Gly Pro Ala Phe
           550                 555                 560
   TAT ATT GAA CGA AAA AAC GGT GGC GGC GCT AAA AAT AAC TCG TCG GGA      2868
   Tyr Ile Glu Arg Lys Asn Gly Gly Gly Ala Lys Asn Asn Ser Ser Gly
       565                 570                 575
   GCA GGA AAT AGC AAA GAT TGG GGC GGG AAC GGG CAT GGA AAT CAC CGA      2916
   Ala Gly Asn Ser Lys Asp Trp Gly Gly Asn Gly His Gly Asn His Arg
   580                 585                 590                 595
   AAT AAT GCC TCC GAC CTG AAT AAA CCG GAC GGA AAT AAT GGG AAT AAC      2964
   Asn Asn Ala Ser Asp Leu Asn Lys Pro Asp Gly Asn Asn Gly Asn Asn
                   600                 605                 610
   CAA AAT AAC GGA AGC AAT CAA GAT AAT CAT AGC GAT GTG AAT GCG CCA      3012
   Gln Asn Asn Gly Ser Asn Gln Asp Asn His Ser Asp Val Asn Ala Pro
               615                 620                 625
   AAT AAC CCG GGA CGT AAC TAT GAT ATT TAC GAT CCT TTA GCT TTA GAT      3060
   Asn Asn Pro Gly Arg Asn Tyr Asp Ile Tyr Asp Pro Leu Ala Leu Asp
           630                 635                 640
   TTA GAT GGA GAT GGG CTT GAA ACC GTG TCG ATG AAC GGG CGA CAA GGC      3108
   Leu Asp Gly Asp Gly Leu Glu Thr Val Ser Met Asn Gly Arg Gln Gly
       645                 650                 655
   GCG TTA TTC GAT CAT GAA GGA AAA GGT ATT CGT ACC GCA ACG GGC TGG      3156
   Ala Leu Phe Asp His Glu Gly Lys Gly Ile Arg Thr Ala Thr Gly Trp
   660                 665                 670                 675
   CTC GCT GCG GAT GAC GGT TTT TTA GTG TTA GAT CGT AAC CAA GAC GGC      3204
   Leu Ala Ala Asp Asp Gly Phe Leu Val Leu Asp Arg Asn Gln Asp Gly
                   680                 685                 690
   ATT ATT AAT GAT ATA AGC GAG TTA TTT AGT AAT AAA AAT CAA CTT TCC      3252
   Ile Ile Asn Asp Ile Ser Glu Leu Phe Ser Asn Lys Asn Gln Leu Ser
               695                 700                 705
   GAC GGC AGT ATT TCT GCA CAC GGT TTT GCG ACA TTA GCC GAT TTG GAT      3300
   Asp Gly Ser Ile Ser Ala His Gly Phe Ala Thr Leu Ala Asp Leu Asp
           710                 715                 720
   ACA AAC CAA GAT CAG CGT ATC GAC CAA AAT GAT AAG CTG TTT TCT AAA      3348
   Thr Asn Gln Asp Gln Arg Ile Asp Gln Asn Asp Lys Leu Phe Ser Lys
       725                 730                 735
   CTC CAA ATT TGG CGG GAT TTA AAT CAA AAC GGT TTT AGT GAA GCG AAT      3396
   Leu Gln Ile Trp Arg Asp Leu Asn Gln Asn Gly Phe Ser Glu Ala Asn
   740                 745                 750                 755
   GAG CTG TTT AGC TTA GAA AGT TTG AAT ATT AAA TCT TTA CAT ACC GCC      3444
   Glu Leu Phe Ser Leu Glu Ser Leu Asn Ile Lys Ser Leu His Thr Ala
                   760                 765                 770
   TAT GAA GAG CGT AAT GAT TTT CTA GCG GGC AAT AAT ATC CTT GCT CAG      3492
   Tyr Glu Glu Arg Asn Asp Phe Leu Ala Gly Asn Asn Ile Leu Ala Gln
               775                 780                 785
   CTT GGG AAG TAT GAA AAA ACG GAC GGT ACT TTT GCA CAA ATG GGC GAT      3540
   Leu Gly Lys Tyr Glu Lys Thr Asp Gly Thr Phe Ala Gln Met Gly Asp
           790                 795                 800
   TTA AAT TTC AGT TTT AAC CCG TTT TAT AGC CGA TTT ACC GAA GCG TTA      3588
   Leu Asn Phe Ser Phe Asn Pro Phe Tyr Ser Arg Phe Thr Glu Ala Leu
       805                 810                 815
   AAT TTA ACC GAG CAA CAA CGT CGC ACA ATT AAT CTA ACC GGC ACC GGT      3636
   Asn Leu Thr Glu Gln Gln Arg Arg Thr Ile Asn Leu Thr Gly Thr Gly
   820                 825                 830                 835
   CGG GTT CGG GAT TTG CGT GAA GCC GCC GCA CTT TCT GAG GAG TTG GCT     3684
   Arg Val Arg Asp Leu Arg Glu Ala Ala Ala Leu Ser Glu Glu Leu Ala
                   840                 845                 850
   GCT TTA TTA CAA CAG TAC ACT AAG GCC TCC GAT TTT CAG GCA CAA CGA     3732
   Ala Leu Leu Gln Gln Tyr Thr Lys Ala Ser Asp Phe Gln Ala Gln Arg
               855                 860                 865
   GAA TTA TTG CCT GCC ATT TTA GAT AAA TGG GCG GCA ACG GAT TTA CAG     3780
   Glu Leu Leu Pro Ala Ile Leu Asp Lys Trp Ala Ala Thr Asp Leu Gln
           870                 875                 880
   TAT CAA CAT TAT GAT AAA ACA TTA CTT AAA ACG GTA GAA AGT ACC GAT     3828
   Tyr Gln His Tyr Asp Lys Thr Leu Leu Lys Thr Val Glu Ser Thr Asp
       885                 890                 895
   AGT AGT GCT TCT GTC GTT AGA GTC ACG CCT TCT CAA TTA AGT AGT ATA     3876
   Ser Ser Ala Ser Val Val Arg Val Thr Pro Ser Gln Leu Ser Ser Ile
   900                 905                 910                 915
   CGC AAT GCA AAG CAT GAT CCT ACC GTT ATG CAA AAC TTT GAA CAG AGT     3924
   Arg Asn Ala Lys His Asp Pro Thr Val Met Gln Asn Phe Glu Gln Ser
                   920                 925                 930
   AAG GCA AAA ATT GCG ACT TTA AAT TCG CTC TAC GGG TTA AAT ATC GAT     3972
   Lys Ala Lys Ile Ala Thr Leu Asn Ser Leu Tyr Gly Leu Asn Ile Asp
               935                 940                 945
   CAA CTT TAT TAC ACG ACG GAT AAA GAC ATT CGC TAT ATT ACT GAT AAA     4020
   Gln Leu Tyr Tyr Thr Thr Asp Lys Asp Ile Arg Tyr Ile Thr Asp Lys
           950                 955                 960
   GTG AAT AAT ATG TAT CAA ACA ACC GTA GAA CTT GCC TAC CGT TCT TTA     4068
   Val Asn Asn Met Tyr Gln Thr Thr Val Glu Leu Ala Tyr Arg Ser Leu
       965                 970                 975
   CTT TTA CAA ACG CGT TTG AAG AAA TAT GTT TAT AGC GTT AAT GCG AAA     4116
   Leu Leu Gln Thr Arg Leu Lys Lys Tyr Val Tyr Ser Val Asn Ala Lys
   980                 985                 990                 995
   CAA TTC GAA GGG AAA TGG GTA ACC GAT TAT TCT CGT ACT GAA GCC TTA     4164
   Gln Phe Glu Gly Lys Trp Val Thr Asp Tyr Ser Arg Thr Glu Ala Leu
                   1000                1005                1010
   TTT AAC TCT ACT TTT AAA CAA TCG CCT GAA AAT GCA TTA TAT GAT TTA     4212
   Phe Asn Ser Thr Phe Lys Gln Ser Pro Glu Asn Ala Leu Tyr Asp Leu
               1015                1020                1025
   AGC GAA TAC CTT TCT TTC TTT AAC GAT CCT ACG GAA TGG AAA GAA GGG     4260
   Ser Glu Tyr Leu Ser Phe Phe Asn Asp Pro Thr Glu Trp Lys Glu Gly
           1030                1035                1040
   CTA TTA CTG TTA AGC CGT TAT ATA GAT TAT GCT AAA GCA CAA GGA TTT     4308
   Leu Leu Leu Leu Ser Arg Tyr Ile Asp Tyr Ala Lys Ala Gln Gly Phe
       1045                1050                1055
   TAT GAA AAC TGG GCG GCT ACT TCT AAC TTA ACT ATT GCC CGT TTA AGA     4356
   Tyr Glu Asn Trp Ala Ala Thr Ser Asn Leu Thr Ile Ala Arg Leu Arg
   1060                1065                1070                1075
   GAG GCT GGA GTA ATT TTT GCA GAA TCG ACG GAT TTA AAA GGC GAT GAA     4404
   Glu Ala Gly Val Ile Phe Ala Glu Ser Thr Asp Leu Lys Gly Asp Glu
                   1080                1085                1090
   AAA AAT AAT ATT TTG TTA GGT AGC CAA AAA GAT AAT AAC TTA TCG GGT     4452
   Lys Asn Asn Ile Leu Leu Gly Ser Gln Lys Asp Asn Asn Leu Ser Gly
               1095                1100                1105
   AGT GCA GGT GAT GAT CTA CTT ATC GGC GGA GAG GGT AAT GAT ACG TTA     4500
   Ser Ala Gly Asp Asp Leu Leu Ile Gly Gly Glu Gly Asn Asp Thr Leu
           1110                1115                1120
   AAA GGC AGC TAC GGT GCA GAC ACC TAT ATC TTT AGC AAA GGA CAC GGA     4548
   Lys Gly Ser Tyr Gly Ala Asp Thr Tyr Ile Phe Ser Lys Gly His Gly
       1125                1130                1135
   CAG GAT ATC GTT TAT GAA GAT ACC AAT AAT GAT AAC CGC GCA AGA GAT     4596
   Gln Asp Ile Val Tyr Glu Asp Thr Asn Asn Asp Asn Arg Ala Arg Asp
   1140                1145                1150                1155
   ATC GAC ACC TTA AAA TTT ACC GAT GTG AAT TAT GCG GAA GTG AAG TTT     4644
   Ile Asp Thr Leu Lys Phe Thr Asp Val Asn Tyr Ala Glu Val Lys Phe
                   1160                1165                1170
   CGA CGA GTA GAT AAT GAC TTA ATG TTA TTC GGT TAT CAT GAT ACG GAT     4692
   Arg Arg Val Asp Asn Asp Leu Met Leu Phe Gly Tyr His Asp Thr Asp
               1175                1180                1185
   TCG GTC ACG GTA AAA TCC TTC TAC AGC CAT GTA GAT TAT CAA TTT GAC      4740
   Ser Val Thr Val Lys Ser Phe Tyr Ser His Val Asp Tyr Gln Phe Asp
           1190                1195                1200
   AAA TTG GAG TTT GCT GAC CGC AGT ATA ACT CGC GAT GAA CTG ATT AAA      4788
   Lys Leu Glu Phe Ala Asp Arg Ser Ile Thr Arg Asp Glu Leu Ile Lys
       1205                1210                1215
   GCA GGG CTT CAT CTA TAC GGC ACC GAT GGC AAT GAT GAT ATA AAG GAT      4836
   Ala Gly Leu His Leu Tyr Gly Thr Asp Gly Asn Asp Asp Ile Lys Asp
   1220                1225                1230                1235
   CAT GCG GAT TGG GAC AGC ATT TTG GAA GGC GGC AAA GGC AAC GAT ATT      4884
   His Ala Asp Trp Asp Ser Ile Leu Glu Gly Gly Lys Gly Asn Asp Ile
                   1240                1245                1250
   CTA AGA GGT GGC TAC GGT GCG GAC ACC TAT ATC TTT AGC AAA GGA CAC      4932
   Leu Arg Gly Gly Tyr Gly Ala Asp Thr Tyr Ile Phe Ser Lys Gly His
               1255                1260                1265
   GGA CAG GAT ATC GTT TAT GAA GAT ACC AAT AAT GAT AAC CGC GCA AGA      4980
   Gly Gln Asp Ile Val Tyr Glu Asp Thr Asn Asn Asp Asn Arg Ala Arg
           1270                1275                1280
   GAT ATC GAC ACC TTA AAA TTT ACT GAT GTG AAT TAT GCG GAA GTG AAA      5028
   Asp Ile Asp Thr Leu Lys Phe Thr Asp Val Asn Tyr Ala Glu Val Lys
       1285                1290                1295
   TTC CGA CGA GTA GAT AAT GAC TTA ATG TTA TTC GGT TAT CAT GAT ACG      5076
   Phe Arg Arg Val Asp Asn Asp Leu Met Leu Phe Gly Tyr His Asp Thr
   1300                1305                1310                1315
   GAT TCG GTC ACG ATA AAA TCC TTC TAC AAC CAT GTA GAT TAT CAA TTT      5124
   Asp Ser Val Thr Ile Lys Ser Phe Tyr Asn His Val Asp Tyr Gln Phe
                   1320                1325                1330
   GAC AAA TTG GAA TTT GCT GAC CGC AGT ATA ACT CGT GAT GAA CTA GGT      5172
   Asp Lys Leu Glu Phe Ala Asp Arg Ser Ile Thr Arg Asp Glu Leu Gly
               1335                1340                1345
   AAA CAA GGT ATG GCA TTA TTT GGC ACT GAC GGT GAT GAT AAT ATC AAC      5220
   Lys Gln Gly Met Ala Leu Phe Gly Thr Asp Gly Asp Asp Asn Ile Asn
           1350                1355                1360
   GAC TGG GGA CGT AAC TCG GTG ATT GAT GCC GGT GCG GGT AAT GAT ACG      5268
   Asp Trp Gly Arg Asn Ser Val Ile Asp Ala Gly Ala Gly Asn Asp Thr
       1365                1370                1375
   GTT AAT GGC GGT AAT GGC GAT GAC ACC CTC ATC GGC GGC AAA GGT AAT      5316
   Val Asn Gly Gly Asn Gly Asp Asp Thr Leu Ile Gly Gly Lys Gly Asn
   1380                1385                1390                1395
   GAT ATT CTA AGA GGT GGC TAC GGT GCG GAC ACC TAT ATC TTT AGC AAA      5364
   Asp Ile Leu Arg Gly Gly Tyr Gly Ala Asp Thr Tyr Ile Phe Ser Lys
                   1400                1405                1410
   GGA CAC GGA CAG GAT ATC GTT TAT GAA GAT ACC AAT AAT GAT AAC CGC      5412
   Gly His Gly Gln Asp Ile Val Tyr Glu Asp Thr Asn Asn Asp Asn Arg
               1415                1420                1425
   GCA AGA GAT ATC GAC ACC TTA AAA TTT ACC GAT GTG AAT TAT GCG GAA      5460
   Ala Arg Asp Ile Asp Thr Leu Lys Phe Thr Asp Val Asn Tyr Ala Glu
           1430                1435                1440
   GTG AAA TTC CGA CGA GTA GAT AAT GAC TTA ATG TTA TTC GGT TAT CAT      5508
   Val Lys Phe Arg Arg Val Asp Asn Asp Leu Met Leu Phe Gly Tyr His
       1445                1450                1455
   GAT ACG GAT TCG GTC ACG GTA AAA TCC TTC TAC AGC CAT GTA GAT TAT      5556
   Asp Thr Asp Ser Val Thr Val Lys Ser Phe Tyr Ser His Val Asp Tyr
   1460                1465                1470                1475
   CAA TTT GAC AAA TTG GAG TTT GCT GAC CGC AGT ATA ACT CGC GAT GAA      5604
   Gln Phe Asp Lys Leu Glu Phe Ala Asp Arg Ser Ile Thr Arg Asp Glu
                   1480                1485                1490
   CTG ATT AAA GCA GGG CTT CAT CTA TAC GGC ACC GAT GGC AAT GAT GAT      5652
   Leu Ile Lys Ala Gly Leu His Leu Tyr Gly Thr Asp Gly Asn Asp Asp
               1495                1500                1505
   ATA AAG GAT CAT GCG GAT TGG GAC AGC ATT TTG GAA GGC GGC AAA GGC      5700
   Ile Lys Asp His Ala Asp Trp Asp Ser Ile Leu Glu Gly Gly Lys Gly
            1510                     1515                     1520
   AAC GAT ATT CTA AGA GGT GGC TAC GGT GCG GAC ACC TAT ATC TTT AGC        5748
   Asn Asp Ile Leu Arg Gly Gly Tyr Gly Ala Asp Thr Tyr Ile Phe Ser
       1525                     1530                1535
   AAA GGA CAC GGA CAG GAT ATC GTT TAT GAA GAT ACC AAT AAT GAT AAC        5796
   Lys Gly His Gly Gln Asp Ile Val Tyr Glu Asp Thr Asn Asn Asp Asn
   1540                1545                1550                1555
   CGA GCA AGA GAT ATC GAC ACC TTA AAA TTT ACT GAT GTG AAT TAT GCG        5844
   Arg Ala Arg Asp Ile Asp Thr Leu Lys Phe Thr Asp Val Asn Tyr Ala
                   1560                1565                1570
   GAA GTG AAA TTC CGA CGA GTA GAT AAT GAC TTA ATG TTA TTC GGT TAT        5892
   Glu Val Lys Phe Arg Arg Val Asp Asn Asp Leu Met Leu Phe Gly Tyr
               1575                1580                1585
   CAT GAT ACG GAT TCG GTC ACG ATA AAA TCC TTC TAC AAC CAT GTA GAT        5940
   His Asp Thr Asp Ser Val Thr Ile Lys Ser Phe Tyr Asn His Val Asp
           1590                1595                1600
   TAT CAA TTT GAC AAA TTG GAA TTT GCT GAC CGC AGT ATA ACT CGT GAT        5988
   Tyr Gln Phe Asp Lys Leu Glu Phe Ala Asp Arg Ser Ile Thr Arg Asp
       1605                1610                1615
   GAA CTA GGT AAA CAA GGT ATG GCA TTA TTT GGC ACT GAC GGT GAT GAT        6036
   Glu Leu Gly Lys Gln Gly Met Ala Leu Phe Gly Thr Asp Gly Asp Asp
   1620                1625                1630                1635
   AAT ATC AAC GAC TGG GGA CGT AAC TCG GTG ATT GAT GCC GGT GCG GGT        6084
   Asn Ile Asn Asp Trp Gly Arg Asn Ser Val Ile Asp Ala Gly Ala Gly
                   1640                1645                1650
   AAT GAT ACG GTT AAT GGC GGT AAT GGC GAT GAC ACC CTC ATC GGC GGC        6132
   Asn Asp Thr Val Asn Gly Gly Asn Gly Asp Asp Thr Leu Ile Gly Gly
               1655                1660                1665
   AAA GGT AAT GAT ATT CTA AGA GGT GGC TAC GGT GCG GAC ACC TAT ATC        6180
   Lys Gly Asn Asp Ile Leu Arg Gly Gly Tyr Gly Ala Asp Thr Tyr Ile
           1670                1675                1680
   TTT AGC AAA GGA CAC GGA CAG GAT ATC GTT TAT GAA GAT ACC AAT AAT        6228
   Phe Ser Lys Gly His Gly Gln Asp Ile Val Tyr Glu Asp Thr Asn Asn
       1685                1690                1695
   GAT AAC CGC GCA AGA GAT ATC GAC ACC TTA AAA TTT ACT GAT ATT AAT        6276
   Asp Asn Arg Ala Arg Asp Ile Asp Thr Leu Lys Phe Thr Asp Ile Asn
   1700                1705                1710                1715
   TTA TCC GAA CTT TGG TTT AGC CGA GAA AAT AAC GAT TTG ATT ATT AAA        6324
   Leu Ser Glu Leu Trp Phe Ser Arg Glu Asn Asn Asp Leu Ile Ile Lys
                   1720                1725                1730
   TCA TTA TTA AGT GAG GAT AAA GTC ACG GTT CAA AAT TGG TAT TCA CAC        6372
   Ser Leu Leu Ser Glu Asp Lys Val Thr Val Gln Asn Trp Tyr Ser His
               1735                1740                1745
   CAA GAT CAT AAA ATA GAA AAT ATT CGT TTA TCG AAT GAG CAA ACG TTG        6420
   Gln Asp His Lys Ile Glu Asn Ile Arg Leu Ser Asn Glu Gln Thr Leu
           1750                1755                1760
   GTG AGC ACT CAG GTG GAG AAG ATG GTT GAG TCG ATG GCC GGC TTT GCT        6468
   Val Ser Thr Gln Val Glu Lys Met Val Glu Ser Met Ala Gly Phe Ala
       1765                1770                1775
   CAG AAG CAC GGA GGA GAG ATA TCT CTT GTG TCG CTT GAA GAG GTA AAA        6516
   Gln Lys His Gly Gly Glu Ile Ser Leu Val Ser Leu Glu Glu Val Lys
   1780                1785                1790                1795
   CAA TAT ATC AAT AGC TTA ACA GCT GCT TTA TAA CATACGAAAG AAATCGGCAC      6569
   Gln Tyr Ile Asn Ser Leu Thr Ala Ala Leu  *
                   1800                1805
   AGTTTTTTTG AACTGTGCCG ATTTGATTTT AGTGTAAGAA TATAGCCTGA TTTTAAGAAA      6629
   TTTACTCTTG GCTAATAACT ATTTCCCATT TTATAAGTTA TTGACGGATG GTTTTATCAA      6689
   ATATGAGATC AAATCTTATT TTAAATTCGC TTTCCATTAA GCGATAT                    6736
(2) INFORMATION FOR SEQ ID NO: 6:
   (i) SEQUENCE CHARACTERISTICS:
      (A) LENGTH: 151 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Ile Asp Met Pro Pro Gly Thr Gly Asp Ile Gln Leu Thr Leu Ser Gln
    1               5                  10                  15
   Gln Ile Pro Val Thr Gly Ala Val Val Val Thr Thr Pro Gln Asp Ile
               20                  25                  30
   Ala Leu Leu Asp Ala Val Lys Gly Ile Ser Met Phe Gln Lys Val Ser
           35                  40                  45
   Val Pro Val Leu Gly Ile Ile Glu Asn Met Ser Val His Ile Cys Gln
       50                  55                  60
   Asn Cys Gly His His Glu Asp Ile Phe Gly Thr Gly Gly Ala Glu Lys
   65                  70                  75                  80
   Val Ala Lys Lys Tyr Gly Thr Lys Val Leu Gly Gln Met Pro Leu His
                   85                  90                  95
   Ile Arg Leu Arg Gln Asp Leu Asp Ala Gly Thr Pro Thr Val Val Ala
               100                 105                 110
   Ala Pro Glu His Glu Thr Ser Arg Ala Tyr Ile Glu Leu Ala Ala Lys
           115                 120                 125
   Val Ala Ser Glu Leu Tyr Trp Gln Gly Ser Val Ile Pro Ser Glu Ile
       130                 135                 140
   Met Ile Arg Glu Val Lys  *
   145                 150
(2) INFORMATION FOR SEQ ID NO: 7:
   (i) SEQUENCE CHARACTERISTICS:
      (A) LENGTH: 1806 amino acids
      (B) TYPE: amino acid
      (D) TOPOLOGY: linear
   (ii) MOLECULE TYPE: protein
   (xi) SEQUENCE DESCRIPTION: SEQ ID NO: 7:
   Met Thr Lys Leu Thr Met Gln Asp Val Thr Asn Leu Tyr Leu Tyr Lys
   1 5 10 15
   Thr Lys Thr Leu Pro Lys Asp Arg Leu Asp Asp Ser Leu Ile Ser Glu
   20 25 30
   Ile Gly Lys Gly Asp Asp Asp Ile Asp Arg Lys Glu Phe Met Val Gly
   35 40 45
   Pro Gly Arg Phe Val Thr Ala Asp Asn Phe Ser Val Val Arg Asp Phe
   50 55 60
   Phe Asn Ala Gly Lys Ser Arg Ile Ile Ala Pro Gln Val Pro Pro Ile
   65 70 75 80
   Arg Ser Gln Gln Glu Lys Ile Leu Val Gly Leu Lys Pro Gly Lys Tyr
   85 90 95
   Ser Lys Ala Gln Ile Leu Glu Met Leu Gly Tyr Thr Lys Gly Gly Glu
   100 105 110
   Val Val Asn Gly Met Phe Ala Gly Glu Val Gln Thr Leu Gly Phe Tyr
   115 120 125
   Asp Asp Gly Lys Gly Asp Leu Leu Glu Arg Ala Tyr Ile Trp Asn Thr
   130 135 140
   Thr Gly Phe Lys Met Ser Asp Asn Ala Phe Phe Val Ile Glu Glu Ser
   145 150 155 160
   Gly Lys Arg Tyr Ile Glu Asn Phe Gly Ile Glu Pro Leu Gly Lys Gln
   165 170 175
   Glu Asp Phe Asp Phe Val Gly Gly Phe Trp Ser Asn Leu Val Asn Arg
   180 185 190
   Gly Leu Glu Ser Ile Ile Asp Pro Ser Gly Ile Gly Gly Thr Val Asn
   195 200 205
   Leu Asn Phe Thr Gly Glu Val Glu Thr Tyr Thr Leu Asp Glu Thr Arg
       210                 215                 220
   Phe Lys Ala Glu Ala Ala Lys Lys Ser His Trp Ser Leu Val Asn Ala
   225                 230                 235                 240
   Ala Lys Val Tyr Gly Gly Leu Asp Gln Ile Ile Lys Lys Leu Trp Asp
                   245                 250                 255
   Ser Gly Ser Ile Lys His Leu Tyr Gln Asp Lys Asp Thr Gly Lys Leu
               260                 265                 270
   Lys Pro Ile Ile Tyr Gly Thr Ala Gly Asn Asp Ser Lys Ile Glu Gly
           275                 280                 285
   Thr Lys Ile Thr Arg Arg Ile Ala Gly Lys Glu Val Thr Leu Asp Ile
       290                 295                 300
   Ala Asn Gln Lys Ile Glu Lys Gly Val Leu Glu Lys Leu Gly Leu Ser
   305                 310                 315                 320
   Val Ser Gly Ser Asp Ile Ile Lys Leu Leu Phe Gly Ala Leu Thr Pro
                   325                 330                 335
   Thr Leu Asn Arg Met Leu Leu Ser Gln Leu Ile Gln Ser Phe Ser Asp
               340                 345                 350
   Ser Leu Ala Lys Leu Asp Asn Pro Leu Ala Pro Tyr Thr Lys Asn Gly
           355                 360                 365
   Val Val Tyr Val Thr Gly Lys Gly Asn Asp Val Leu Lys Gly Thr Glu
       370                 375                 380
   His Glu Asp Leu Phe Leu Gly Gly Glu Gly Asn Asp Thr Tyr Tyr Ala
   385                 390                 395                 400
   Arg Val Gly Asp Thr Ile Glu Asp Ala Asp Gly Lys Gly Lys Val Tyr
                   405                 410                 415
   Phe Val Arg Glu Lys Gly Val Pro Lys Ala Asp Pro Lys Arg Val Glu
               420                 425                 430
   Phe Ser Glu Tyr Ile Thr Lys Glu Glu Ile Lys Glu Val Glu Lys Gly
           435                 440                 445
   Leu Leu Thr Tyr Ala Val Leu Glu Asn Tyr Asn Trp Glu Glu Lys Thr
       450                 455                 460
   Ala Thr Phe Ala His Ala Thr Met Leu Asn Glu Leu Phe Thr Asp Tyr
   465                 470                 475                 480
   Thr Asn Tyr Arg Tyr Glu Val Lys Gly Leu Lys Leu Pro Ala Val Lys
                   485                 490                 495
   Lys Leu Lys Ser Pro Leu Val Glu Phe Thr Ala Asp Leu Leu Thr Val
               500                 505                 510
   Thr Pro Ile Asp Glu Asn Gly Lys Ala Leu Ser Glu Lys Ser Ile Thr
           515                 520                 525
   Val Lys Asn Phe Lys Asn Gly Asp Leu Gly Ile Arg Leu Leu Asp Pro
       530                 535                 540
   Asn Ser Tyr Tyr Tyr Phe Leu Glu Gly Gln Asp Thr Gly Phe Tyr Gly
   545                 550                 555                 560
   Pro Ala Phe Tyr Ile Glu Arg Lys Asn Gly Gly Gly Ala Lys Asn Asn
                   565                 570                 575
   Ser Ser Gly Ala Gly Asn Ser Lys Asp Trp Gly Gly Asn Gly His Gly
               580                 585                 590
   Asn His Arg Asn Asn Ala Ser Asp Leu Asn Lys Pro Asp Gly Asn Asn
           595                 600                 605
   Gly Asn Asn Gln Asn Asn Gly Ser Asn Gln Asp Asn His Ser Asp Val
       610                 615                 620
   Asn Ala Pro Asn Asn Pro Gly Arg Asn Tyr Asp Ile Tyr Asp Pro Leu
   625                 630                 635                 640
   Ala Leu Asp Leu Asp Gly Asp Gly Leu Glu Thr Val Ser Met Asn Gly
                   645                 650                 655
   Arg Gln Gly Ala Leu Phe Asp His Glu Gly Lys Gly Ile Arg Thr Ala
   660 665 670
   Thr Gly Trp Leu Ala Ala Asp Asp Gly Phe Leu Val Leu Asp Arg Asn
   675 680 685
   Gln Asp Gly Ile Ile Asn Asp Ile Ser Glu Leu Phe Ser Asn Lys Asn
   690 695 700
   Gln Leu Ser Asp Gly Ser Ile Ser Ala His Gly Phe Ala Thr Leu Ala
   705 710 715 720
   Asp Leu Asp Thr Asn Gln Asp Gln Arg Ile Asp Gln Asn Asp Lys Leu
   725 730 735
   Phe Ser Lys Leu Gln Ile Trp Arg Asp Leu Asn Gln Asn Gly Phe Ser
   740 745 750
   Glu Ala Asn Glu Leu Phe Ser Leu Glu Ser Leu Asn Ile Lys Ser Leu
   755 760 765
   His Thr Ala Tyr Glu Glu Arg Asn Asp Phe Leu Ala Gly Asn Asn Ile
   770 775 780
   Leu Ala Gln Leu Gly Lys Tyr Glu Lys Thr Asp Gly Thr Phe Ala Gln
   785 790 795 800
   Met Gly Asp Leu Asn Phe Ser Phe Asn Pro Phe Tyr Ser Arg Phe Thr
   805 810 815
   Glu Ala Leu Asn Leu Thr Glu Gln Gln Arg Arg Thr Ile Asn Leu Thr
   820 825 830
   Gly Thr Gly Arg Val Arg Asp Leu Arg Glu Ala Ala Ala Leu Ser Glu
   835 840 845
   Glu Leu Ala Ala Leu Leu Gln Gln Tyr Thr Lys Ala Ser Asp Phe Gln
   850 855 860
   Ala Gln Arg Glu Leu Leu Pro Ala Ile Leu Asp Lys Trp Ala Ala Thr
   865 870 875 880
   Asp Leu Gln Tyr Gln His Tyr Asp Lys Thr Leu Leu Lys Thr Val Glu
   885 890 895
   Ser Thr Asp Ser Ser Ala Ser Val Val Arg Val Thr Pro Ser Gln Leu
   900 905 910
   Ser Ser Ile Arg Asn Ala Lys His Asp Pro Thr Val Met Gln Asn Phe
   915 920 925
   Glu Gln Ser Lys Ala Lys Ile Ala Thr Leu Asn Ser Leu Tyr Gly Leu
   930 935 940
   Asn Ile Asp Gln Leu Tyr Tyr Thr Thr Asp Lys Asp Ile Arg Tyr Ile
   945 950 955 960
   Thr Asp Lys Val Asn Asn Met Tyr Gln Thr Thr Val Glu Leu Ala Tyr
   965 970 975
   Arg Ser Leu Leu Leu Gln Thr Arg Leu Lys Lys Tyr Val Tyr Ser Val
   980 985 990
   Asn Ala Lys Gln Phe Glu Gly Lys Trp Val Thr Asp Tyr Ser Arg Thr
   995 1000 1005
   Glu Ala Leu Phe Asn Ser Thr Phe Lys Gln Ser Pro Glu Asn Ala Leu
   1010 1015 1020
   Tyr Asp Leu Ser Glu Tyr Leu Ser Phe Phe Asn Asp Pro Thr Glu Trp
   1025 1030 1035 1040
   Lys Glu Gly Leu Leu Leu Leu Ser Arg Tyr Ile Asp Tyr Ala Lys Ala
   1045 1050 1055
   Gln Gly Phe Tyr Glu Asn Trp Ala Ala Thr Ser Asn Leu Thr Ile Ala
   1060 1065 1070
   Arg Leu Arg Glu Ala Gly Val Ile Phe Ala Glu Ser Thr Asp Leu Lys
   1075 1080 1085
   Gly Asp Glu Lys Asn Asn Ile Leu Leu Gly Ser Gln Lys Asp Asn Asn
   1090 1095 1100
   Leu Ser Gly Ser Ala Gly Asp Asp Leu Leu Ile Gly Gly Glu Gly Asn
   1105 1110 1115 1120
   Asp Thr Leu Lys Gly Ser Tyr Gly Ala Asp Thr Tyr Ile Phe Ser Lys
   1125 1130 1135
   Gly His Gly Gln Asp Ile Val Tyr Glu Asp Thr Asn Asn Asp Asn Arg
   1140 1145 1150
   Ala Arg Asp Ile Asp Thr Leu Lys Phe Thr Asp Val Asn Tyr Ala Glu
   1155 1160 1165
   Val Lys Phe Arg Arg Val Asp Asn Asp Leu Met Leu Phe Gly Tyr His
   1170 1175 1180
   Asp Thr Asp Ser Val Thr Val Lys Ser Phe Tyr Ser His Val Asp Tyr
   1185 1190 1195 1200
   Gln Phe Asp Lys Leu Glu Phe Ala Asp Arg Ser Ile Thr Arg Asp Glu
   1205 1210 1215
   Leu Ile Lys Ala Gly Leu His Leu Tyr Gly Thr Asp Gly Asn Asp Asp
   1220 1225 1230
   Ile Lys Asp His Ala Asp Trp Asp Ser Ile Leu Glu Gly Gly Lys Gly
   1235 1240 1245
   Asn Asp Ile Leu Arg Gly Gly Tyr Gly Ala Asp Thr Tyr Ile Phe Ser
   1250 1255 1260
   Lys Gly His Gly Gln Asp Ile Val Tyr Glu Asp Thr Asn Asn Asp Asn
   1265 1270 1275 1280
   Arg Ala Arg Asp Ile Asp Thr Leu Lys Phe Thr Asp Val Asn Tyr Ala
   1285 1290 1295
   Glu Val Lys Phe Arg Arg Val Asp Asn Asp Leu Met Leu Phe Gly Tyr
   1300 1305 1310
   His Asp Thr Asp Ser Val Thr Ile Lys Ser Phe Tyr Asn His Val Asp
   1315 1320 1325
   Tyr Gln Phe Asp Lys Leu Glu Phe Ala Asp Arg Ser Ile Thr Arg Asp
   1330 1335 1340
   Glu Leu Gly Lys Gln Gly Met Ala Leu Phe Gly Thr Asp Gly Asp Asp
   1345 1350 1355 1360
   Asn Ile Asn Asp Trp Gly Arg Asn Ser Val Ile Asp Ala Gly Ala Gly
   1365 1370 1375
   Asn Asp Thr Val Asn Gly Gly Asn Gly Asp Asp Thr Leu Ile Gly Gly
   1380 1385 1390
   Lys Gly Asn Asp Ile Leu Arg Gly Gly Tyr Gly Ala Asp Thr Tyr Ile
   1395 1400 1405
   Phe Ser Lys Gly His Gly Gln Asp Ile Val Tyr Glu Asp Thr Asn Asn
   1410 1415 1420
   Asp Asn Arg Ala Arg Asp Ile Asp Thr Leu Lys Phe Thr Asp Val Asn
   1425 1430 1435 1440
   Tyr Ala Glu Val Lys Phe Arg Arg Val Asp Asn Asp Leu Met Leu Phe
   1445 1450 1455
   Gly Tyr His Asp Thr Asp Ser Val Thr Val Lys Ser Phe Tyr Ser His
   1460 1465 1470
   Val Asp Tyr Gln Phe Asp Lys Leu Glu Phe Ala Asp Arg Ser Ile Thr
   1475 1480 1485
   Arg Asp Glu Leu Ile Lys Ala Gly Leu His Leu Tyr Gly Thr Asp Gly
   1490 1495 1500
   Asn Asp Asp Ile Lys Asp His Ala Asp Trp Asp Ser Ile Leu Glu Gly
   1505 1510 1515 1520
   Gly Lys Gly Asn Asp Ile Leu Arg Gly Gly Tyr Gly Ala Asp Thr Tyr
   1525 1530 1535
   Ile Phe Ser Lys Gly His Gly Gln Asp Ile Val Tyr Glu Asp Thr Asn
   1540 1545 1550
   Asn Asp Asn Arg Ala Arg Asp Ile Asp Thr Leu Lys Phe Thr Asp Val
   1555 1560 1565
   Asn Tyr Ala Glu Val Lys Phe Arg Arg Val Asp Asn Asp Leu Met Leu
   1570 1575 1580
   Phe Gly Tyr His Asp Thr Asp Ser Val Thr Ile Lys Ser Phe Tyr Asn
   1585 1590 1595 1600
   His Val Asp Tyr Gln Phe Asp Lys Leu Glu Phe Ala Asp Arg Ser Ile
   1605 1610 1615
   Thr Arg Asp Glu Leu Gly Lys Gln Gly Met Ala Leu Phe Gly Thr Asp
   1620 1625 1630
   Gly Asp Asp Asn Ile Asn Asp Trp Gly Arg Asn Ser Val Ile Asp Ala
   1635 1640 1645
   Gly Ala Gly Asn Asp Thr Val Asn Gly Gly Asn Gly Asp Asp Thr Leu
   1650 1655 1660
   Ile Gly Gly Lys Gly Asn Asp Ile Leu Arg Gly Gly Tyr Gly Ala Asp
   1665 1670 1675 1680
   Thr Tyr Ile Phe Ser Lys Gly His Gly Gln Asp Ile Val Tyr Glu Asp
   1685 1690 1695
   Thr Asn Asn Asp Asn Arg Ala Arg Asp Ile Asp Thr Leu Lys Phe Thr
   1700 1705 1710
   Asp Ile Asn Leu Ser Glu Leu Trp Phe Ser Arg Glu Asn Asn Asp Leu
   1715 1720 1725
   Ile Ile Lys Ser Leu Leu Ser Glu Asp Lys Val Thr Val Gln Asn Trp
   1730 1735 1740
   Tyr Ser His Gln Asp His Lys Ile Glu Asn Ile Arg Leu Ser Asn Glu
   1745 1750 1755 1760
   Gln Thr Leu Val Ser Thr Gln Val Glu Lys Met Val Glu Ser Met Ala
   1765 1770 1775
   Gly Phe Ala Gln Lys His Gly Gly Glu Ile Ser Leu Val Ser Leu Glu
   1780 1785 1790
   Glu Val Lys Gln Tyr Ile Asn Ser Leu Thr Ala Ala Leu *
   1795 1800 1805